# FASCICULE DE BREVET EUROPEEN

(11) **EP 3 233 837 B1**
(45) Date de publication et mention de la délivrance du brevet: **05.12.2018**
(21) Numéro de dépôt: 15817295.7
(22) Date de dépôt: 18.12.2015
(51) Int. Cl.: C07D 405/06, C07D 401/06, C07D 409/06, C07D 215/36, C07D 401/04, C07D 413/06, C07D 409/04, C07D 405/12, C07D 405/04, A61K 31/4709, A61P 17/00

(54) **DÉRIVÉS TÉTRAHYDROQUINOLINES SULFONAMIDES EN TANT QU'AGONISTES INVERSES DU RÉCEPTEUR GAMMA ORPHELIN ASSOCIÉ AUX RÉTINOÏDES ROR GAMMA (T)**
TETRAHYDROCHINOLINSULFONAMIDDERIVATE ALS INVERSE AGONISTEN VON RETINOID-RELATED ORPHAN RECEPTOR GAMMA (ROR GAMMA (T))
TETRAHYDROQUINOLINE SULFONAMIDE DERIVATIVES AS INVERSE AGONISTS OF RETINOID-RELATED ORPHAN RECEPTOR GAMMA (ROR GAMMA (T))

(30) Priorité: 19.12.2014 FR 1463035; 03.07.2015 FR 1556341
(43) Date de publication de la demande: 25.10.2017
(73) Titulaire: Galderma Research & Development, 06410 Biot (FR)
(72) Inventeur: MUSICKI, Branislav, 06000 Nice (FR); OUVRY, Gilles, 06410 Biot (FR); THOREAU, Etienne, 06460 Saint Vallier de Thiey (FR); BOUIX-PETER, Claire, 06220 Vallauris (FR)
(74) Mandataire: Casalonga
(86) Numéro de dépôt international: PCT/EP2015/080690
(87) Numéro de publication internationale: WO 2016/097392

(56) Documents cités:
- WO-A1-2011/137089
- WO-A1-2013/160418
- WO-A1-2014/090712
- YAN ZHANG ET AL: "Discovery of 2-oxo-1,2-dihydrobenzo[cd]indole-6-sulfona mide derivatives as new ROR[gamma] inhibitors using virtual screening, synthesis and biological evaluation", EUROPEAN JOURNAL OF MEDICINAL CHEMISTRY, vol. 78, 22 mars 2014 (2014-03-22), pages 431-441, XP028847891, ISSN: 0223-5234, DOI: 10.1016/J.EJMECH.2014.03.065

## Description

La présente invention concerne des dérivés sulfonamides bicycles particuliers, leurs sels d'addition pharmaceutiquement acceptables leurs hydrates et/ou leurs solvates ainsi que leur utilisation en tant qu'agoniste inverse du récepteur gamma orphelin associé aux rétinoïdes RORγt.

L'invention est également relative à une composition pharmaceutique comprenant de tels composés ainsi que son utilisation pour le traitement par voie topique et/ou orale des maladies inflammatoires médiées par les récepteurs RORγt, notamment l'acné, dermatite atopique et/ou le psoriasis.

Les récepteurs nucléaires forment une grande famille (appelée superfamille) de facteurs de transcription qui correspondent à des protéines capables d'être activées par un ligand, de se fixer sur des séquences d'ADN spécifiques et de réguler la transcription de gènes cibles. Ainsi ces récepteurs se retrouvent impliqués dans la régulation d'une grande variété de fonctions biologiques, dont la croissance, le développement, la reproduction, la différenciation et le métabolisme dans une multitude d'organismes vivants.

Les premiers membres de cette superfamille à avoir été identifiés et décrits dans la littérature scientifique sont les récepteurs nucléaires des hormones stéroïdes tels que les récepteurs aux glucocorticoïdes et les récepteurs oestrogènes. Cette superfamille comprend également parmi ses membres de nombreux récepteurs pour lesquels aucun ligand n'a été identifié. Ces récepteurs nucléaires sont appelés « récepteurs orphelins ».

Les récepteurs orphelins associés aux rétinoïdes (*Retinoid-related orphan receptors* en langue anglaise) constituent donc une sous-famille des récepteurs nucléaires. Cette sous-famille est composée de trois membres ayant chacun leur propre profil d'expression : ROR alpha (dénommé RORα), ROR beta (dénommé RORβ) et ROR gamma (dénommé RORγ). Deux isoformes des récepteurs orphelins RORγ ont déjà été identifiés, à savoir RORγ1, qui s'exprime dans une variété de tissus tels que le thymus, les reins, les muscles et le foie, et RORγ2 (aussi nommé RORγt) qui s'exprime exclusivement dans les cellules du système immunitaire.

En particulier, le récepteur RORγt joue un rôle important de régulateur dans la différenciation cellulaire des lymphocytes Th17 qui correspondent à des lymphocytes T auxiliaires ayant pour fonction d'assurer la défense de l'organisme par rapport à un grand nombre d'agents pathogènes extracellulaires tels que les bactéries et les infections fongiques.

Toutefois, il a été démontré que les lymphocytes Th17 sont également impliqués dans une large variété de désordres inflammatoires, tels que l'acné, et de maladies auto-immunes telles que le psoriasis, l'arthrite rhumatoïde ou encore la sclérose en plaques (Peck A, Mellins ED. Precarious balance; Th17 cells in host defense. Infect Immun. 2010 Jan ; 78(1) :32-8 ; Suarez-Farinas: J. Allergy Clin. Immunol. 2014; J. Invest. Dermatol. 2008, 128(11), 2625).

En effet, les lymphocytes Th17 produisent de nombreuses cytokines ayant des profils distincts telles que l'interleukine-17A (IL-17A), l'interleukine-17F (IL-17F), l'interleukine-26 (IL-26), l'interleukine-21 (IL-21), l'interleukine-22 (IL-22) et le TNFα dont leur développement, leur survie et leur prolifération dépendent de l'interleukine-23 (IL-23). Ces cytokines sont capables d'activer différents types de cellules effectrices, telles que les kératinocytes, conduisant ainsi à leur hyperprolifération et à la production supplémentaire de cytokines pro-inflammatoires, de chimiokines et de peptides antimicrobiens, qui à leur tour recrutent et activent d'autres cellules du système immunitaire dans la peau enflammée, ce qui peut conduire à une amplification de la réponse inflammatoire.

Ainsi l'activation des lymphocytes Th17 est responsable du recrutement de cytokines, notamment d'interleukine-17 (IL17), et d'autres types de cellules pro-inflammatoires qui vont conduire à la médiation de désordres inflammatoires tels que l'acné et/ou de maladies auto-immunes telles que le psoriasis.

Des expériences menées sur des souris montrent qu'une diminution au niveau de l'expression du récepteur RORγt conduit à une baisse de l'activité des lymphocytes Th17 ce qui permet, par conséquent, de fortement réduire l'expression d'interleukine-17 (IL-17) (Ivanov II, McKenzie BS, Zhou L, Tadokoro CE, Lepelley A, Lafaille JJ, Cua DJ, Littman DR : Cell 2006, 126, 1121-1133) et de traiter efficacement les désordres inflammatoires et les maladies auto-immunes médiées par ces cytokines, notamment celles pour lesquelles des taux importants en interleukine-17 (IL-17) sont détectés.

A cet effet, la demande de brevet WO 2013/160418 décrit des composés sulfonamides utilisés comme agonistes inverses du récepteur RORγt afin de pouvoir traiter les désordres inflammatoires et les maladies auto-immunes. De la même façon, d'autres composés ont également été développés en tant qu'agonistes inverses du récepteur RORγt comme ceux décrits dans les demandes de brevet WO 2014/090712, WO 2014/008214, WO2013/169588, WO2013/160419, WO2013/1002027, WO2013/092939, WO2013/092941, WO2013/085890 et WO2012/ 100732.

Il existe donc un réel besoin de développer de nouveaux composés en tant qu'agonistes inverses du récepteur RORγt afin de pouvoir traiter efficacement les maladies médiées par un tel récepteur, notamment les désordres inflammatoires, tels que l'acné et/ou les maladies auto-immunes tels que le psoriasis ou la dermatite atopique.

Ce but est atteint grâce à la mise en oeuvre de dérivés sulfonamides bicycles particuliers tels que décrits ci-après qui permettent de moduler l'activité du récepteur RORγt et de traiter par conséquent efficacement les désordres inflammatoires et les maladies auto-immunes de certaines pathologies.

La présente invention a donc notamment pour objet un ou des composés de formule (I), leurs sels d'addition pharmaceutiquement acceptables, leurs hydrates et/ou leurs solvates :

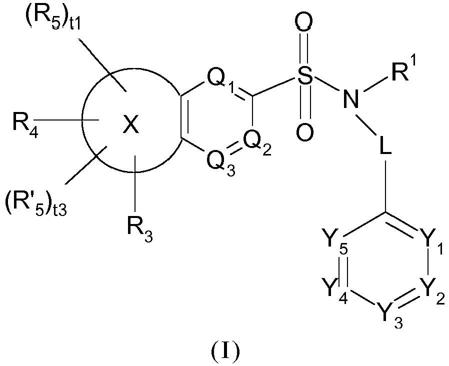

Formule (I) dans laquelle :
- L représente une liaison simple ou un groupe méthylène CH₂,
- X représente le radical cyclique suivant : 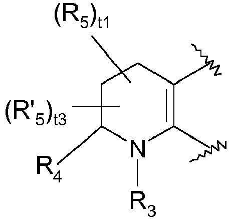
- un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
- un ou deux des éléments Q¹, Q² et Q³ représente(nt) un atome d'azote et le ou les autres éléments corresponde(nt) à un groupement-CR^{2a}, ou chacun des éléments Q¹, Q² et Q³ correspond à un groupement -CR^{2a} ,
- R¹ représente un radical alkyle, linéaire ou ramifié, en C₃-C₅, un radical cycloalkyle en C₃-C₅, un radical alkényle, linéaire ou ramifié, en C₂-C₅, un radical -CH₂-cycloalkyle en C₃-C₅, un radical hétérocycloalkyle en C₄-C₅, un radical -CH₂-hétérocycloalkyle en C₄-C₆,
- R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
- R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement-CH(R^{3a})OH, un groupement carboxylique -COOH, un groupement carbamoyle -CONR^{2c}R^{2d}, un groupement amido -NR^{2c}COR^{2d}, un groupement -SO₂R^{2c}, un groupement -SOR^{2c}, un groupement-S(=O)(=NH-R^{2c}), lesdits radicaux alkyle, alkényle et alcoxy pouvant être substitués par un ou plusieurs atomes d'halogène,
- R^{2c} et R^{2d}, identiques ou différents, représentent un atome d'hydrogène ou un radical alkyle, linéaire ou ramifié, en C₁-C₅ ;
- R^{3a} représente un atome d'hydrogène ou un radical alkyle, linéaire ou ramifié, en C₁-C₅ ;
- t₁ et t₃, identiques ou différents, désignent un entier naturel 0 ou 1,
- R³ et R⁴, identiques ou différents, représentent un atome d'hydrogène ou un groupement (CHR⁶)ₙ-(Z)ₒ-(CHR'⁶)ₚ-R⁷,
- n, o et p, identiques ou différents, représentent zéro ou un entier naturel allant de 1 à 3,
- lorsque R³ représente un groupement (CHR⁶)ₙ-(Z)ₒ-(CHR'⁶)ₚ-R⁷, alors R⁴ représente un atome d'hydrogène,
- lorsque R⁴ représente un groupement (CHR⁶)ₙ-(Z)ₒ-(CHR'⁶)ₚ-R⁷, alors R³ représente un atome d'hydrogène,
- Z représente un groupement divalent choisi parmi un groupement méthylène -CH₂-, un groupement amino -NH- et un atome d'oxygène -O-,
- R⁶ et R'⁶, identiques ou différents, représentent un atome d'hydrogène, un groupement méthyle -CH₃, un groupement -OH, un groupement hydroxyméthyle, une fonction carboxylique -COOH,
- R⁷ représente :
   - un atome d'hydrogène ou un atome d'halogène,
   - un groupement COOR'⁷ avec R'⁷ désignant alkyl(C₁)hétérocycle(C₆),
   - un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{8b}),
   - un radical cycloalkyle non cationique en C₃-C₆ éventuellement substitué par un ou plusieurs radicaux méthyle, un ou plusieurs atomes d'halogène, un groupement cyano -CN ou un ou plusieurs groupements -COR¹³; R¹³ désignant un radical alcoxy en C₁-C₃, linéaire ou ramifié, ou un groupement hydroxy,
   - un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido-CONR¹¹R¹², un ou plusieurs groupements -SOR¹¹, un ou plusieurs groupements -SO₂R , un ou plusieurs groupements-NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
- lorsque R³ ou R⁴ représente un groupement -CH=R⁷ ou un groupement -C=CH-R⁷ alors R⁷ ne représente pas un atome d'hydrogène, un atome d'halogène ou un groupement COOR'⁷,
- R⁵ représente un atome d'hydrogène ou un atome d'halogène, un radical alkyle en C₁-C₃, linéaire ou ramifié éventuellement substitué par un ou plusieurs atomes d'halogène; un radical amino -NH₂, un radical CH₂R'^{7a} avec R'^{7a} désignant un radical méthoxy, un groupement hydroxy -OH, un groupement -CH₂COOH, un groupement -CH(R^{5b})OH, un groupement carboxylique -COOH, un groupement -CN, une fonction thioxo,
- R'₅ représente une fonction carbonyle (C=O) ou une fonction thioxo (C=S),
- R^{8a} et R^{8b}, identiques ou différents, désignent un atome d'hydrogène, un radical alkyle, linéaire ou ramifié, en C₁-C₃ ou un radical cyclopropyle.

Le ou les composés selon l'invention correspondent ainsi à des dérivés sulfonamides bicycles, soit donc à un ou des composés sulfonamides comportant dans leur structure au moins deux cycles qui sont condensés l'un sur l'autre.

Conformément à la formule (I) selon l'invention, la liaison intracyclique entre le radical cyclique X, tel que représenté ci-avant, et le noyau atromatique comportant les éléments Q₁ à Q₃ est une liaison double commune aux deux cycles.

Les composés selon l'invention permettent de moduler, c'est-à-dire d'inhiber, l'activité du récepteur RORγt.

La présente invention a également pour objet le ou les composés tels que définis précédemment en tant que médicament et cosmétique.

Un autre objet de l'invention porte sur le ou les composés tels que définis précédemment pour leur utilisation dans le traitement des maladies médiées par le récepteur RORγt, notamment les désordres inflammatoires et/ou les maladies auto-immunes médiées par le récepteur RORγt.

Par ailleurs, l'invention concerne également une composition pharmaceutique comprenant, dans un milieu pharmaceutiquement acceptable, un ou plusieurs composés de formule (Ia) telle que définie précédemment, leurs sels d'addition pharmaceutiquement acceptables, leurs hydrates et/ou leurs solvates.

La présente invention a aussi trait à la composition pharmaceutique telle que décrite précédemment pour son utilisation dans le traitement des maladies médiées par le récepteur RORγt, notamment les désordres inflammatoires et/ou les maladies auto-immunes.

Enfin, l'invention est relative à une méthode de traitement des maladies médiées par le récepteur RORγt comprenant l'administration, notamment par voie topique ou orale, d'une quantité thérapeutiquement efficace d'un ou plusieurs composés tels que définis ci-avant à un patient.

D'autres objets et caractéristiques, aspects et avantages de l'invention apparaîtront encore plus clairement à la lecture de la description et des exemples qui suivent.

Selon un mode de réalisation, dans la formule (I), L représente une liaison simple.

Selon un autre mode de réalisation, dans la formule (I), L représente un groupe méthylène -CH₂.

Préférentiellement, dans la formule (I), L représente une liaison simple.

De préférence, R⁵ représente un groupement hydroxy -OH, un groupement -CH(R^{5b})OH, un groupement amino -NH₂, un groupement carboxylique -COOH, un atome d'halogène ou un groupement -CN.

Préférentiellement, R³ et R⁴, identiques ou différents, représentent un atome d'hydrogène ou un groupement (CHR⁶)ₙ-(Z)ₒ-(CHR'⁶)ₚ-R⁷.

Selon un mode de réalisation, dans la formule (I), R³ et R⁴ représentent un atome d'hydrogène.

Selon un mode de réalisation, dans la formule (I), R³ représente un groupement (CHR⁶)ₙ-(Z)ₒ-(CHR'⁶)ₚ-R⁷ et R⁴ représente un atome d'hydrogène.

Selon un mode de réalisation, dans la formule (I), R³ représente un atome d'hydrogène et R⁴ représente un groupement (CHR⁶)ₙ-(Z)ₒ-(CHR'⁶)ₚ-R⁷.

De préférence, R¹¹ et R¹² sont différents d'un groupement -OH.

Selon un mode de réalisation, dans la formule (I), les indices n, o, et p, identiques ou différents, désignent zéro.

Selon un mode de réalisation, dans la formule (I), les indices n, o, et p, identiques ou différents, désignent un entier naturel variant de 1 à 3.

Selon un mode de réalisation, dans la formule (I), les indices n et p désignent zéro et l'indice o vaut 1.

Selon un mode de réalisation, dans la formule (I), Z représente un groupe méthylène -CH₂.

Selon un mode de réalisation, dans la formule (I), Z représente un groupe divalent -O-.

Selon un mode de réalisation, dans la formule (I), Z représente un groupe divalent -NH-.

Selon un mode de réalisation, dans la formule (I), R³ représente un groupement Z-R⁷, avec Z ayant la signification précédemment décrite.

Selon un mode de réalisation particulier, dans la formule (I), R³ représente un groupement -CH₂-R⁷.

Selon un mode de réalisation particulier, dans la formule (I), R³ représente un groupement -O-R⁷.

Selon un mode de réalisation particulier, dans la formule (I), R³ représente un groupement -NH-R⁷.

Selon un mode de réalisation, dans la formule (I), R⁷ représente un radical hétérocyclique choisi parmi les hétérocycles suivants : 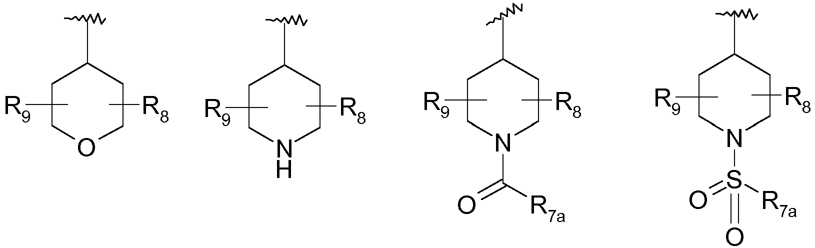 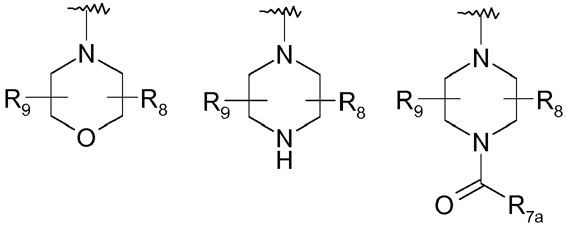 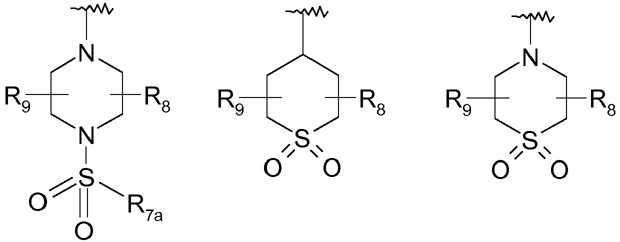 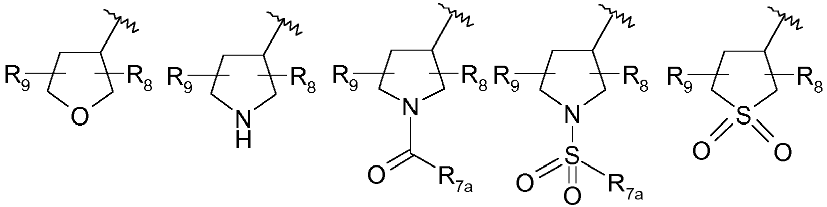 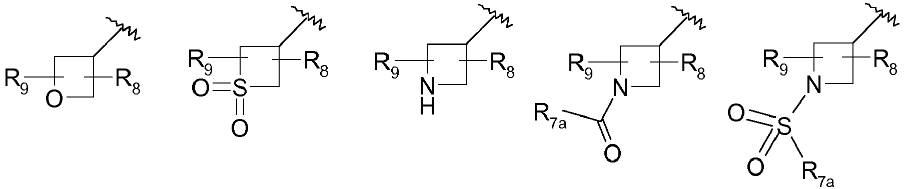 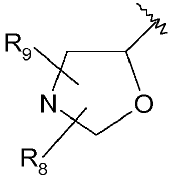 dans lesquels :
- R₇ₐ représente un radical alkyle, linéaire ou ramifié, en C₁-C₃, un radical alcoxy en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁ (-CH₂OH), un groupe amino NH₂,
- R₈ et R₉ peuvent former ensemble avec les atomes de carbone auxquels ils sont attachés un cycle carbocyclique comportant de 5 à 7 chaînons.

Selon un mode de réalisation, R⁷ peut aussi représenter un radical spiro bicyclique comportant un hétéroatome.

Selon un mode de réalisation, dans la formule (I), R⁷ représente un radical aromatique ou hétéroaromatique choisi parmi : 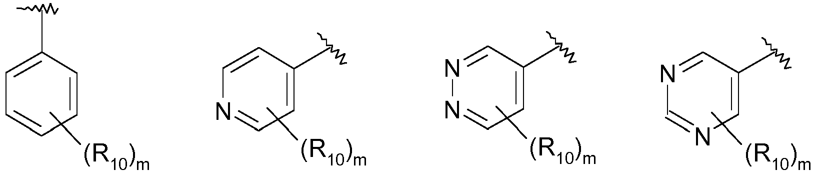 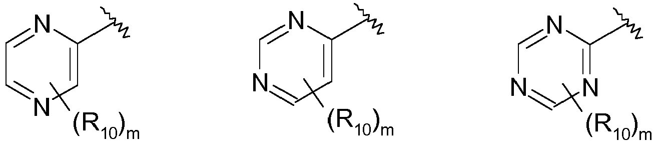 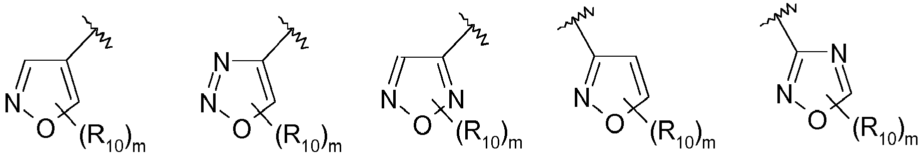 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁-C₃, un groupement amino -NR¹¹R¹², un groupement -COR¹¹, un groupement -COOR¹¹, un groupement amido-CONR¹¹R¹², un groupement -SOR¹¹, un groupement -SO₂R¹¹, un groupement -NHCOR¹¹, un groupement -NHCOOR¹¹, un groupement-SO₂NR¹¹R¹² ou un groupement -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
- m désigne zéro ou un entier naturel allant de 1 à 3.

De préférence, R¹¹ et R¹² sont différents d'un groupement -OH.

Préférentiellement, R⁷ représente un radical aromatique ou hétéroaromatique tel que défini précédemment éventuellement substitué par un ou plusieurs groupements méthyle -CH₃, un ou plusieurs groupements méthoxy -OCH₃, un ou plusieurs groupements hydroxy-OH, un ou plusieurs groupements amino -NH₂, un ou plusieurs groupements -CH₂OH, un ou plusieurs groupements cyano -CN, un ou plusieurs atomes d'halogène ou une ou plusieurs fonctions carbonyles.

Selon un mode de réalisation, l'indice m vaut zéro.

Selon un mode de réalisation, l'indice m désigne un entier naturel allant de 1 à 3.

Préférentiellement, l'indice m vaut 1.

Selon un mode de réalisation, dans la formule (I), chacun des éléments Y¹, Y², Y³, Y⁴ et Y⁵ correspond à un groupement -CR² avec R² ayant la même signification que celle décrite précédemment.

Selon un mode de réalisation, dans la formule (I), chacun des éléments Y¹, Y², Y³, Y⁴ et Y⁵ correspond à un groupement -CR² avec R² représentant un atome d'hydrogène.

Selon un mode de réalisation, dans la formule (I), chacun des éléments Y¹, Y², Y³, Y⁴ et Y⁵ correspond à un groupement -CR² avec R² représentant un radical alkyle, linéaire ou ramifié en C₁-C₅.

Selon un mode de réalisation, dans la formule (I), chacun des éléments Q¹, Q² et Q³ représente un groupement -CR^{2a} avec R^{2a} ayant la même signification que celle décrite précédemment.

Selon un mode de réalisation, dans la formule (I), chacun des éléments Q¹, Q² et Q³ représente un groupement -CR^{2a} avec R^{2a} représentant un atome d'hydrogène.

Selon un mode de réalisation, dans la formule (I), Q¹ et Q² représentent un groupement -CR^{2a} avec R^{2a} représentant un atome d'hydrogène et Q³ représente un groupement -CR^{2a} avec R^{2a} représentant un radical alkyle, linéaire ou ramifié, en C₁-C₅.

Selon un mode de réalisation, dans la formule (I), R¹ représente un radical alkyle, linéaire ou ramifié, en C₃-C₅, de préférence un radical alkyle ramifié en C₃-C₅, plus préférentiellement ramifié en C₄.

Selon un mode de réalisation, dans la formule (I), R¹ représente un radical cycloalkyle en C₃-C₅, de préférence cyclopropyle.

Selon un mode de réalisation, dans la formule (I), R¹ représente un radical alkényle, linéaire ou ramifié, en C₂-C₅.

Selon un mode de réalisation, dans la formule (I), R¹ représente un radical CH₂-cycloalkyle en C₃-C₅.

Selon un mode de réalisation, dans la formule (I), R¹ représente un radical hétérocycloalkyle en C₄-C₅.

Selon un mode de réalisation, dans la formule (I), R¹ représente un radical CH₂-hétérocycloalkyle en C₄-C₆, en particulier un radical CH₂-hétérocycloalkyle en C₄-C₅.

Préférentiellement, R¹ représente un radical alkyle, linéaire ou ramifié, en C₃-C₅ ou un radical CH₂-hétérocycloalkyle en C₄-C₅.

Selon un mode de réalisation, R⁵ représente un atome d'hydrogène.

Selon un mode de réalisation, R'⁵ représente une fonction carbonyle (C=O).

Plus préférentiellement, R³ représente un groupement CH₂-R⁷.

De préférence, le ou les composés de formule (I) est ou sont choisis parmi le ou les composés de formule (V), leurs sels d'addition pharmaceutiquement acceptables, leurs hydrates et/ou leurs solvates :

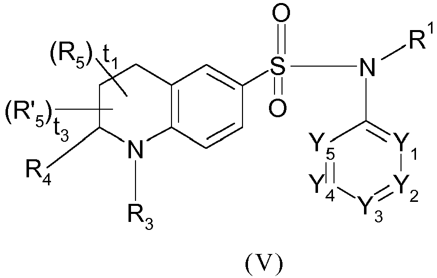

Formule (V) dans laquelle R¹, R³, R⁴, R⁵, R'⁵ et Y¹ à Y⁵, les indices t₁ et t₃ ont les mêmes significations que dans la formule (I) précédemment décrite.

Préférentiellement, dans la formule (V), R⁴ désigne un atome d'hydrogène.

Préférentiellement, dans la formule (V), lorsque t₁ est égal à zéro alors t₃ est égal à 1 et réciproquement lorsque t₁ est égal à 1 alors t₃ désigne zéro.

Selon un mode de réalisation, R'⁵ désigne une fonction carbonyle.

Selon un mode de réalisation, R⁵ représente un groupement hydroxy -OH.

Conformément à ces deux modes de réalisation, R⁴ désigne préférentiellement un atome d'hydrogène.

Selon un mode de réalisation préféré, le ou les composés de formule (V) est ou sont choisis parmi le ou les composés de formule (V) :

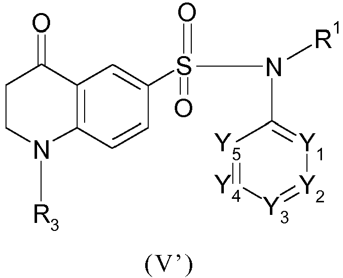

Formule (V') dans laquelle R¹, R³, et Y¹ à Y⁵ ont les mêmes significations que dans la formule (I) précédemment décrite.

Dans ce mode de réalisation, t₁ est égal à zéro, t₃ est égal à 1, R'⁵ désigne une fonction carbonyle et R⁴ représente un atome d'hydrogène.

Préférentiellement, R³ représente un groupement CH₂-R⁷.

Selon un mode de réalisation préféré, le ou les composés de formule (V) sont également choisis parmi le ou les composés de formule (V")

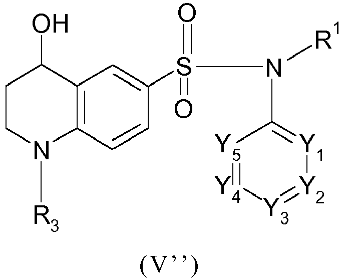

Formule (V") dans laquelle R¹, R³ et Y¹ à Y⁵ ont les mêmes significations que dans la formule (I) précédemment décrite.

Dans ce mode de réalisation, t₁ est égal à 1, t₃ est égal à zéro, R⁵ désigne un groupement hydroxy -OH et R⁴ représente un atome d'hydrogène

Préférentiellement, R³ représente un groupement CH₂-R⁷.

Ainsi le ou les composés de formule (I) selon l'invention est ou sont choisis parmi le ou les composés de formule (V).

Les composés de formule (I) peuvent se présenter sous la forme de sels pharmaceutiquement acceptables. Des exemples de sels pharmaceutiquement acceptables sont décrits dans Berge et al., 1977, «sels pharmaceutiquement acceptables», J. Pharm. Sci., Vol. 66, pp 1 - 19.

En particulier, lorsque que les composés de formule selon l'invention se présentent sous la forme de sels alors l'électroneutralité desdits composés est assurée par un contre ion cationique Y externe pouvant être organique ou minérale.

Y peut être choisi parmi les cations inorganiques appropriés tels que les ions de métaux alcalins, notamment Na⁺, K⁺, les ions métaux alcalino-terreux, notamment Ca²⁺, Mg²⁺, ou encore d'autres cations tels que l'ion aluminium Al³⁺.

Y peut être choisi parmi les cations organiques appropriés tels que l'ion ammonium NH₄⁺, les ions ammonium substitués tels que NH₃R⁺, NHR₂⁺, NR₄⁺ avec R représentant un radical alkyle en C₁-C₄.

En particulier, les ions ammonium substitués sont ceux choisis parmi les dérivés de l'éthylamine, la diéthylamine, la dicyclohexylamine, la triéthylamine, la butylamine, l'étylènediamine, l'éthanolamine, la diéthanolamine, la pipérazine, la benzylamine, la phénylbenzylamine, la choline, la meglumine, et trométhamine, les acides aminés tels que la lysine et l'arginine.

Un exemple d'un ion ammonium quaternaire peut être l'ion N⁺ (CH₃)₄.

Le ou les composés selon l'invention peuvent se présenter sous la forme de leurs solvates.

Au sens de la présente invention, le terme « solvate » signifie un complexe de soluté (c'est-à-dire le composé selon l'invention ou le sel dudit composé) et de solvant.

Si le solvant est l'eau alors le solvate peut être commodément considéré comme un hydrate, par exemple, un semi-hydrate, un monohydrate, un dihydrate, un trihydrate, etc.

Par exemple, les solvates et/ou hydrates peuvent être obtenus directement à la fin du processus de synthèse, le composé cible étant isolé sous la forme d'un hydrate, par exemple un monohydrate ou hémi-hydrate, ou sous la forme d'un solvate du solvant de réaction et/ou du solvant de purification.

Sauf indication contraire, toute référence à un composé selon l'invention inclut également le solvate ou l'hydrate du composé correspondant.

Des procédures typiques pour la préparation et l'identification des hydrates et des solvates sont bien connus de l'homme du métier, voir par exemple, pages 202-209 de KJ Guillory, « Génération of Polymorphs, Hydrates, Solvates, and Amorphous Solids » dans Polymorphism in Pharmaceutical Solids, édition. Harry G. Britain, Vol. 95, Marcel Dekker, Inc., New York, 1999.

Les hydrates et les solvates peuvent être isolés et caractérisés par des méthodes connues dans l'art telles que l'analyse thermogravimétrique (TGA), la spectroscopie TGA-masse, la spectroscopie TGA-infrarouge, la diffraction de poudres aux rayons X, le titrage de Karl Fisher, la diffraction haute résolution aux rayons X et analogue.

De préférence, le ou les composés de formule (I) sont choisis parmi les composés tels que décrits dans les tableaux ci-après, ainsi que leurs sels d'addition pharmaceutiquement acceptable, leurs hydrates et/ou leurs solvates :

**Tableau 1 :**

| | | IC50 hRORg | IC50 hCD4/IL17 |
|---|---|---|---|
| 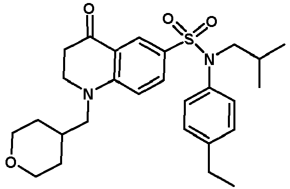 | Acide 4-oxo-1-(tetrahydro-pyran-4-ylmethyl)-1,2,3,4-tetrahydro-quinoline-6-sulfonique (4-éthyl-phényl)-isobutyl-amide | B | B |
| | Composé 1 | | |
| 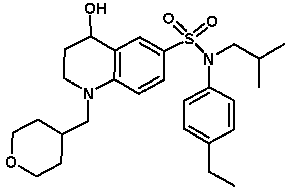 | Acide 4-hydroxy-1-(tetrahydro-pyran-4-ylmethyl)-1,2,3,4-tetrahydro-quinoline-6-sulfonique (4-éthyl-phényl)- isobutyl-amide | A | B |
| | Composé 2 | | |
| Enantiomère 1 | | A | A |
| Enantiomère 2 | | B | A |
| 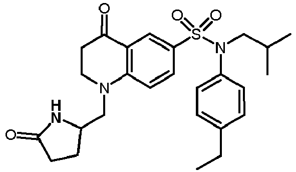 | Acide 4-oxo-1-(5-oxo-pyrrolidin-2-ylméthyl)-1,2,3,4-tétrahydro-quinoline-6-sulfonique (4-éthyl-phényl)-isobutyl-amide | C | ND |
| | Composé 3 | | |
| 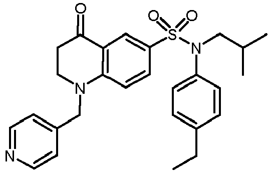 | Acide 4-oxo-1-pyridin-4-ylméthyl-1,2,3,4-tétrahydro-quinoline-6-sulfonique (4-éthyl-phényl)-isobutyl-amide | A | A |
| | Composé 4 | | |
| 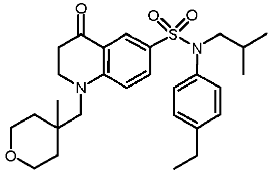 | Acide 1-(4-méthyl-tétrahydro-pyran-4-ylméthyl)-4-oxo-1,2,3,4-tétrahydro-quinoline-6-sulfonique (4-éthyl-phényl)-isobutyl-amide | B | ND |
| | Composé 6 | | |
| 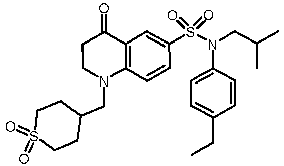 | Acide 1-(1,1-dioxo-hexahydro-1 λ⁶-thiopyran-4-ylmethyl)-4-oxo-1,2,3,4-tetrahydro-quinoline-6-sulfonique (4-éthyl-phényl)- isobutyl-amide | C | ND |
| | Composé 7 | | |
| 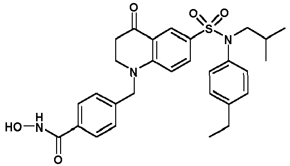 | 4-{6-[(4-éthyl-phényl)-isobutyl-sulfamoyl]-4-oxo-3,4-dihydro-2H-quinolin-1-ylméthyl}-N-hydroxy-benzamide | C | ND |
| | Composé 8 | | |
| 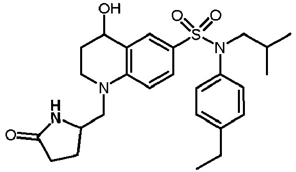 | Acide 4-hydroxy-1-(5-oxo-pyrrolidin-2-ylméthyl)-1,2,3,4-tetrahydro-quinoline-6-sulfonique (4-éthyl-phényl)-isobutyl-amide | B | ND |
| | Composé 9 | | |
| 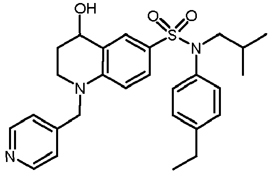 | Acide 4-hydroxy-1-pyridin-4-ylméthyl-1,2,3,4-tétrahydro-quinoline-6-sulfonique (4-éthyl-phényl)-isobutyl-amide | A | A |
| | Composé 10 | | |
| 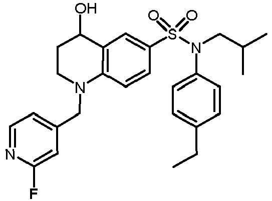 | Acide 1-(2-fluoro-pyridin-4-ylméthyl)-4-hydroxy-1,2,3,4-etrahydro-quinoline-6-sulfonique (4-éthyl-phényl)-isobutyl-amide | A | A |
| | Composé 11 | | |
| | | | |
| 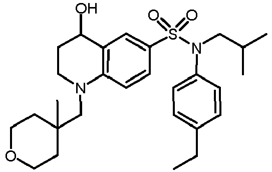 | Acide 1-(4-méthyl-tétrahydro-pyran-4-ylméthyl)-4-hydroxy-1,2,3,4-tétrahydro-quinoline-6-sulfonique (4-éthyl-phényl)- isobutyl-amide | A | A |
| | Composé 14 | | |
| 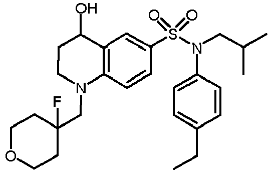 | Acide 1-(4-fluoro-tétrahydro-pyran-4-ylméthyl)-4-hydroxy-1,2,3,4-tétrahydro-quinoline-6-sulfonique (4-éthyl-phényl)- isobutyl-amide | B | A |
| | Composé 15 | | |
| 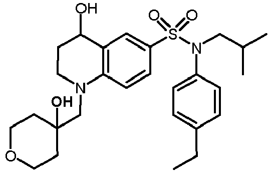 | Acide 4-hydroxy-1-(4-hydroxy-tétrahydro-pyran-4-ylméthyl)-1,2,3,4-tétrahydro-quinoline-6-sulfonique (4-éthyl-phényl)-isobutyl-amide | C | ND |
| | Composé 16 | | |
| 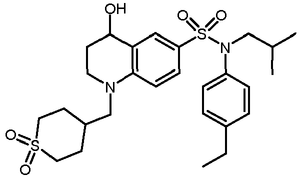 | Acide 1-(1,1-dioxo-hexahydro-1 λ⁶-thiopyran-4-ylméthyl)-4-hydroxy-1,2,3,4-tetrahydro-quinoline-6-sulfonique (4-éthyl-phényl)-isobutyl-amide | C | ND |
| | Composé 17 | | |
| 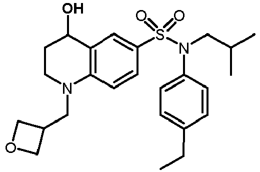 | Acide 4-hydroxy-1-oxetan-3-ylméthyl-1,2,3,4-tétrahydro-quino line-6-sulfonique (4-éthyl-phényl)-isobutyl-amide | B | ND |
| | Composé 18 | | |
| 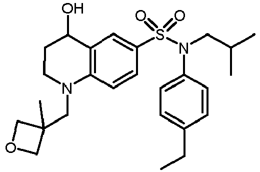 | Acide 4-hydroxy-1-(3-méthyl-oxétan-3-ylméthyl)-1,2,3,4-tetrahydro-quinoline-6-sulfonique (4-éthyl-phényl)- isobutyl-amide | B | A |
| | Composé 19 | | |
| 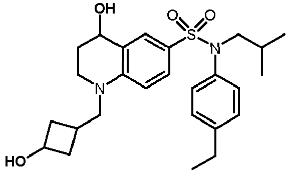 | Acide 4-hydroxy-1-(3-hydroxy-cyclobutylméthyl)-1,2,3,4-tetrahydro-quinoline-6-sulfonique (4-éthyl-phényl)-isobutyl-amide mélange de diastéréoisomères : | B | A |
| | Composé 20 | | |
| 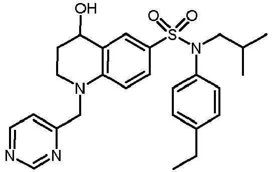 | Acide 4-hydroxy-1-pyrimidin-4-ylméthyl-1,2,3,4-tétrahydro-quinoline-6-sulfonique (4-éthyl-phényl)-isobutyl-amide | A | ND |
| | Composé 21 | | |
| 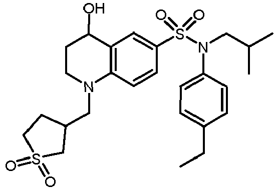 | Acide 1-(1,1-Dioxo-tétrahydro-1λ⁶ -thiophen-3-ylméthyl)-4-hydroxy-1,2,3,4-tétrahydro-quinoline-6-sulfonique (4-éthyl-phényl)-isobutyl-amide | B | ND |
| | Composé 22 | | |
| 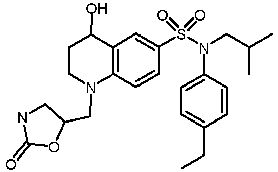 | Acide 4-hydroxy-1-(2-oxo-oxazolidin-5-ylméthyl)-1,2,3,4-tétrahydro-quinoline-6-sulfonique (4-éthyl-phényl)-isobutyl-amide | B | ND |
| | Composé 23 | | |
| 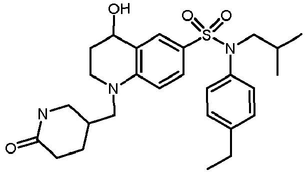 | Acide 4-hydroxy-1-(6-oxo-piperidin-3-ylméthyl)-1,2,3,4-tétrahydro-quinoline-6-sulfonique (4-éthyl-phényl)-isobutyl-amide | B | ND |
| | Composé 24 | | |
| 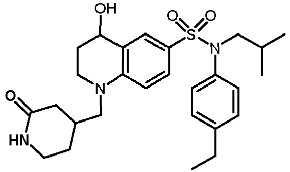 | Acide 4-hydroxy-1-(2-oxo-piperidin-4-ylmethyl)-1,2,3,4-tétrahydro-quinoline-6-sulfonique (4-éthyl-phényl)-isobutyl-amide | C | ND |
| | Composé 25 | | |
| 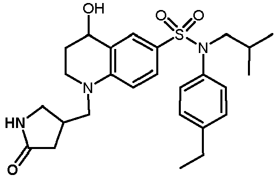 | Acide 4-hydroxy-1-(5-oxo-pyrrolidin-3-ylméthyl)-1,2,3,4-tétrahydro-quinoline-6-sulfonique (4-éthyl-phényl)-isobutyl-amide | B | ND |
| | Composé 26 | | |
| 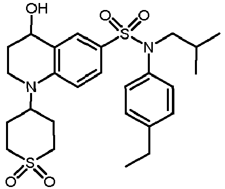 | Acide 1-(1,1-dioxo-hexahydro-1 λ⁶-thiopyran-4-yl)-4-hydroxy-1,2,3,4-tétrahydro-quinoline-6-sulfonique (4-éthyl-phényl)- isobutyl-amide | B | ND |
| | Composé 27 | | |
| 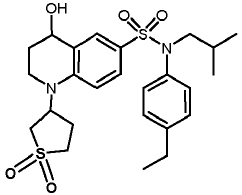 | Acide 1-(1,1-dioxo-tétrahydro-1λ⁶-thiophen-3-yl)-4-hydroxy-1,2,3,4-tétrahydro-quinoline-6-sulfonique (4-éthyl-phényl)-isobutyl-amide | B | ND |
| | Composé 28 | | |
| 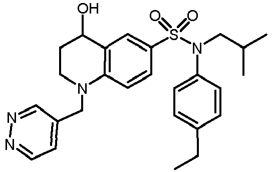 | Acide 4-hydroxy-1-pyridazin-4-ylmethyl-1,2,3,4-tetrahydro-quinoline-6-sulfonique (4 éthyl-phényl)- isobutyl-amide | A | A |
| | Composé 29 | | |
| 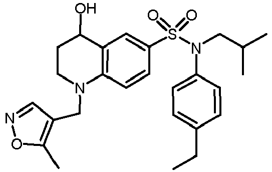 | Acide 4-hydroxy-1-(5-methyl-isoxazol-4-ylméthyl)-1,2,3,4-tetrahydro-quinoline-6-sulfonique (4-éthyl-phényl)-isobutyl-amide | B | ND |
| | Composé 30 | | |
| 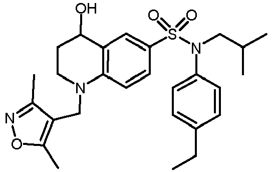 | Acide 1-(3,5-Diméthyl-isoxazol-4-ylmethyl)-4-hydroxy-1,2,3,4-tetrahydro-quinoline-6-sulfonique (4-éthyl-phényl)- isobutyl-amide | B | ND |
| | Composé 31 | | |
| 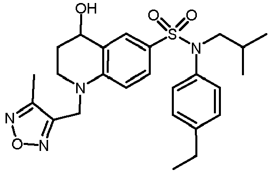 | Acide 4-hydroxy-1-(4-methyl-furazan-3-ylmethyl)-1,2,3,4-tetrahydro-quinoline-6-sulfonique (4-éthyl-phényl)-isobutyl-amide | B | ND |
| | Composé 32 | | |
| 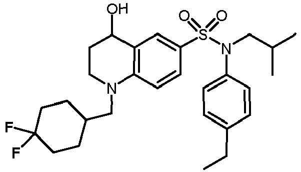 | Acide 1-(4,4-difluoro-cyclohexylmethyl)-4-hydroxy-1,2,3,4-tetrahydro-quinoline-6-sulfonique (4-éthyl-phényl)- isobutyl-amide | C | ND |
| | Composé 33 | | |
| 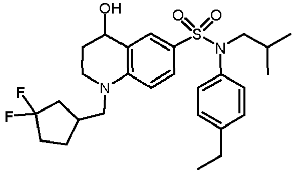 | Acide 1-(3,3-difluoro-cyclopentylmethyl)-4-hydroxy-1,2,3,4-tetrahydro-quinoline-6-sulfonique (4-éthyl-phényl)- isobutyl-amide | B | ND |
| | Composé 34 | | |
| 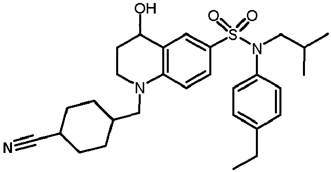 | Acide 1-(4-cyano-cyclohexylmethyl)-4-hydroxy-1,2,3,4-tetrahydro-quinoline-6-sulfonique (4-éthyl-phényl)- isobutyl-amide | C | ND |
| | Composé 35 | | |
| 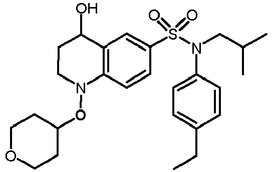 | Acide 4-hydroxy-1-(tétrahydro-pyran-4-yloxy)-1,2,3,4-tétrahydro-quinoline-6-sulfonique (4-éthyl-phényl)- isobutyl-amide | B | ND |
| | Composé 36 | | |
| 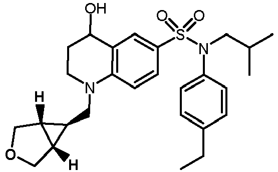 | Acide 4-hydroxy-1-[(1S,5R,6S)-1-(3-oxa-bicyclo-[3.1.0]hex-6-yl)methyl]-1,2,3,4-tetrahydro-quinoline-6-sulfonique (4-éthyl-phényl)-isobutyl-amide | A | A |
| | Composé 37 | | |
| 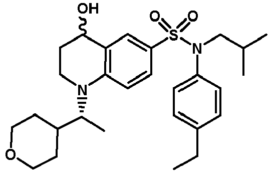 | Acide 4-hydroxy-1-[(R)-1-(tétrahydro-pyran-4- yl)-ethyl]-1,2,3,4-tétrahydro-quinoline-6-sulfonique (4-éthyl-phényl)- isobutyl-amide | C | ND |
| | Composé 38 | | |
| 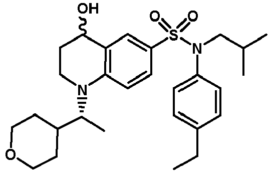 | Acide 4-hydroxy-1-[(R)-1-(tétrahydro-pyran-4- yl)-éthyl]-1,2,3,4-tétrahydro-quinoline-6-sulfonique (4-éthyl-phényl)- isobutyl-amide | C | ND |
| | Composé 39 | | |
| 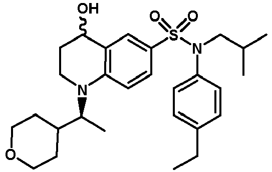 | Acide 4-hydroxy-1-[(S)-1-(tétrahydro-pyran-4-yl)-éthyl]-1,2,3,4-tétrahydro-quinoline-6-sulfonique (4-éthyl-phényl)- isobutyl-amide | B | ND |
| | Composé 40 | | |
| 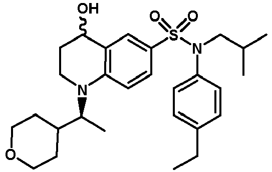 | 4-hydroxy-1-[(S)-1-(tétrahydro-pyran-4-yl)-éthyl]-1,2,3,4-tetrahydro-quinoline-6-sulfonique (4-éthyl-phényl)- isobutyl-amide | B | ND |
| | Composé 41 | | |
| 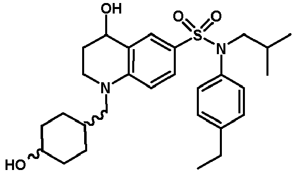 | Acide 4-hydroxy-1-(4-hydroxy-cyclohexylmethyl)-1,2,3,4-tetrahydro-quinoline-6-sulfonique (4-éthyl-phényl)-isobutyl-amide | B | ND |
| | Composé 42 | | |
| 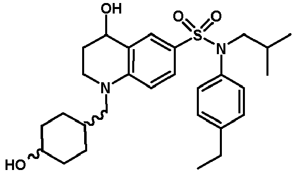 | Acide 4-hydroxy-1-(4-hydroxy-cyclohexylmethyl)-1,2,3,4-tetrahydro-quinoline-6-sulfonique (4-éthyl-phényl)-isobutyl-amide | B | ND |
| | Composé 43 | | |
| 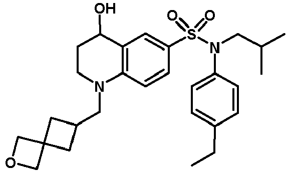 | Acide 4-hydroxy-1-(2-oxa-spiro[3.3]hept-6-ylmethyl)-1,2,3,4-tetrahydro-quinoline-6-sulfonique (4-éthyl-phényl)- isobutyl-amide | B | ND |
| | Composé 44 | | |
| 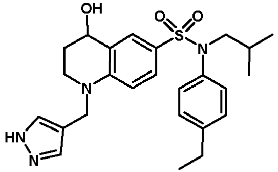 | Acide 4-hydroxy-1-(1H-pyrazol-4-ylméthyl)-1,2,3,4-tétrahydro-quinoline-6-sulfonique (4-éthyl-phényl)- isobutyl-amide | B | A |
| | Composé 45 | | |
| 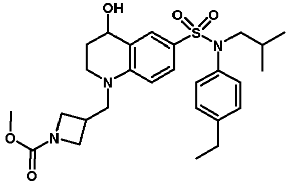 | Ester de l'acide méthyl 3-{6-[(4-éthyl-phényl)-isobutyl-sulfamoyl] -4-hydroxy-3,4-dihydro-2H-quinolin-1-ylméthyl}-azetidine-1 -carboxylique | B | B |
| | Composé 46 | | |
| 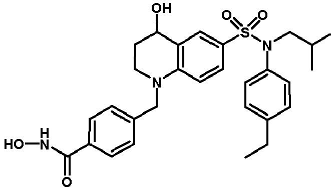 | 4-{6-[(4-éthyl-phényl)-isobutyl-sulfamoyl]-4-hydroxy-3,4-dihydro-2H-quinolin-1-ylméthyl}-N-hydroxy-benzamide | C | ND |
| | Composé 47 | | |
| 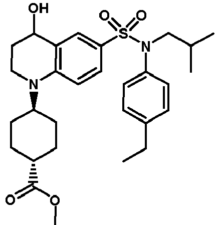 | méthyl (1r,4r)-4-(6-(N-(4-éthylphényl)-N-iso butylsulfamoyl)-4-hydroxy-3,4-dihydroquinolin-1(2H)-yl)cyclohexane-1-carboxylate | C | ND |
| | Composé 48 | | |
| 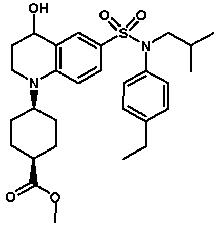 | Méthyl (1s,4s)-4-(6-(N-(4-ethylphenyl)-N-isobutylsulfamoyl)-4-hydroxy-3,4-dihydroquinolin-1(2H)-yl)cyclohexane-1-carboxylate | C | ND |
| | Composé 49 | | |
| 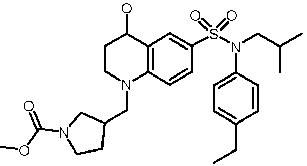 | Ester de l'acide méthyl carboxylique 3-{6-[(4-éthyl-phényl)-isobutyl-sulfamoyl]-4-hydroxy-3,4-dihydro-2H-quinolin-1-ylméthyl}-pyrrolidine-1 | C | ND |
| | Composé 50 | | |
| 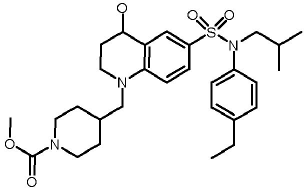 | Ester de l'acide méthyl carboxylique 4-{6-[(4-Ethyl-phenyl)-isobutyl-sulfamoyl]-4-hydroxy-3,4-dihydro-2H-quinolin-1-ylméthyl}-piperidine-1 | C | ND |
| | Composé 51 | | |
| 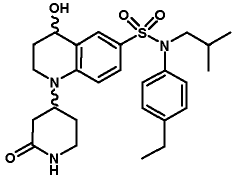 | **Diastéréoisomère 1** Acide 4-hydroxy-1-(2-oxo-pipéridin-4-yl)-1,2,3,4-tétrahydro-quinoline-6-sulfonique (4-éthyl-phényl)-isobutyl-amide | C | ND |
| | Composé 52 | | |
| 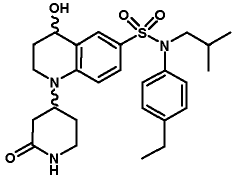 | **Diastéréoisomère 2** Acide 4-Hydroxy-1-(2-oxo-piperidin-4-yl)-1,2,3,4-tétrahydro-quinoline-6-sulfonique (4-éthyl-phényl)-isobutyl-amide | C | ND |
| | Composé 53 | | |
| 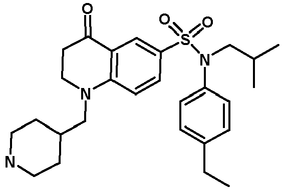 | Acide 4-oxo-1-piperidin-4-ylméthyl-1,2,3,4-tétrahydro-quinoline-6-sulfonique (4-éthyl-phényl)-isobutyl-amide | C | ND |
| | Composé 54 | | |
| 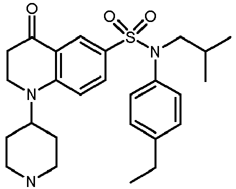 | Acide 4-oxo-1-piperidin-4-yl-1,2,3,4-tétrahydro-quinoline-6-sulfonique (4-éthyl-phényl)-isobutyl-amide | C | ND |
| | Composé 55 | | |
| 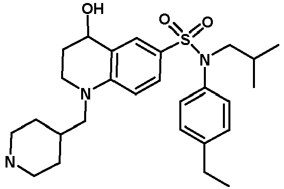 | Acide 4-hydroxy-1-piperidin-4-ylmethyl-1,2,3,4-tetrahydro-quinoline-6-sulfonique (4-éthyl-phényl)-isobutyl-amide | C | ND |
| | Composé 56 | | |
| 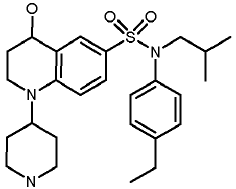 | Acide 4-hydroxy-1-piperidin-4-yl-1,2,3,4-tetrahydro-quinoline-6-sulfonique (4-éthyl-phényl)-isobutyl-amide | C | ND |
| | Composé 57 | | |
| 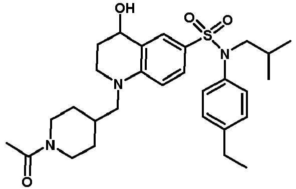 | Acide 4-hydroxy-1-piperidin-4-ylméthyl-1,2,3,4-tetrahydro-quinoline-6-sulfonique (4-éthyl-phényl)- isobutyl-amide | C | ND |
| | Composé 58 | | |
| 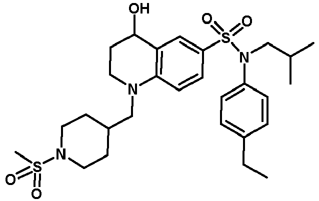 | Acide 4-hydroxy-1-pipéridin-4-ylméthyl-1,2,3,4-tétrahydro-quinoline-6-sulfonique (4-éthyl-phényl)- isobutyl-amide | C | ND |
| | Composé 59 | | |
| 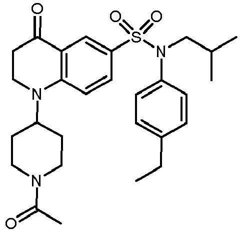 | Acide 1-(1-acétyl-pipéridin-4-yl)-4-oxo-1,2,3,4-tétrahydro-quino line-6-sulfonique (4-éthyl-phényl)-isobutyl-amide | C | ND |
| | Composé 60 | | |
| 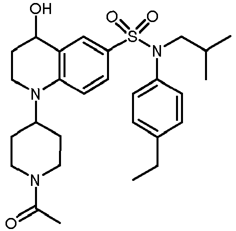 | Acide 1-(1-acétyl-piperidin-4-yl)-4-hydroxy-1,2,3,4-tetrahydro-quinoline-6-sulfonique (4-éthyl-phényl)-isobutyl-amide | C | ND |
| | Composé 61 | | |
| 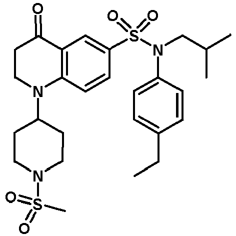 | Acide 1-(1-acetyl-piperidin-4-yl)-4-oxo-1,2,3,4-tetrahydro-quinoline-6-sulfonique (4-ethyl-phenyl)-isobutyl-amide | C | ND |
| | Composé 63 | | |
| 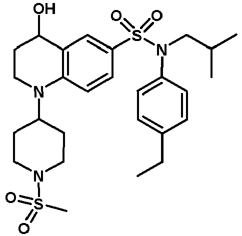 | Acide 4-hydroxy-1-(1-methanesulfonyl-piperidin-4-yl)-1,2,3,4-tétrahydro-quinoline-6-sulfonique (4-éthyl-phényl)-isobutyl-amide | C | ND |
| | Composé 64 | | |
| 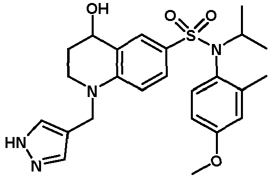 | Acide 4-hydroxy-1-(1H-pyrazol-4-ylmethyl)-1,2,3,4-tetrahydro-quinoline-6-sulfonique isopropyl-(4-méthoxy-2-methyl-phényl)-amide | C | ND |
| | Composé 65 | | |
| 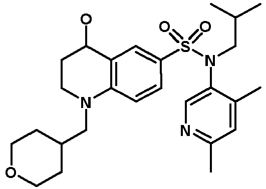 | Acide 4-Hydroxy-1-(tétrahydro-pyran-4-ylméthyl)-1,2,3,4-tétrahydro-quinoline-6-sulfonique (4,6-diméthyl-pyridin-3-yl)-isobutyl-amide | C | C |
| | Composé 66 | | |

| | | | |
|---|---|---|---|
| ND : non déterminé ; A : IC50 <100 nM.; B : IC50 = 100nM-1µM; C : IC50 > 1µM | | | |

Dans les tableaux décrits ci-avant, les concentrations inhibitrices médianes IC₅₀ pour les composés appartenant à la formule (I) selon l'invention ont été données selon les modèles suivants :

### Transactivation GAL4-RORγ

Le modèle de transactivation RORγ a été développé à partir de la lignée HG5LN qui est une lignée HeLa exprimant stablement un gène reporter luciférase contrôlé par un pentamère du domaine de reconnaissance GAL4 de levure et d'un promoteur β-globine. La lignée HG5LN a été transfectée stablement par le DNA-binding domain (DBD) (ou domaine de liaison à l'ADN) de GAL4 fusionné au ligand-binding domain (LBD) ROR gamma. Les molécules inhibant l'activité constitutive ROR gamma réduisent l'expression de la luciférase induisant ainsi une baisse de la luminescence émise.

Les cellules sont ensemencées en plaques 384 puits (5 000 cellules dans 45µL/puits de milieu de culture contenant 10% de sérum de veau foetal) et incubées pendant 4 heures à 37°C, 5% CO₂. 5µL des molécules à tester (composés décrits dans les tableaux décrits ci-avant) sont ensuite ajoutés à chaque puits et les plaques sont incubées pendant 18 heures à une température de 37°C sous 5% de CO₂. 20µL du substrat de la luciférase (Promega) sont ajoutés à chaque puits et la luminescence émise est lue par un lecteur de microplaques.

Les unités de luminescence ('RLU') sont normalisées par des contrôles positifs ('POS' contenant une concentration saturante de benzenesulfonamide, N-(2,2,2-trifluoroéthyl)-*N*-[4-[2,2,2-trifluoro-1-hydroxy-1-(trifluorométhyl)éthyl]phényl]) et des contrôles négatifs ('NEG' contenant du DMSO) : % inhibition=((RLU-NEG)*100)/(POS-NEG). Les IC50 sont calculées à partir d'un modèle logistique à 4 paramètres à l'aide du logiciel XLFit (IDBS).

### Sécrétion IL-17A

Ce modèle permet de mesurer l'effet d'inhibiteurs sur la sécrétion d'IL-17A par des cellules CD4+. Les cellules sont des CD4+ congelées (STEMCELL, # 70026), isolées de sang humain périphérique et activées par les anticorps anti-CD3 et anti-CD28. La quantité d'IL-17A sécrété est mesurée par la technologie TR-FRET (kit HTRF® Human Interleukin 17A (Cisbio, #64H17PEC)).

Les cellules sont décongelées rapidement, resuspendues dans leur milieu de culture (RPMI 10% SVF inactivé) supplémenté avec des anticorps anti-CD28 solubles et ensemencées (100 000 cellules/puits) dans des plaques 96 puits préalablement coatées avec des anticorps anti-CD3. Les cellules sont ensuite traitées par les gammes des inhibiteurs à tester (de 1000nM à 0.05nM, 0.1% DMSO). Après 4 jours d'incubation, le signal HTRF est mesuré à l'aide d'un lecteur de microplaque (λexcitation=337nm, λemission=620/665nm). Les ratios obtenus (665/620) sont normalisés par rapport au contrôle positif (cellules activées par anti-CD3 et anti-CD28, 0.1% DMSO). Les IC₅₀ sont calculées à partir d'un modèle logistique à 4 paramètres à l'aide du logiciel XLFit (IDBS).

Préférentiellement, les composés de formule (I) selon l'invention sont choisis parmi les composés suivants :

**Tableau 3 :**

| | |
|---|---|
| 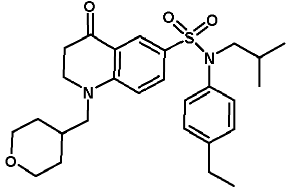 | Acide 4-oxo-1-(tetrahydro-pyran-4-ylmethyl)-1,2,3,4-tetrahydro-quinoline-6-sulfonique (4-éthyl-phényl)-isobutyl-amide |
| | Composé 1 |
| 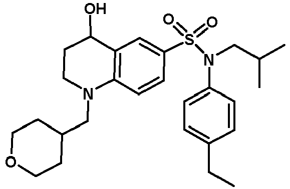 | Acide 4-hydroxy-1-(tetrahydro-pyran-4-ylmethyl)-1 ,2,3,4-tetrahydro-quino line-6-sulfonique (4-éthyl-phényl)-isobutyl-amide |
| | Composé 2 |
| 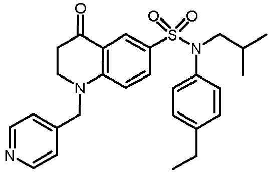 | Acide 4-oxo-1-pyridin-4-ylméthyl-1,2,3,4-tétrahydro-quinoline-6-sulfonique (4-éthyl-phényl)-isobutyl-amide |
| | Composé 4 |
| 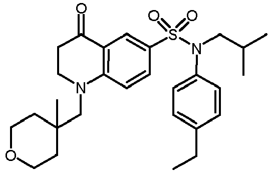 | Acide 1-(4-méthyl-tétrahydro-pyran-4-ylméthyl)-4-oxo-1 ,2,3,4-tétrahydro-quinoline-6-sulfonique (4-éthyl-phényl)-isobutyl-amide |
| | Composé 6 |
| 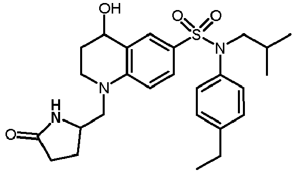 | Acide 4-hydroxy-1-(5-oxo-pyrrolidin-2-ylméthyl)-1,2,3,4-tetrahydro-quinoline-6-sulfonique (4- éthyl-phényl)-iso but yl-amide |
| | Composé 9 |
| 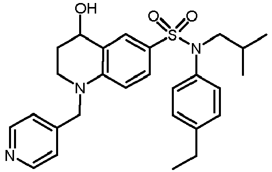 | Acide 4-hydroxy-1-pyridin-4-ylméthyl-1,2,3,4-tétrahydro-quinoline-6-sulfonique (4-éthyl-phényl)-isobutyl-amide |
| | Composé 10 |
| 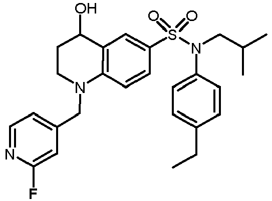 | Acide 1-(2-fluoro-pyridin-4-ylmethyl)-4-hydroxy-1,2,3,4-tetrahydro-quinoline-6-sulfonique (4-éthyl-phényl)-isobutyl-amide |
| | Composé 11 |
| 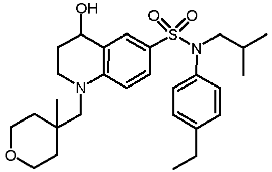 | Acide 1-(4-méthyl-tétrahydro-pyran-4-ylméthyl)-4-hydroxy-1,2,3,4-tétrahydro-quinoline-6-sulfonique (4-éthyl-phényl)-isobutyl-amide |
| | Composé 14 |
| 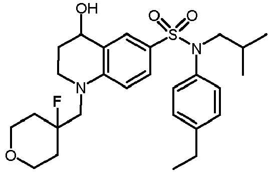 | Acide 1-(4-fluoro-tétrahydro-pyran-4-ylméthyl)-4-hydroxy-1,2,3,4-tétrahydro-quinoline-6-sulfonique (4-éthyl-phényl)-isobutyl-amide |
| | Composé 15 |
| 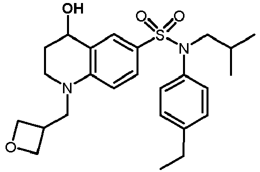 | Acide 4-hydroxy-1-oxetan-3-ylméthyl-1,2,3,4-tétrahydro-quinoline-6-sulfonique (4-éthyl-phényl)-isobutyl-amide |
| | Composé 18 |
| 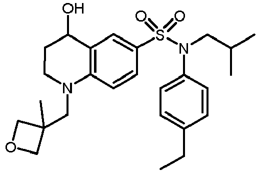 | Acide 4-hydroxy-1-(3-méthyl-oxetan-3-ylméthyl)-1,2,3,4-tetrahydro-quinoline-6-sulfonique (4-éthyl-phényl)-isobutyl-amide |
| | Composé 19 |
| 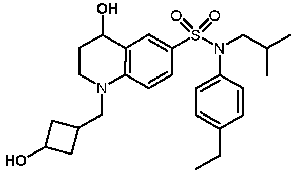 | Acide 4-hydroxy-1-(3-hydroxy-cyclobutylmethyl)-1,2,3,4-tetrahydro-quinoline-6- sulfonique (4-éthyl-phényl)-iso butyl-amide mélange de diastéréoisomères : |
| | Composé 20 |
| 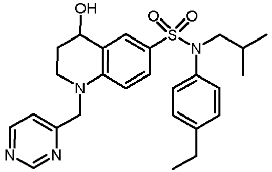 | Acide 4-hydroxy-1-oxetan-3-ylméthyl-1,2,3,4-tétrahydro-quinoline-6-sulfonique (4-éthyl-phényl)-isobutyl-amide |
| | Composé 21 |
| 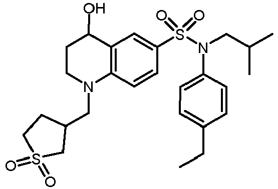 | Acide 1-(1,1-Dioxo-tétrahydro-1λ⁶-thiophen-3-ylméthyl)-4-hydroxy-1,2,3,4-tétrahydro-quinoline-6-sulfonique (4-éthyl-phényl)-isobutyl-amide |
| | Composé 22 |
| 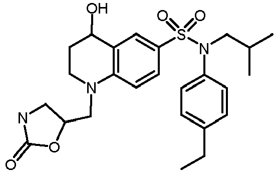 | Acide 4-hydroxy-1-(2-oxo-oxazolidin-5-ylméthyl)-1,2,3,4-tétrahydro-quinoline-6-sulfonique (4-éthyl-phényl)-isobutyl-amide |
| | Composé 23 |
| 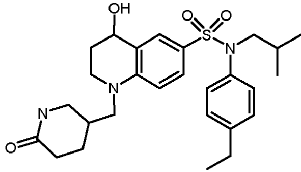 | Acide 4-hydroxy-1-(6-oxo-piperidin-3-ylméthyl)-1,2,3,4-tétrahydro-quinoline-6-sulfonique (4-éthyl-phényl)-isobutyl-amide |
| | Composé 24 |
| 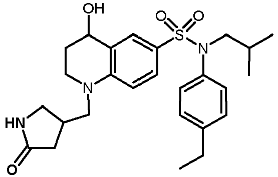 | Acide 4-hydroxy-1-(5-oxo-pyrrolidin-3-ylméthyl)-1,2,3,4-tétrahydro-quinoline-6-sulfonique (4-éthyl-phényl)-isobutyl-amide |
| | Composé 26 |
| 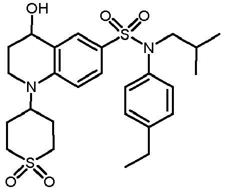 | Acide 1-(1,1-dioxo-hexahydro-1λ⁶-thiopyran-4-yl)-4-hydroxy-1,2,3,4-tétrahydro-quinoline-6-sulfonique (4-éthyl-phényl)-isobutyl-amide |
| | Composé 27 |
| 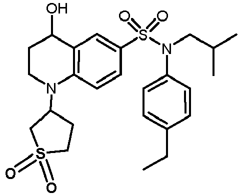 | Acide 1-(1,1-dioxo-tétrahydro-1lambda*6*-thiophen-3-yl)-4-hydroxy-1,2,3,4-tétrahydro-quinoline-6-sulfonique (4-éthyl-phényl)-isobutyl-amide |
| | Composé 28 |
| 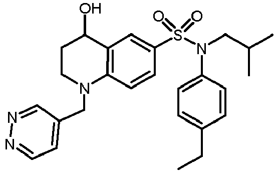 | Acide 4-hydroxy-1-pyridazin-4-ylmethyl-1,2,3,4-tetrahydro-quinoline-6-sulfonique (4 éthyl-phényl)-isobutyl-amide |
| | Composé 29 |
| 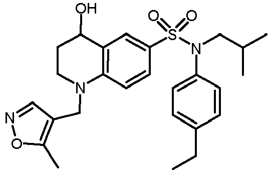 | Acide 4-hydroxy-1-(5-methyl-isoxazol-4-ylméthyl)-1,2,3,4-tetrahydro-quinoline-6-sulfonique (4-éthyl-phényl)-isobutyl-amide |
| | Composé 30 |
| 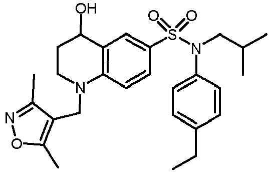 | Acide 1-(3,5-Diméthyl-isoxazol-4-ylmethyl)-4-hydroxy-1,2,3,4-tetrahydro-quino line-6-sulfonique (4-éthyl-phényl)-isobutyl-amide |
| | Composé 31 (Composé 235) |
| 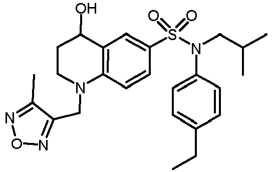 | Acide 4-hydroxy-1-(4-methyl-furazan-3-ylmethyl)-1,2,3,4-tetrahydro-quinoline-6-sulfonique (4-éthyl-phényl)-isobutyl-amide |
| | Composé 32 |
| 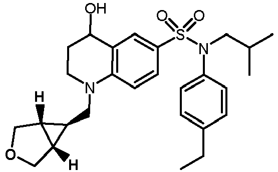 | Acide 4-hydroxy-1-[(1S,5R,6S)-1-(3-oxa-bicyclo-[3.1.0]hex-6-yl)methyl]-1,2,3,4-tetrahydro-quinoline-6-sulfonique (4-éthyl-phényl)-isobutyl-amide |
| | Composé 37 (Composé 241) |
| 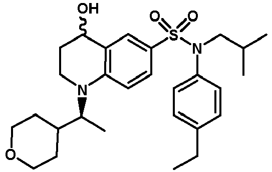 | Acide 4-hydroxy-1-[(S)-1-(tétrahydro-pyran-4-yl)-éthyl]-1,2,3,4-tétrahydro-quinoline-6-sulfonique (4-éthyl-phényl)-isobutyl-amide |
| | Composé 40 |
| 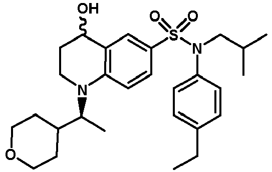 | 4-hydroxy-1-[(S)-1-(tétrahydro-pyran-4-yl)-éthyl]-1,2,3,4-tetrahydro-quino line-6-sulfonique (4-éthyl-phényl)-isobutyl-amide |
| | Composé 41 |
| 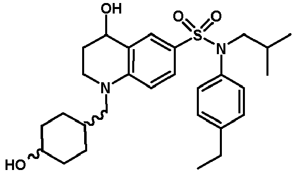 | Acide 4-hydroxy-1-(4-hydroxy-cyclohexylmethyl)-1,2,3,4-tetrahydro-quino line-6-sulfonique (4-éthyl-phényl)-isobutyl-amide |
| | Composé 42 |
| 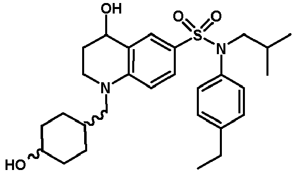 | Acide 4-hydroxy-1-(4-hydroxy-cyclohexylmethyl)-1,2,3,4-tetrahydro-quino line-6-sulfonique (4-éthyl-phényl)-isobutyl-amide |
| | Composé 43 |
| 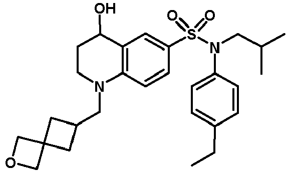 | Acide 4-hydroxy-1-(2-oxa-spiro[3.3]hept-6-ylmethyl)-1,2,3,4-tetrahydro-quinoline-6-sulfonique (4-éthyl-phényl)-isobutyl-amide |
| | Composé 44 |
| 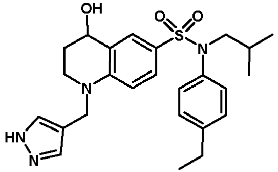 | Acide 4-hydroxy-1-(1H-pyrazol-4-ylméthyl)-1,2,3,4-tétrahydro-quinoline-6-sulfonique (4-éthyl-phényl)-isobutyl-amide |
| | Composé 45 |

Ainsi les composés 1, 2, 4, 6, 9-11, 14, 15, 18 à 24, 26 à 32, 37, 40 et 45 sont préférés.

L'invention concerne également le ou les composés tels que décrits précédemment en tant que médicament et cosmétique.

De préférence, l'invention concerne également le ou les composés tels que décrits précédemment en tant que médicament.

En effet, les composés selon l'invention présentent des propriétés pharmacologiques intéressantes étant donné que lesdits composés modulent, c'est-à-dire inhibent, l'activité du récepteur RORyt.

Ainsi ces propriétés rendent le ou les composés de formule (Ia) telle que décrite précédemment utilisables en tant que médicament dans le traitement des maladies médiées par le récepteur RORyt.

De préférence, le ou les composés selon l'invention sont utilisés dans le traitement des désordres inflammatoires et/ou des maladies auto-immunes médiées par le récepteur RORyt.

Plus préférentiellement, le ou les composés selon l'invention, de préférence ceux choisis parmi les composés répondant à la formule (V), préférentiellement (V') et (V"), sont utilisés dans le traitement de l'acné, le psoriasis et/ou la dermatite atopique.

Selon un autre mode de réalisation, les composés selon l'invention sont utilisés pour le traitement cosmétique de la peau.

Comme indiqué ci-avant, la présente invention est aussi relative à une composition pharmaceutique comprenant, dans un milieu pharmaceutiquement acceptable, un ou plusieurs composés de formule (I) telle que définie précédemment, leurs sels d'addition pharmaceutiquement acceptables, leurs hydrates et/ou leurs solvates.

De préférence, la composition pharmaceutique comprend un ou plusieurs composés choisis parmi les composés de formule (V) telle que définie précédemment, leurs sels d'addition pharmaceutiquement acceptables, leurs hydrates et/ou leurs solvates.

Plus préférentiellement, la composition pharmaceutique comprend un ou plusieurs composés de formule (I) choisis parmi les composés (1) à (66) définis précédemment.

L'administration de la composition pharmaceutique selon l'invention telle que décrite précédemment peut être effectuée par voie orale ou topique.

De préférence, la composition pharmaceutique est conditionnée sous une forme convenant à une application par voie topique.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie topique, la composition pharmaceutique selon l'invention est plus particulièrement destinée au traitement de la peau et des muqueuses et peut se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de suspensions, de sticks, de shampoings, ou de bases lavantes. Elle peut également se présenter sous forme de suspensions de microsphères ou nanosphères ou de vésicules lipidiques ou polymériques ou de patches polymériques ou gélifiés permettant une libération contrôlée.

La composition pharmaceutique est utilisée pour le traitement des désordres inflammatoires et/ou les maladies auto-immunes médiées par le récepteur RORγt.

Plus préférentiellement, la composition pharmaceutique est utilisée dans le traitement de l'acné et/ou le psoriasis.

L'invention concerne aussi un procédé de traitement des maladies médiées par le récepteur RORγt comprenant l'administration, notamment par voie topique ou orale, d'une quantité thérapeutiquement efficace de la composition pharmaceutique telle que définie ci-avant à un patient.

De préférence, la composition pharmaceutique est appliquée par voie topique.

Préférentiellement, l'invention a pour objet le ou les composés de formule (V) pour leur utilisation dans le traitement de l'acné.

En variante, l'invention a également pour objet le ou les composés de formule (V) pour leur utilisation dans le traitement du psoriasis.

Alternativement, le ou les composés de formule (V) selon l'invention sont utilisés pour le traitement cosmétique de la peau.

Les exemples suivants servent à illustrer l'invention sans toutefois présenter un caractère limitatif.

### EXEMPLES

La méthode standard LCMS pour l'analyse des produits est la suivante : colonne standard BEH C₁₈ (150*2.1mm, 1.8 µm) solvant eau/acétonitrile 0.1% acide formique.

Les purifications par HPLC préparative ont été réalisées sur colonne C₁₈, avec pour éluant : 85% d'acétonitrile dans eau/0.1% d'acide formique.

### Partie I : Synthèse des sulfonamides bicycliques à partir du schéma réactionnel 1

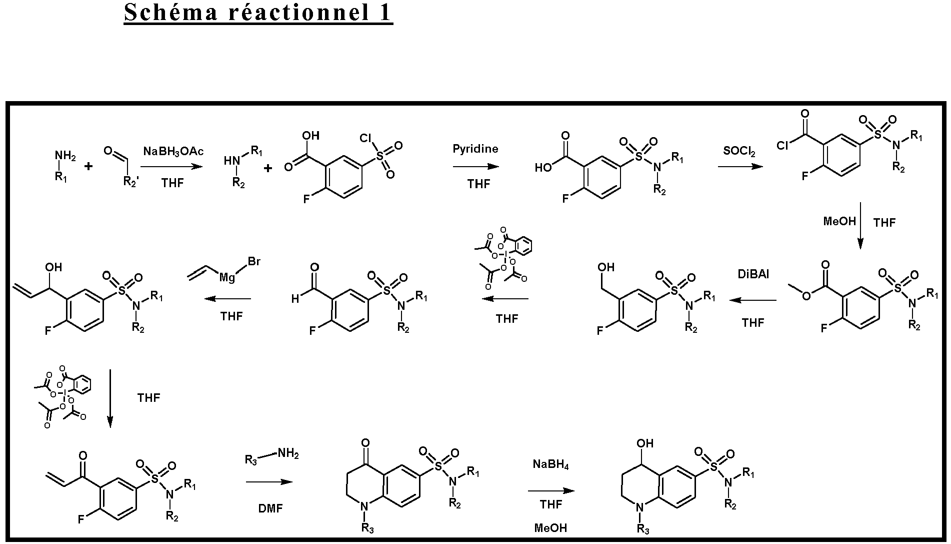

### Exemple 1: acide 4-Oxo-1-(tetrahydro-pyran-4-ylmethyl)-1,2,3,4-tetrahydro-quinoline-6-sulfonique (4-ethyl-phenyl)-isobutyl-amide

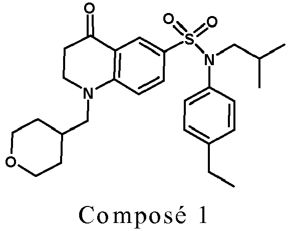

### 1. Synthèse de l'intermédiaire 1.1

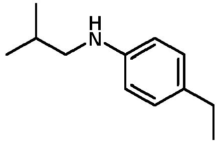

### (4-éthyl-phényl)-isobutyl-amine

A la 4-éthylaniline (9.48 ml; 0.08 mol) est ajouté l'isobutyraldehyde (6.33 ml; 0.07 mol.) dans le tétrahydrofurane (100ml). Le mélange est agité 2 heures à température ambiante.

Puis le triacétoxy-borohydrure de sodium (22.04 g; 0.10 mol) est ajouté. Le mélange est agité pendant une nuit à température ambiante, additionné d'eau (100ml) et extrait à l'acétate d'éthyle (2 x 100ml). Les phases organiques sont rassemblées, lavées à la saumure (100ml), séchées (Na₂SO₄) et concentrées.

Le produit brut est chromatographié sur gel de silice (éluant heptane/dichlorométhane de 0 à 50% de dichlorométhane). La (4-éthyl-phényl)-isobutyl-amine est obtenu sous la forme d'une huile orange avec une RMN¹H conforme.
MS : [M+H] = 179

### 2. Synthèse de l'intermédiaire 1.2

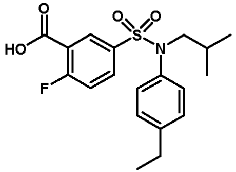

### Acide 5-[(4-éthyl-phényl)-isobutyl-sulfamoyl]-2-fluoro-benzoïque

A une solution d'acide 5-chlorosulfonyl-2-fluoro-benzoïque (1.44 g; 5.87 mmol) dans du tétrahydrofurane (8 ml) est ajoutée une solution de (4-ethyl-phenyl)-isobutyl-amine (0.80 g; 4.51 mmol) et de pyridine (0.36 ml; 4.51 mmol) dans du tétrahydrofurane (8 ml).

Le milieu réactionnel agité pendant 19 heures à température ambiante, hydrolysé avec une solution aqueuse de HCl 1N et dilué à l'acétate d'éthyle.

La phase organique est lavée avec une solution aqueuse de HCl 1N. La phase organique est séchée (Na₂SO₄), filtrée et concentrée.

Le produit brut est chromatographié sur gel de silice (éluant dichlorométhane/méthanol de 0 à 10% de méthanol) puis par HPLC préparative (colonne C18, éluant : de 60% à 70% d'acétonitrile dans eau/0.1% d'acide formique). L'acide 5-[(4-éthyl-phényl)-isobutyl-sulfamoyl]-2-fluoro-benzoique (0.56 g; 33%) est obtenu sous la forme d'un solide blanc cassé avec une RMN¹H conforme.
MS : [M-H] = 378

### 3. Synthèse de l'intermédiaire 1.3

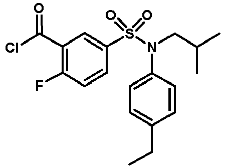

### Chlorure de 5-[(4-éthyl-phényl)-isobutyl-sulfamoyl]-2-fluoro-benzoïque

Une solution d'acide 5-[(4-éthyl-phényl)-isobutyl-sulfamoyl]-2-fluoro-benzoïque (0.59 g; 1.55 mmol) dans du chlorure de thionyle (5.0 ml) est agité 3 heures au reflux puis est concentrée à sec. Le chlorure de 5-[(4-éthyl-phényl)-isobutyl-sulfamoyl]-2-fluoro-benzoyle (0.62 g; 100%) est obtenu sous la forme d'une huile brune.
MS : [M-H] = 397

### 4. Synthèse de l'intermédiaire 1.4

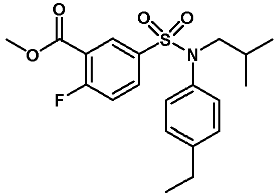

### 5-[(4-éthyl-phényl)-isobutyl-sulfamoyl]-2-fluoro-benzoate de méthyle

Un mélange de chlorure de 5-[(4-éthyl-phényl)-isobutyl-sulfamoyl]-2-fluoro-benzoyle (0.31 g; 0.78 mmol) et de méthanol (0.2 ml; 4.87 mmol.) dans du tétrahydrofurane (3ml) est agité pendant 24 heures à température ambiante.

Le milieu réactionnel est hydrolysé avec une solution aqueuse saturée (NaHCO₃) et extrait à l'acétate d'éthyle. La phase organique est lavée à la saumure, séchée (Na₂SO₄), filtrée et concentrée. Le 5-[(4-éthyl-phényl)-isobutyl-sulfamoyl]-2-fluoro-benzoate de méthyle (0.30 g; 98%) est obtenu sous la forme d'un solide blanc.
MS : [M-H] = 394

### 5. Synthèse de l'intermédiaire 1.5

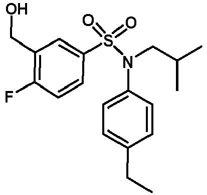

### N-(4-éthyl-phényl)-4-fluoro-3-hydroxyméthyl-N-isobutyl-benzenesulfonamide

Un mélange, maintenu à une température de -10°C, sous argon, de 5-[(4-éthyl-phenyl)-isobutyl-sulfamoyl]-2-fluoro-benzoate de méthyle (2.78 g; 7.14 mmol) dans du dichlorométhane (45ml) et d'une solution d'hydrure de diisobutylaluminum 1M (7.85 ml; 7.85 mmol) est agité pendant 30 minutes. De la solution d'hydrure de diisobutylaluminum 1M (7.85 ml; 7.85 mmol) est rajoutée et l'agitation est prolongée pendant une heure supplémentaire.

Le milieu réactionnel est hydrolysé avec une solution 1M de tartrate mixte sodium potassium, dilué avec de l'eau et du dichlorométhane et agité la nuit à température ambiante.

La phase organique est extraite, séchée (Na₂SO₄), filtrée et concentrée. Le N-(4-éthyl-phényl)-4-fluoro-3-hydroxyméthyl-N-isobutyl-benzenesulfonamide (2.65 g; 100 %) est obtenu sous la forme d'une huile incolore avec une RMN¹H conforme.
MS : [M-H] = 366

### 6. Synthèse de l'intermédiaire 1.6

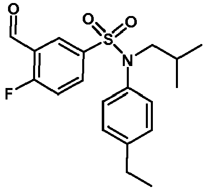

### N-(4-éthyl-phényl)-4-fluoro-3-formyl-N-isobutyl-benzenesulfonamide

Un mélange de N-(4-éthyl-phényl)-4-fluoro-3-hydroxyméthyl-N-isobutyl-benzenesulfonamide (1.10 g; 3.01 mmol) dans du dichlorométhane (20ml) et de periodinane de Dess-Martin (1.56 g; 3.68 mmol) est agité une heure. Le milieu réactionnel est hydrolysé avec une solution aqueuse à 10% de Na₂S₂O₃ et dilué avec du dichlorométhane.

Une solution aqueuse saturée d'hydrogénocarbonate de sodium (10 ml) est ajouté et le milieu réactionnel est agité pendant deux heures.

La phase organique est extraite, séchée (Na₂SO₄), filtrée et concentrée. Le N-(4-éthyl-phényl)-4-fluoro-3-formyl-N-isobutyl-benzenesulfonamide (1.16 g; 106%) est obtenu sous la forme d'un solide jaune pâle avec une RMN¹H conforme.
MS : [M-H] =364

### 7. Synthèse de l'intermédiaire 1.7

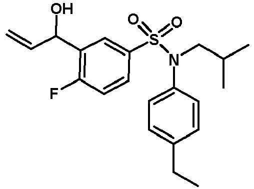

### N-(4-éthyl-phényl)-4-fluoro-3-(1-hydroxy-allyl)-N-isobutyl-benzenesulfonamide

A un mélange de N-(4-éthyl-phényl)-4-fluoro-3-formyl-N-isobutyl-benzenesulfonamide (1.12 g; 3.08 mmol) dans du tétrahydrofurane anhydre (20 ml) sous argon à une température de-78°C est ajouté, goutte à goutte, une solution de bromure de vinylmagnésium 1M dans du tétrahydrofurane (3.4 ml; 3.40 mmol).

Le milieu réactionnel est agité pendant 50 minutes, hydrolysé avec une solution aqueuse à 1 M d'acide chlorhydrique et extrait avec de l'acétate d'éthyle. La phase organique est lavée à l'eau, séchée (Na₂SO₄), filtrée et concentrée.

Le produit brut est chromatographié sur gel de silice (80 g, dépôt liquide/solide, éluant heptane/acétate d'éthyle de 0 à 30% d'acétate d'éthyle). Le N-(4-éthyl-phényl)-4-fluoro-3-(1-hydroxy-allyl)-N-isobutyl-benzenesulfon-amide (0.93 g; 77%) est obtenu sous la forme d'une huile incolore avec une RMN¹H conforme.
MS : [M-H] =392

### 8. Synthèse de l'intermédiaire 1.8

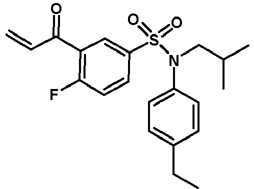

### 3-Acryloyl-N-(4-éthyl-phényl)-4-fluoro-N-isobutyl-benzenesulfonamide

Avec un mode opératoire analogue à celui décrit pour la synthèse de l'intermédiaire 1.6 sur le N-(4-éthyl-phényl)-4-fluoro-3-(1-hydroxy-allyl)-N-isobutyl-benzenesulfonamide (0.31 g; 0.79 mmol), le 3-acryloyl-N-(4-éthyl-phényl)-4-fluoro-N-isobutyl-benzenesulfonamide (0.31 g; 100%) est obtenu sous la forme d'un solide blanc avec une RMN¹H conforme.
MS : [M-H] =390

### 9. Synthèse du composé 1 selon l'invention

A une solution de 3-acryloyl-N-(4-éthyl-phényl)-4-fluoro-N-isobutyl-benzenesulfonamide (0.38 g; 0.73 mmol) dans du N,N-diméthylformamide (2ml) est rajoutée de la 4-aminométhyltétrahydropyran (0.13 ml; 1.10 mmol).

Le milieu réactionnel est agité pendant 5 minutes et purifié directement par HPLC préparative (colonne C18, éluant : acétonitrile dans eau/0.1% d'acide formique). L'acide 4-oxo-1-(tétrahydro-pyran-4-ylméthyl)-1,2,3,4-tétrahydro-quinoline-6-sulfonique (4-éthyl-phényl)-isobutyl-amide (0.16 g; 44%) est obtenu sous la forme d'un solide jaune.
¹H NMR (DMSO-d6) δ: 0.83 (d, J = 6.6 Hz, 6H), 1.18 (t, J = 7.6 Hz, 3H), 1.30 (ddd, J = 24.2, 12.1, 7.7 Hz, 2H), 1.36 - 1.44 (m, 1H), 1.58 - 1.66 (m, 2H), 1.97 (ddd, J = 11.4, 7.6, 3.7 Hz, 1H), 2.61 (q, J = 7.6 Hz, 2H), 2.64 - 2.69 (m, 2H), 3.23 (d, J = 7.3 Hz, 2H), 3.29 (dd, J = 11.8, 2.0 Hz, 2H), 3.35 (d, J = 7.3 Hz, 2H), 3.61 - 3.72 (m, 2H), 3.83 - 3.96 (m, 2H), 7.02 (t, J = 9.1 Hz, 3H), 7.16 - 7.20 (m, 2H), 7.34 (dd, J = 9.1, 2.5 Hz, 1H), 7.77 (d, J = 2.4 Hz, 1H)
MS : [M-H] =485

### Exemple 2 : Synthèse de l'acide 4-hydroxy-1-(tetrahydro-pyran-4-ylmethyl)-1,2,3,4-tetrahydro-quinoline-6-sulfonique (4-éthyl-phényl)-isobutyl-amide

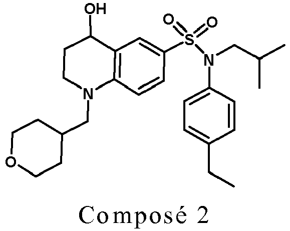

A une solution d'acide 4-oxo-1-(tetrahydro-pyran-4-ylmethyl)-1,2,3,4-tétrahydro-quinoline-6-sulfonique (4-éthyl-phényl)-isobutyl-amide (0.14 g; 0.29 mmol) dans du tétrahydrofurane (1ml) à une température de 0°C est ajouté du borohydure de sodium (22.00 mg; 0.58 mmol) et du méthanol (1ml).

Le milieu réactionnel est agité pendant 30 minutes, hydrolysé avec une solution aqueuse de dihydrogénophosphate de sodium (1M) et extrait avec du dichlorométhane. La phase organique est concentrée. Le produit brut est purifié par HPLC préparative (colonne C18, éluant : acétonitrile dans eau/0.1% d'acide formique).

L'acide 4-hydroxy-1-(tétrahydro-pyran-4-ylméthyl)-1,2,3,4-tetrahydro-quinoline-6-sulfonique (4-éthyl-phényl)-isobutyl-amide (100mg; 71%) est obtenu sous la forme d'une meringue jaune.
¹H NMR (DMSO-d6) δ: 0.82 (dd, J = 6.7, 2.6 Hz, 6H), 1.17 (t, J = 7.6 Hz, 3H), 1.26 (tt, J = 12.1, 6.0 Hz, 2H), 1.39 (dt, J = 13.7, 6.9 Hz, 1H), 1.51 - 1.59 (m, 2H), 1.75 - 1.85 (m, 2H), 1.94 (ddd, J = 11.2, 7.5, 3.9 Hz, 1H), 2.59 (q, J = 7.6 Hz, 3H), 3.12 - 3.27 (m, 6H), 3.31 (s, 6H), 3.39 - 3.49 (m, 1H), 3.84 (dd, J = 11.1, 3.9 Hz, 2H), 4.49 (q, J = 4.8 Hz, 1H), 5.29 (d, J = 4.9 Hz, 1H), 6.65 (d, J = 8.9 Hz, 1H), 6.94 - 7.02 (m, 2H), 7.08 (dd, J = 8.9, 2.4 Hz, 1H), 7.12 - 7.19 (m, 2H), 7.30 (d, J = 2.4 Hz, 1H)
MS : [M-H] = 467

Par chromatographie SFC chirale du mélange racémique, on obtient :
L'énantiomère 1 du composé 2 avec un temps de rétention de 7.4 minutes
   ¹H NMR (DMSO-d6) δ: 0.83 (dd, J = 6.6, 2.6 Hz, 6H), 1.18 (t, J = 7.6 Hz, 3H), 1.21 - 1.33 (m, 2H), 1.40 (hept, J = 5.8 Hz, 1H), 1.56 (d, J = 12.8 Hz, 2H), 1.78 - 1.86 (m, 2H), 1.89 - 2.03 (m, 1H), 2.60 (q, J = 7.6 Hz, 2H), 3.18 - 3.38 (m, 7H), 3.41 - 3.50 (m, 1H), 3.77 - 3.91 (m, 2H), 4.51 (q, J = 4.4 Hz, 1H), 5.31 (d, J = 4.9 Hz, 1H), 6.66 (d, J = 9.0 Hz, 1H), 6.99 (d, J = 8.3 Hz, 2H), 7.09 (dd, J = 8.9, 2.4 Hz, 1H), 7.17 (d, J = 8.3 Hz, 2H), 7.31 (d, J = 2.3 Hz, 1H)
   MS : [M-H] = 467
L'énantiomère 2 du composé 2 avec un temps de rétention de 6.8 minutes
   ¹H NMR (DMSO-d6) δ: 0.83 (dd, J = 6.6, 2.6 Hz, 6H), 1.18 (t, J = 7.6 Hz, 3H), 1.21 - 1.33 (m, 2H), 1.40 (hept, J = 5.8 Hz, 1H), 1.56 (d, J = 12.8 Hz, 2H), 1.78 - 1.86 (m, 2H), 1.89 - 2.03 (m, 1H), 2.60 (q, J = 7.6 Hz, 2H), 3.18 - 3.38 (m, 7H), 3.41 - 3.50 (m, 1H), 3.77 - 3.91 (m, 2H), 4.51 (q, J = 4.4 Hz, 1H), 5.31 (d, J = 4.9 Hz, 1H), 6.66 (d, J = 9.0 Hz, 1H), 6.99 (d, J = 8.3 Hz, 2H), 7.09 (dd, J = 8.9, 2.4 Hz, 1H), 7.17 (d, J = 8.3 Hz, 2H), 7.31 (d, J = 2.3 Hz, 1H)
   MS : [M-H] = 467

Avec un mode opératoire analogue à celui décrit dans l'exemple 1, on obtient les cétones du tableau ci-dessous :

| | | |
|---|---|---|
| | 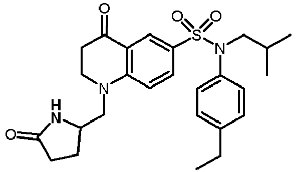 | **Acide 4-oxo-1-(5-oxo-pyrrolidin-2- ylmethyl)-1,2,3,4-tetrahydro-quinoline-6- sulfonique (4-ethyl-phenyl)-isobutyl-amide** |
| **Exemple 3** | | 1H NMR (DMSO-d6) δ: 0.84 (d, J = 6.7 Hz, 6H), 1.18 (t, J = 7.6 Hz, 3H), 1.41 (hept, J = 6.6 Hz, 1H), 1.67 - 1.81 (m, 1H), 2.04 - 2.25 (m, 2H), 2.25 - 2.38 (m, 1H), 2.61 (q, J = 7.5 Hz, 2H), 2.66 - 2.83 (m, 2H), 3.23 (d, J = 7.3 Hz, 2H), 3.41 - 3.49 (m, 1H), 3.50 - 3.60 (m, 1H), 3.61 - 3.76 (m, 2H), 3.82 - 3.93 (m, 1H), 7.01 (d, J = 8.3 Hz, 2H), 7.05 (d, J = 9.2 Hz, 1H), 7.20 (d, J = 7.9 Hz, 2H), 7.33 (dd, J = 9.0, 2.6 Hz, 1H), 7.79 (d, J = 2.3 Hz, 1H), 7.83 (s, 1H). |
| | Composé 3 | MS : [M+H] =484 |
| **Exemple 4** | 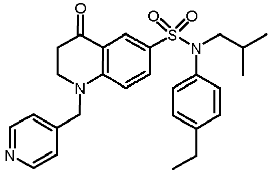 | **Acide 4-oxo-1-pyridin-4-ylmethyl-1,2,3,4- tetrahydro-quinoline-6-sulfonique (4- ethyl-phenyl)-isobutyl-amide** |
| | | 1H NMR (DMSO-d6) δ: 0.83 (d, J = 6.7 Hz, 6H), 1.17 (t, J = 7.6 Hz, 3H), 1.40 (hept, J = 7.0 Hz, 1H), 2.59 (q, J = 7.6 Hz, 2H), 2.80 (t, J = 7.2 Hz, 2H), 3.23 (d, J = 7.2 Hz, 2H), 3.79 (t, J = 7.0 Hz, 2H), 4.81 (s, 2H), 6.80 (d, J = 9.0 Hz, 1H), 6.98 (d, J = 8.3 Hz, 2H), 7.17 (d, J = 8.2 Hz, 2H), 7.30 - 7.36 (m, 3H), 7.82 (d, J = 2.9 Hz, 1H), 8.55 (d, J = 5.7 Hz, 2H). |
| | Composé 4 | MS : [M+H] =478 |
| **Exemple 5** | 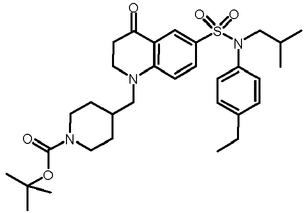 | **4-{6-[(4-Ethyl-phenyl)-isobutyl-sulfamoyl]- 4-oxo-3,4-dihydro-2H-quinolin-1- ylmethyl}-piperidine-1-carboxylate de tert-butyle** |
| | | 1H NMR (DMSO-d6) δ: 0.83 (d, J = 6.7 Hz, 6H), 1.05 - 1.22 (m, 5H), 1.40 (s, 10H), 1.63 - 1.73 (m, 2H), 1.83 - 1.97 (m, 1H), 2.55 - 2.76 (m, 6H), 3.23 (d, J = 7.2 Hz, 2H), 3.33 (s, 2H), 3.65 (t, J = 7.1 Hz, 2H), 3.90 - 4.07 (m, 2H), 6.98 - 7.06 (m, 3H), 7.19 (d, J = 8.0 Hz, 2H), 7.33 (dd, J = 9.2, 2.5 Hz, 1H), 7.77 (d, J = 2.5 Hz, 1H). |
| | Composé 5 | MS : [M+H] =584 |
| | intermédiaire | |
| **Exemple 6** | 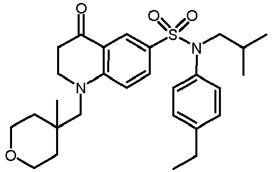 | **Acide 1-(4-methyl-tetrahydro-pyran-4- ylmethyl)-4-oxo-1,2,3,4-tetrahydro- quinoline-6-sulfonique (4-ethyl-phenyl)- isobutyl-amide** |
| | | 1H NMR (DMSO-d6) δ: 0.83 (d, J = 6.7 Hz, 6H), 1.08 (s, 3H), 1.18 (t, J = 7.6 Hz, 3H), 1.30 - 1.47 (m, 3H), 1.56 - 1.68 (m, 2H), 2.60 (q, J = 7.6 Hz, 2H), 2.65 - 2.71 (m, 2H), 3.23 (d, J = 7.3 Hz, 2H), 3.30 - 3.37 (m, 2H), 3.53 (td, J = 11.3, 2.3 Hz, 2H), 3.58 - 3.73 (m, 4H), 7.00 (d, J = 8.3 Hz, 2H), 7.15 - 7.20 (m, 3H), 7.31 (dd, J = 9.1, 2.4 Hz, 1H), 7.82 (d, J = 2.4 Hz, 1H). |
| | Composé 6 | MS : [M+H] =499 |
| **Exemple 7** | 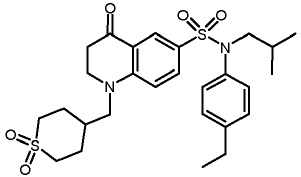 | **Acide 1-(1,1-Dioxo-hexahydro-1□⁶**- **thiopyran-4-ylmethyl)-4-oxo-1,2,3,4- tetrahydro-quinoline-6-sulfonique (4- ethyl-phenyl)-isobutyl-amide** |
| | | 1H NMR (DMSO-d6) δ: 0.84 (d, J = 6.6 Hz, 6H), 1.18 (t, J = 7.5 Hz, 3H), 1.33 - 1.49 (m, 1H), 1.67 - 1.83 (m, 2H), 2.02 - 2.12 (m, 3H), 2.61 (q, J = 7.6 Hz, 2H), 2.68 (t, J = 7.1 Hz, 2H), 3.00 - 3.19 (m, 4H), 3.23 (d, J = 7.2 Hz, 2H), 3.41 (d, J = 6.8 Hz, 2H), 3.65 (t, J = 7.0 Hz, 2H), 7.01 (d, J = 7.9 Hz, 2H), 7.06 (d, J = 9.3 Hz, 1H), 7.19 (d, J = 7.9 Hz, 2H), 7.35 (dd, J = 8.9, 2.7 Hz, 1H), 7.78 (d, J = 2.8 Hz, 1H). |
| | Composé 7 | MS : [M+H] =533 |
| **Exemple 8** | 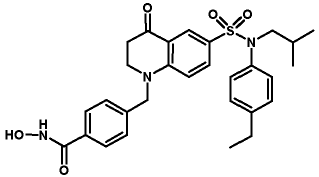 | **4-{6-[(4-éthyl-phényl)-isobutyl-sulfamoyl]- 4-oxo-3,4-dihydro-2H-quinolin-1- ylmethyl}-N-hydroxy-benzamide** |
| | | ¹H NMR (DMSO-d6) δ: 0.83 (d, J = 6.6 Hz, 6H), 1.17 (t, J = 7.5 Hz, 3H), 1.40 (hept, J = 6.6 Hz, 1H), 2.59 (q, J = 7.6 Hz, 2H), 2.78 (t, J = 7.1 Hz, 2H), 3.24 (d, J = 7.2 Hz, 2H), 3.77 (t, J = 7.1 Hz, 2H), 4.81 (s, 2H), 6.88 (d, J = 9.1 Hz, 1H), 6.99 (d, J = 7.9 Hz, 2H), 7.17 (d, J = 8.1 Hz, 2H), 7.34 (dd, J = 9.1, 2.4 Hz, 1H), 7.40 (d, J = 8.1 Hz, 2H), 7.75 (d, J = 8.0 Hz, 2H), 7.81 (d, J = 2.4 Hz, 1H), 9.02 (s, 1H), 11.20 (s, 1H). |
| | Composé 8 | MS : [M+H] =536 |

Avec un mode opératoire analogue à celui décrit dans l'exemple 2 ou en enchainant la cyclisation (exemple 1) et la réduction (exemple 2) sans isoler la cétone, on obtient les alcools du tableau ci-après:

| | | |
|---|---|---|
| **Exemple 9** | 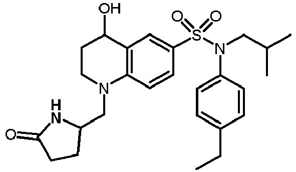 | **Acide 4-hydroxy-1-(5-oxo- pyrrolidin-2-ylméthyl)-1,2,3,4- tétrahydro-quinoline-6-sulfonique (4-éthyl-phényl)-isobutyl-amide** |
| | | ¹H NMR (DMSO-d6) δ: 0.83 (dd, J = 6.7, 2.0 Hz, 6H), 1.18 (t, J = 7.6 Hz, 3H), 1.41 (hept, J = 6.9 Hz, 1H), 1.64 - 1.96 (m, 3H), 2.04 - 2.31 (m, 3H), 2.60 (q, J = 7.6 Hz, 2H), 3.14 - 3.27 (m, 2H), 3.29 - 3.51 (m, 4H), 3.78 - 3.89 (m, 1H), 4.46 - 4.57 (m, 1H), 5.31 (t, J = 6.0 Hz, 1H), 6.70 (d, J = 8.8 Hz, 1H), 6.99 (d, J = 8.0 Hz, 2H), 7.07 (dd, J = 8.9, 2.4 Hz, 1H), 7.18 (d, J = 7.9 Hz, 2H), 7.35 (dd, J = 7.3, 2.4 Hz, 1H), 7.72 (d, J = 8.8 Hz, 1H). |
| | Composé 9 | MS : [M+H] =486 |
| **Exemple 10** | 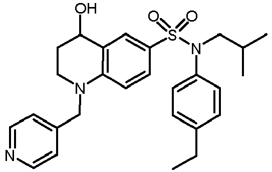 | **Acide 4-hydroxy-1-pyridin-4- ylmethyl-1,2,3,4-tétrahydro- quinoline-6-sulfonique (4-ethyl- phenyl)-isobutyl-amide** |
| | | ¹H NMR (DMSO-d6) δ: 0.82 (dd, J = 6.5, 2.7 Hz, 6H), 1.17 (t, J = 7.6 Hz, 3H), 1.39 (hept, J = 6.8 Hz, 1H), 1.87 - 2.04 (m, 2H), 2.59 (q, J = 7.5 Hz, 2H), 3.17 - 3.25 (m, 2H), 3.40 - 3.49 (m, 1H), 3.51 - 3.61 (m, 1H), 4.56 - 4.76 (m, 3H), 5.44 (d, J = 4.8 Hz, 1H), 6.45 (d, J = 8.8 Hz, 1H), 6.96 (d, J = 8.0 Hz, 2H), 7.03 (dd, J = 8.8, 2.4 Hz, 1H), 7.15 (d, J = 7.9 Hz, 2H), 7.22 (d, J = 5.0 Hz, 2H), 7.39 (d, J = 2.4 Hz, 1H), 8.52 (d, J = 5.1 Hz, 2H). |
| | Composé 10 | MS : [M+H] =480 |
| **Exemple 11** | 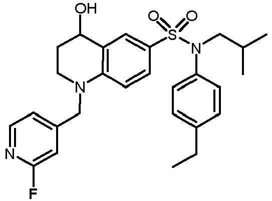 | **Acide 1-(2-fluoro-pyridin-4- ylméthyl)-4-hydroxy-1,2,3,4- tétrahydro-quinoline-6-sulfonique (4-ethyl-phenyl)-isobutyl-amide** |
| | | ¹H NMR (DMSO-d6) δ: 0.82 (d, J = 6.2 Hz, 3H), 0.83 (d, J = 6.2 Hz, 3H), 1.17 (t, J = 7.5 Hz, 3H), 1.33 - 1.47 (m, 1H), 1.87 - 2.05 (m, 2H), 2.59 (q, J = 7.7 Hz, 2H), 3.17 - 3.27 (m, 2H), 3.41 - 3.51 (m, 1H), 3.52 - 3.63 (m, 1H), 4.57 - 4.83 (m, 3H), 5.44 (d, J = 4.9 Hz, 1H), 6.45 (d, J = 8.9 Hz, 1H), 6.93 - 6.99 (m, 3H), 7.04 (dd, J = 8.7, 2.4 Hz, 1H), 7.15 (d, J = 7.9 Hz, 2H), 7.21 (d, J = 5.0 Hz, 1H), 7.41 (d, J = 2.3 Hz, 1H), 8.20 (d, J = 5.3 Hz, 1H). |
| | Composé 11 | MS : [M+H] =498 |
| **Exemple 12** | 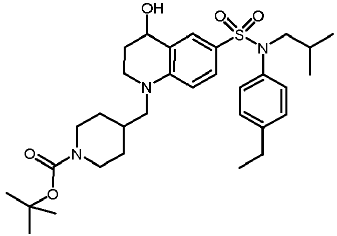 | **4-{6-[(4-éthyl-phényl)-isobutyl- sulfamoyl]-4-hydroxy-3,4-dihydro- 2H-quinolin-1-ylméthyl}- pipéridine-1-carboxylic acide tert- butyl ester** |
| | | ¹H NMR (DMSO-d6) δ: 0.83 (dd, J = 6.6, 2.7 Hz, 6H), 1.02 - 1.15 (m, 2H), 1.18 (t, J = 7.6 Hz, 3H), 1.40 (s, 10H), 1.62 (d, J = 12.6 Hz, 2H), 1.72 - 2.00 (m, 3H), 2.60 (q, J = 7.5 Hz, 2H), 3.15 - 3.27 (m, 4H), 3.27 - 3.36 (m, 3H), 3.38 - 3.49 (m, 1H), 3.90 - 4.03 (m, 2H), 4.46 - 4.54 (m, 1H), 5.32 (d, J = 4.9 Hz, 1H), 6.66 (d, J = 8.9 Hz, 1H), 6.99 (d, J = 8.0 Hz, 2H), 7.09 (dd, J = 8.9, 2.4 Hz, 1H), 7.17 (d, J = 7.9 Hz, 2H), 7.32 (d, J = 2.4 Hz, 1H). |
| | Composé 12 | MS : [M+H] = 586 |
| | intermédiaire | |
| **Exemple 13** | 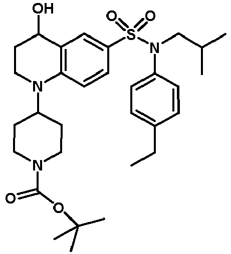 | **4-{6-[(4-Ethyl-phenyl)-isobutyl- sulfamoyl]-4-hydroxy-3,4-dihydro- 2H-quinolin-1-yl}-piperidine-1- carboxylic acide tert-butyl ester** |
| | | ¹H NMR (DMSO) δ: 0.83 (dd, J = 6.7, 1.9 Hz, 6H), 1.18 (t, J = 7.6 Hz, 3H), 1.42 (s, 10H), 1.54 - 1.70 (m, 4H), 1.70 - 1.86 (m, 2H), 2.60 (q, J = 7.6 Hz, 2H), 2.78 - 3.00 (m, 2H), 3.15 - 3.30 (m, 4H), 3.88 - 4.14 (m, 3H), 4.47 (q, J = 5.1 Hz, 1H), 5.33 (d, J = 5.0 Hz, 1H), 6.84 (d, J = 9.2 Hz, 1H), 6.98 - 7.02 (m, 2H), 7.10 (dd, J = 9.0, 2.5 Hz, 1H), 7.18 (d, J = 8.3 Hz, 2H), 7.36 (d, J = 2.5 Hz, 1H). |
| | Composé 13 | |
| | intermédiaire | MS : [M+H] = 572 |
| **Exemple 14** | 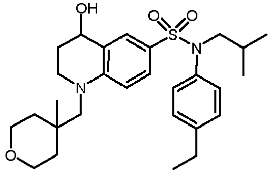 | **Acide 1-(4-méthyl-tetrahydro- pyran-4-ylmethyl)-4-hydroxy- 1,2,3,4-tetrahydro-quinoline-6- sulfonique (4-éthyl-phényl)- isobutyl-amide** |
| | | ¹H NMR (DMSO-d6) δ: 0.83 (dd, J = 6.7, 2.7 Hz, 6H), 1.03 (s, 3H), 1.18 (t, J = 7.6 Hz, 3H), 1.29 (d, J = 13.1 Hz, 2H), 1.34 - 1.47 (m, 1H), 1.53 - 1.66 (m, 2H), 1.73 - 1.92 (m, 2H), 2.60 (q, J = 7.6 Hz, 2H), 3.15 - 3.28 (m, 4H), 3.27 - 3.38 (m, 1H), 3.40 - 3.56 (m, 3H), 3.58 - 3.71 (m, 2H), 4.53 (q, J = 5.0 Hz, 1H), 5.34 (d, J = 5.1 Hz, 1H), 6.81 (d, J = 9.0 Hz, 1H), 6.95 - 7.00 (m, 2H), 7.05 (dd, J = 9.0, 2.5 Hz, 1H), 7.17 (d, J = 8.4 Hz, 2H), 7.35 (d, J = 2.4 Hz, 1H). |
| | Composé 14 | MS : [M+H] =501 |
| **Exemple 15** | 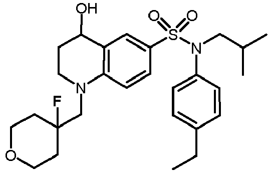 | **Acide 1-(4-fluoro-tetrahydro- pyran-4-ylméthyl)-4-hydroxy- 1,2,3,4-tetrahydro-quinoline-6- sulfonique (4-éthyl-phényl)- isobutyl-amide** |
| | | ¹H 1H NMR (DMSO-d6) δ: 0.83 (d, J = 6.6 Hz, 3H), 0.83 (d, J = 6.2 Hz, 3H), 1.18 (t, J = 7.6 Hz, 3H), 1.41 (hept, J = 6.8 Hz, 1H), 1.64 - 1.93 (m, 6H), 2.60 (q, J = 7.6 Hz, 2H), 3.15 - 3.28 (m, 2H), 3.34 - 3.44 (m, 1H), 3.45 - 3.56 (m, 3H), 3.61 (d, J = 23.1 Hz, 2H), 3.72 - 3.81 (m, 2H), 4.53 (q, J = 4.9 Hz, 1H), 5.34 (d, J = 4.8 Hz, 1H), 6.86 (d, J = 8.8 Hz, 1H), 6.99 (d, J = 8.1 Hz, 2H), 7.06 (dd, J = 9.1, 2.4 Hz, 1H), 7.17 (d, J = 7.8 Hz, 2H), 7.35 (d, J = 2.4 Hz, 1H). |
| | Composé 15 | MS : [M+H] =505 |
| **Exemple 16** | 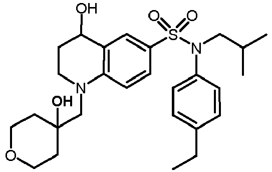 | **Acide 4-hydroxy-1-(4-hydroxy- tetrahydro-pyran-4-ylmethyl)- 1,2,3,4-tetrahydro-quinoline-6- sulfonique (4-ethyl-phenyl)- isobutyl-amide** |
| | | ¹H 1H NMR (DMSO-d6) δ: 0.83 (d, J = 6.7 Hz, 3H), 0.83 (d, J = 6.3 Hz, 3H), 1.18 (t, J = 7.6 Hz, 3H), 1.34 - 1.49 (m, 3H), 1.56 - 1.70 (m, 2H), 1.75 - 1.92 (m, 2H), 2.60 (q, J = 7.7 Hz, 2H), 3.20 (dd, J = 7.5, 4.3 Hz, 4H), 3.39 - 3.57 (m, 2H), 3.56 - 3.67 (m, 4H), 4.52 (q, J = 4.8 Hz, 1H), 4.57 (s, 1H), 5.29 (d, J = 5.0 Hz, 1H), 6.89 (d, J = 9.1 Hz, 1H), 6.99 (d, J = 7.9 Hz, 2H), 7.04 (dd, J = 8.9, 2.5 Hz, 1H), 7.17 (d, J = 8.1 Hz, 2H), 7.33 (d, J = 2.5 Hz, 1H). |
| | Composé 16 | MS : [M+H] =503 |
| **Exemple 17** | 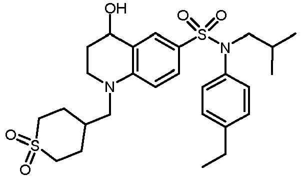 | **Acide 1-(1,1-dioxo-hexahydro-1□⁶**- **thiopyran-4-ylmethyl)-4-hydroxy- 1,2,3,4-tetrahydro-quinoline-6- sulfonique (4-ethyl-phenyl)- isobutyl-amide** |
| | | ¹H NMR (DMSO-d6) δ: 0.83 (dd, J = 6.6, 2.6 Hz, 6H), 1.19 (t, J = 7.6 Hz, 3H), 1.34 - 1.47 (m, 1H), 1.63 - 1.77 (m, 2H), 1.77 - 1.88 (m, 2H), 1.93 - 2.14 (m, 3H), 2.60 (q, J = 7.6 Hz, 2H), 2.96 - 3.26 (m, 6H), 3.26 - 3.34 (m, 3H), 3.36 - 3.49 (m, 1H), 4.51 (q, J = 4.8 Hz, 1H), 5.34 (d, J = 4.9 Hz, 1H), 6.70 (d, J = 9.0 Hz, 1H), 7.00 (d, J = 8.4 Hz, 2H), 7.09 (dd, J = 8.8, 2.4 Hz, 1H), 7.18 (d, J = 8.3 Hz, 2H), 7.32 (d, J = 2.4 Hz, 1H). |
| | Composé 17 | MS : [M+H] =535 |
| **Exemple 18** | 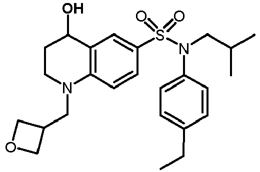 | **Acide 4-hydroxy-1-oxetan-3- ylmethyl-1,2,3,4-tetrahydro- quinoline-6-sulfonique (4-ethyl- phenyl)-isobutyl-amide** |
| | | ¹H NMR (DMSO-d6) δ: 0.83 (dd, J = 6.9, 2.4 Hz, 6H), 1.18 (t, J = 7.6 Hz, 3H), 1.32 - 1.48 (m, 1H), 1.76 - 1.86 (m, 2H), 2.60 (q, J = 7.6 Hz, 2H), 3.16 - 3.27 (m, 2H), 3.26 - 3.44 (m, 3H), 3.61 - 3.77 (m, 2H), 4.41 (q, J = 6.5 Hz, 2H), 4.45 - 4.53 (m, 1H), 4.61 - 4.68 (m, 2H), 5.32 (d, J = 5.1 Hz, 1H), 6.69 (d, J = 8.8 Hz, 1H), 6.99 (d, J = 8.2 Hz, 2H), 7.08 (dd, J = 8.9, 2.6 Hz, 1H), 7.18 (d, J = 7.9 Hz, 2H), 7.33 (d, J = 2.4 Hz, 1H). |
| | Composé 18 | MS : [M+H] =459 |
| **Exemple 19** | 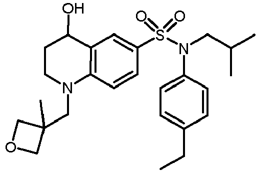 | **Acide 4-hydroxy-1-(3-methyl- oxetan-3-ylmethyl)-1,2,3,4- tetrahydro-quinoline-6-sulfonique (4-ethyl-phenyl)-isobutyl-amide** |
| | | ¹H NMR (DMSO-d6) δ: 0.82 (d, J = 6.7 Hz, 3H), 0.83 (d, J = 6.3 Hz, 3H), 1.18 (t, J = 7.6 Hz, 3H), 1.28 (s, 3H), 1.40 (hept, J = 6.8 Hz, 1H), 1.77 - 1.92 (m, 2H), 2.60 (q, J = 7.6 Hz, 2H), 3.17 - 3.40 (m, 4H), 3.50 - 3.61 (m, 2H), 4.13 - 4.23 (m, 2H), 4.49 - 4.57 (m, 3H), 5.35 (d, J = 4.8 Hz, 1H), 6.67 (d, J = 9.0 Hz, 1H), 6.97 (d, J = 8.0 Hz, 2H), 7.04 (dd, J = 8.9, 2.4 Hz, 1H), 7.16 (d, J = 7.9 Hz, 2H), 7.35 (d, J = 2.4 Hz, 1H). |
| | Composé 19 | MS : [M+H] =473 |
| **Exemple 20** | 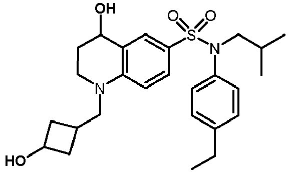 | **Acide 4-hydroxy-1-(3-hydroxy- cyclobutylmethyl)-1,2,3,4- tetrahydro-quinoline-6-sulfonique (4-ethyl-phenyl)-isobutyl-amide** |
| | | mélange de diastéréoisomères : ¹H NMR (DMSO-d6) δ: 0.83 (d, J = 6.2 Hz, 6H), 0.83 (d, J = 6.8 Hz, 6H), 1.18 (t, J = 7.6 Hz, 6H), 1.33 - 1.46 (m, 2H), 1.52 - 1.65 (m, 2H), 1.75 - 1.86 (m, 4H), 1.87 - 2.07 (m, 4H), 2.23 - 2.32 (m, 2H), 2.60 (q, J = 7.6 Hz, 4H), 3.14 - 3.28 (m, 4H), 3.26 - 3.50 (m, 19H), 3.82 - 3.95 (m, 1H), 4.25 - 4.39 (m, 1H), 4.49 (q, J = 5.0 Hz, 2H), 4.99 (t, J = 5.8 Hz, 2H), 5.30 (d, J = 4.9 Hz, 2H), 6.60 (d, J = 9.1 Hz, 1H), 6.65 (d, J = 8.9 Hz, 1H), 6.99 (d, J = 7.9 Hz, 4H), 7.09 (dt, J = 8.9, 3.2 Hz, 2H), 7.17 (d, J = 8.1 Hz, 4H), 7.31 (s, 2H). |
| | Composé 20 | MS : [M+H] =473 |
| **Exemple 21** | 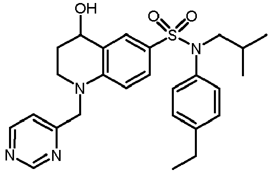 | **Acide 4-hydroxy-1-pyrimidin-4- ylmethyl-1,2,3,4-tetrahydro- quinoline-6-sulfonique (4-ethyl- phenyl)-isobutyl-amide** |
| | | ¹H NMR (DMSO-d6) δ: 0.82 (dd, J = 6.5, 2.6 Hz, 6H), 1.17 (t, J = 7.5 Hz, 3H), 1.30 - 1.47 (m, 1H), 1.85 - 2.07 (m, 2H), 2.59 (q, J = 7.5 Hz, 2H), 3.15 - 3.27 (m, 2H), 3.45 - 3.57 (m, 1H), 3.55 - 3.68 (m, 1H), 4.57 - 4.80 (m, 3H), 5.44 (d, J = 4.9 Hz, 1H), 6.45 (d, J = 8.8 Hz, 1H), 6.96 (d, J = 8.1 Hz, 2H), 7.03 (dd, J = 8.8, 2.4 Hz, 1H), 7.15 (d, J = 8.1 Hz, 2H), 7.33 (d, J = 5.2 Hz, 1H), 7.40 (d, J = 2.4 Hz, 1H), 8.74 (d, J = 5.3 Hz, 1H), 9.16 (s, 1H). |
| | Composé 21 | MS : [M+H] =481 |
| **Exemple 22** | 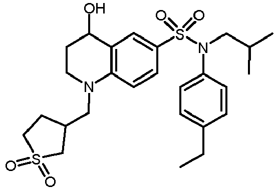 | **Acide 1-(1,1-dioxo-tetrahydro-1□⁶**- **thiophen-3-ylmethyl)-4-hydroxy- 1,2,3,4-tetrahydro-quinoline-6- sulfonique (4-ethyl-phenyl)- isobutyl-amide** |
| | | ¹H NMR (DMSO-d6) δ: 0.79-0.87 (m, 6H), 1.18 (t, J = 7.6 Hz, 3H), 1.33 - 1.46 (m, 1H), 1.74 - 1.94 (m, 3H), 2.23 - 2.32 (m, 1H), 2.60 (q, J = 7.7 Hz, 2H), 2.70 - 2.82 (m, 1H), 2.91 (t, J = 11.8 Hz, 1H), 3.00 - 3.13 (m, 1H), 3.16 - 3.59 (m, 8H), 4.44 - 4.58 (m, 1H), 5.34 (d, J = 4.7 Hz, 1H), 6.76 (d, J = 8.8 Hz, 1H), 6.99 (d, J = 8.0 Hz, 2H), 7.04 - 7.11 (m, 1H), 7.18 (d, J = 7.9 Hz, 2H), 7.36 (s, 1H). |
| | Composé 22 | MS : [M+H] =521 |
| **Exemple 23** | 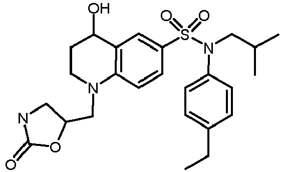 | **Acide 4-hydroxy-1-(2-oxo- oxazolidin-5-ylmethyl)-1,2,3,4- tetrahydro-quinoline-6-sulfonique (4-ethyl-phenyl)-isobutyl-amide** |
| | | ¹H NMR (DMSO-d6) δ: 0.79 - 0.88 (m, 6H), 1.18 (t, J = 7.5 Hz, 3H), 1.33 - 1.48 (m, 1H), 1.74 - 1.94 (m, 2H), 2.60 (q, J = 7.7 Hz, 2H), 3.08 - 3.29 (m, 3H), 3.29 - 3.74 (m, 5H), 4.46 - 4.59 (m, 1H), 4.74 - 4.87 (m, 1H), 5.37 (t, J = 5.5 Hz, 1H), 6.75 (dd, J = 8.9, 3.5 Hz, 1H), 6.99 (d, J = 7.8 Hz, 2H), 7.08 (dd, J = 8.8, 2.4 Hz, 1H), 7.18 (d, J = 7.9 Hz, 2H), 7.33 - 7.39 (m, 1H), 7.58 (s, 1H). |
| | Composé 23 | MS : [M+H] =470 |
| **Exemple 24** | 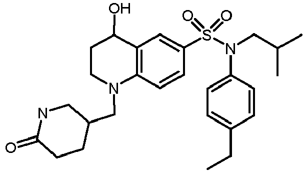 | **Acide 4-hydroxy-1-(6-oxo- piperidin-3-ylmethyl)-1,2,3,4- tetrahydro-quinoline-6-sulfonique (4-ethyl-phenyl)-isobutyl-amide** |
| | | ¹H NMR (DMSO-d6) δ: 0.79 - 0.88 (m, 6H), 1.18 (t, J = 7.6 Hz, 3H), 1.33 - 1.58 (m, 2H), 1.74 - 1.90 (m, 3H), 2.08 - 2.26 (m, 3H), 2.60 (q, J = 7.5 Hz, 2H), 2.94 (t, J = 10.9 Hz, 1H), 3.11 - 3.25 (m, 3H), 3.25 - 3.49 (m, 4H), 4.52 (q, J = 4.8 Hz, 1H), 5.34 (d, J = 4.8 Hz, 1H), 6.70 (d, J = 8.9 Hz, 1H), 6.99 (d, J = 8.1 Hz, 2H), 7.09 (dd, J = 8.9, 2.4 Hz, 1H), 7.18 (d, J = 8.1 Hz, 2H), 7.33 (d, J = 2.4 Hz, 1H), 7.46 (d, J = 3.4 Hz, 1H). |
| | Composé 24 | MS : [M+H-18] =482 |
| **Exemple 25** | 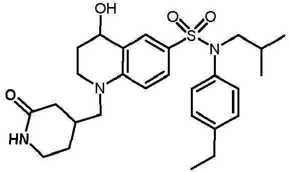 | **Acide 4-hydroxy-1-(2-oxo- piperidin-4-ylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 6.0 Hz, 3H), 0.83 (d, J = 6.3 Hz, 3H), 1.18 (t, J = 7.6 Hz, 3H), 1.34 - 1.50 (m, 2H), 1.74 - 1.88 (m, 3H), 1.88 - 2.00 (m, 1H), 2.15 - 2.29 (m, 2H), 2.60 (q, J = 7.6 Hz, 2H), 3.03 - 3.14 (m, 1H), 3.14 - 3.39 (m, 6H), 3.39 - 3.53 (m, 1H), 4.52 (q, J = 4.9 Hz, 1H), 5.26 - 5.36 (m, 1H), 6.71 (d, J = 9.0 Hz, 1H), 6.99 (d, J = 8.0 Hz, 2H), 7.08 (dd, J = 8.9, 2.4 Hz, 1H), 7.18 (d, J = 7.9 Hz, 2H), 7.33 (d, J = 2.4 Hz, 1H), 7.45 (s, 1H). |
| | Composé 25 | MS : [M+H] =500 |
| **Exemple 26** | 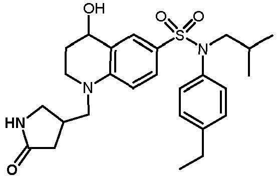 | **Acide 4-hydroxy-1-(5-oxo- pyrrolidin-3-ylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 7.1 Hz, 6H), 1.18 (t, J = 7.6 Hz, 3H), 1.34 - 1.48 (m, 1H), 1.75 - 1.91 (m, 2H), 1.91 - 2.03 (m, 1H), 2.19 - 2.29 (m, 1H), 2.60 (q, J = 7.6 Hz, 2H), 2.72 - 2.85 (m, 1H), 2.94 - 3.04 (m, 1H), 3.16 - 3.27 (m, 2H), 3.28 - 3.51 (m, 5H), 4.51 (q, J = 4.9 Hz, 1H), 5.32 (d, J = 4.8 Hz, 1H), 6.71 (d, J = 8.9 Hz, 1H), 6.99 (d, J = 7.9 Hz, 2H), 7.05 - 7.11 (m, 2H), 7.18 (d, J = 8.0 Hz, 2H), 7.35 (s, 1H), 7.55 (s, 1H). |
| | Composé 26 | MS : [M+H] =486 |
| **Exemple 27** | 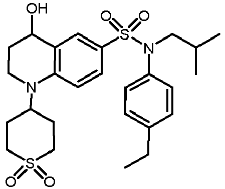 | **Acide 1-(1,1-dioxo-hexahydro-1□⁶**- **thiopyran-4-yl)-4-hydroxy-1,2,3,4- tetrahydro-quinoline-6-sulfonique (4-ethyl-phenyl)-isobutyl-amide** |
| | | ¹H NMR (DMSO-d6) δ: 0.83 (d, J = 6.7 Hz, 6H), 1.19 (t, J = 7.6 Hz, 3H), 1.32 - 1.49 (m, 1H), 1.73 - 1.89 (m, 2H), 1.90 - 2.07 (m, 2H), 2.11 - 2.30 (m, 2H), 2.61 (q, J = 7.5 Hz, 2H), 3.08 - 3.18 (m, 2H), 3.18 - 3.37 (m, 4H), 3.39 - 3.53 (m, 2H), 4.17 - 4.33 (m, 1H), 4.44 - 4.53 (m, 1H), 5.36 (d, J = 5.0 Hz, 1H), 6.87 (d, J = 9.0 Hz, 1H), 7.00 (d, J = 8.3 Hz, 2H), 7.11 (dd, J = 8.8, 2.5 Hz, 1H), 7.18 (d, J = 8.0 Hz, 2H), 7.38 (d, J = 2.6 Hz, 1H). |
| | Composé 27 | MS : [M+H] =521 |
| **Exemple 28** | 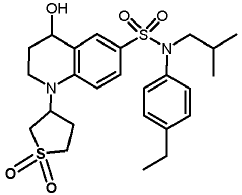 | **Acide 1-(1,1-dioxo-tetrahydro- 1lambda*6*-thiophen-3-yl)-4- hydroxy-1,2,3,4-tetrahydro- quinoline-6-sulfonique (4-ethyl- phenyl)-isobutyl-amide** |
| | | ¹H 1H NMR (DMSO-d6) δ: 0.84 (d, J = 6.6 Hz, 6H), 1.19 (t, J = 7.6 Hz, 3H), 1.41 (hept, J = 6.7 Hz, 1H), 1.72 - 1.96 (m, 2H), 2.23 - 2.41 (m, 2H), 2.61 (q, J = 7.5 Hz, 2H), 3.11 - 3.40 (m, 7H), 3.40 - 3.53 (m, 1H), 4.44 - 4.55 (m, 1H), 4.83 - 4.99 (m, 1H), 5.40 (t, J = 6.1 Hz, 1H), 6.88 - 6.96 (m, 1H), 7.00 (d, J = 8.0 Hz, 2H), 7.09 - 7.24 (m, 3H), 7.36 - 7.42 (m, 1H). |
| | Composé 28 | MS : [M+H] =507 |
| **Exemple 29** | 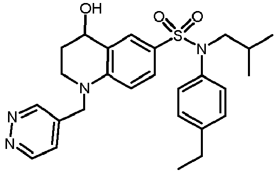 | **Acide 4-hydroxy-1-pyridazin-4- ylmethyl-1,2,3,4-tetrahydro- quinoline-6-sulfonique (4-ethyl- phenyl)-isobutyl-amide** |
| | | ¹H NMR (DMSO-d6) δ: 0.82 (d, J = 6.5 Hz, 3H), 0.83 (d, J = 6.5 Hz, 3H), 1.17 (t, J = 7.6 Hz, 3H), 1.33 - 1.46 (m, 1H), 1.85 - 2.07 (m, 2H), 2.58 (q, J = 7.6 Hz, 2H), 3.16 - 3.29 (m, 2H), 3.41 - 3.52 (m, 1H), 3.52 - 3.64 (m, 1H), 4.58 - 4.65 (m, 1H), 4.69 (d, J = 18.3 Hz, 1H), 4.78 (d, J = 18.4 Hz, 1H), 5.41 - 5.50 (m, 1H), 6.49 (d, J = 8.8 Hz, 1H), 6.96 (d, J = 7.9 Hz, 2H), 7.02 (dd, J = 8.8, 2.3 Hz, 1H), 7.15 (d, J = 8.2 Hz, 2H), 7.41 (d, J = 2.5 Hz, 1H), 7.46 (dd, J = 5.3, 2.4 Hz, 1H), 9.12 - 9.18 (m, 2H). |
| | Composé 29 | MS : [M+H] =481 |
| **Exemple 30** | 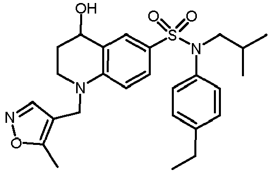 | **Acide 4-hydroxy-1-(5-methyl- isoxazol-4-ylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 6.2 Hz, 3H), 0.83 (d, J = 7.3 Hz, 3H), 1.18 (t, J = 7.6 Hz, 3H), 1.33 - 1.47 (m, 1H), 1.79 - 1.97 (m, 2H), 2.43 (s, 3H), 2.60 (q, J = 7.7 Hz, 2H), 3.15 - 3.28 (m, 2H), 3.28 - 3.50 (m, 2H), 4.37 (d, J = 16.4 Hz, 1H), 4.45 (d, J = 16.5 Hz, 1H), 4.49 - 4.57 (m, 1H), 5.36 (d, J = 4.5 Hz, 1H), 6.77 (d, J = 8.9 Hz, 1H), 6.97 (d, J = 8.1 Hz, 2H), 7.10 (dd, J = 8.9, 2.4 Hz, 1H), 7.16 (d, J = 8.0 Hz, 2H), 7.37 (d, J = 2.5 Hz, 1H), 8.36 (s, 1H). |
| | Composé 30 | MS : [M+H] =484 |
| **Exemple 31** | 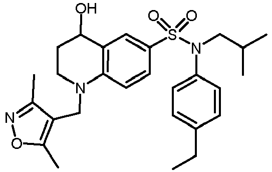 | **Acide 1-(3,5-dimethyl-isoxazol-4- ylmethyl)-4-hydroxy-1,2,3,4- tetrahydro-quinoline-6-sulfonique (4-ethyl-phenyl)-isobutyl-amide** |
| | | ¹H NMR (DMSO-d6) δ: 0.83 (d, J = 6.8 Hz, 3H), 0.83 (d, J = 6.3 Hz, 3H), 1.18 (t, J = 7.6 Hz, 3H), 1.41 (hept, J = 6.6 Hz, 1H), 1.77 - 1.92 (m, 2H), 2.10 (s, 3H), 2.31 (s, 3H), 2.60 (q, J = 7.6 Hz, 2H), 3.16 - 3.37 (m, 4H), 4.38 (s, 2H), 4.48 - 4.58 (m, 1H), 5.36 (d, J = 4.7 Hz, 1H), 6.80 (d, J = 9.0 Hz, 1H), 6.96 (d, J = 8.0 Hz, 2H), 7.12 (dd, J = 8.8, 2.4 Hz, 1H), 7.16 (d, J = 8.1 Hz, 2H), 7.38 (d, J = 2.4 Hz, 1H). |
| | Composé 31 | MS : [M+H] =498 |
| **Exemple 32** | 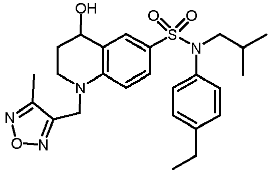 | **Acide 4-hydroxy-1-(4-methyl- furazan-3-ylmethyl)-1,2,3,4- tetrahydro-quinoline-6-sulfonique (4-ethyl-phenyl)-isobutyl-amide** |
| | | ¹H 1H NMR (DMSO-d6) δ: 0.83 (d, J = 6.3 Hz, 3H), 0.83 (d, J = 6.4 Hz, 3H), 1.18 (t, J = 7.6 Hz, 3H), 1.40 (hept, J = 6.7 Hz, 1H), 1.80 - 2.00 (m, 2H), 2.36 (s, 3H), 2.60 (q, J = 7.7 Hz, 2H), 3.16 - 3.29 (m, 2H), 3.36 - 3.56 (m, 2H), 4.57 (q, J = 4.9 Hz, 1H), 4.84 (s, 2H), 5.42 (d, J = 4.7 Hz, 1H), 6.75 (d, J = 8.8 Hz, 1H), 6.97 (d, J = 7.9 Hz, 2H), 7.08 (dd, J = 8.8, 2.4 Hz, 1H), 7.16 (d, J = 8.0 Hz, 2H), 7.41 (d, J = 2.3 Hz, 1H). |
| | Composé 32 | MS : [M+H] =485 |
| **Exemple 33** | 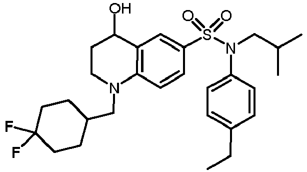 | **Acide 1-(4,4-difluoro- cyclohexylmethyl)-4-hydroxy- 1,2,3,4-tetrahydro-quinoline-6- sulfonique (4-ethyl-phenyl)- isobutyl-amide** |
| | | ¹H 1H NMR (DMSO-d6) δ: 0.83 (d, J = 6.2 Hz, 3H), 0.83 (d, J = 7.3 Hz, 3H), 1.19 (t, J = 7.5 Hz, 3H), 1.23 - 1.33 (m, 2H), 1.34 - 1.49 (m, J = 7.2 Hz, 1H), 1.65 - 1.95 (m, 7H), 2.02 (d, J = 8.8 Hz, 2H), 2.60 (q, J = 7.6 Hz, 2H), 3.16 - 3.36 (m, 5H), 3.38 - 3.52 (m, 1H), 4.51 (q, J = 4.9 Hz, 1H), 5.31 (d, J = 4.8 Hz, 1H), 6.67 (d, J = 8.9 Hz, 1H), 7.00 (d, J = 8.0 Hz, 2H), 7.09 (dd, J = 9.0, 2.4 Hz, 1H), 7.17 (d, J = 8.0 Hz, 2H), 7.32 (d, J = 2.4 Hz, 1H). |
| | Composé 33 | MS : [M+H] =521 |
| **Exemple 34** | 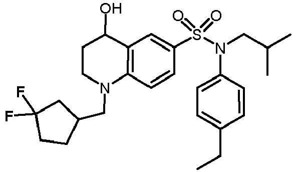 | **Acide 1-(3.3-difluoro- cyclopentylmethyl)-4-hydroxy- 1,2,3,4-tetrahydro-quinoline-6- sulfonique (4-ethyl-phenyl)- isobutyl-amide** |
| | | ¹H NMR (DMSO-d6) δ: 0.83 (d, J = 6.4 Hz, 3H), 0.83 (d, J = 6.2 Hz, 3H), 1.18 (t, J = 7.6 Hz, 3H), 1.41 (hept, J = 6.9 Hz, 1H), 1.47 - 1.61 (m, 1H), 1.76 - 1.97 (m, 4H), 1.97 - 2.11 (m, 1H), 2.11 - 2.32 (m, 2H), 2.60 (q, J = 7.6 Hz, 2H), 3.16 - 3.27 (m, 2H), 3.28 - 3.50 (m, 5H), 4.45 - 4.56 (m, 1H), 5.32 (d, J = 4.8 Hz, 1H), 6.70 (d, J = 8.9 Hz, 1H), 6.99 (d, J = 7.9 Hz, 2H), 7.09 (dd, J = 8.8, 2.4 Hz, 1H), 7.17 (d, J = 8.1 Hz, 2H), 7.34 (d, J = 2.4 Hz, 1H). |
| | Composé 34 | MS : [M+H] =507 |
| **Exemple 35** | 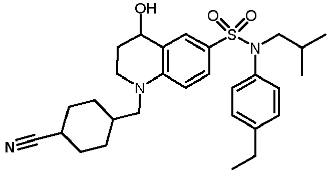 | **Acide 1-(4-cyano- cyclohexylmethyl)-4-hydroxy- 1,2,3,4-tetrahydro-quinoline-6- sulfonique (4-ethyl-phenyl)- isobutyl-amide** |
| | | ¹H 1H NMR (DMSO-d6) δ: 0.83 (d, J = 6.6 Hz, 3H), 0.83 (d, J = 6.3 Hz, 3H), 1.04 (q, J = 12.9 Hz, 2H), 1.19 (t, J = 7.6 Hz, 3H), 1.35 - 1.54 (m, 3H), 1.64 - 1.87 (m, 5H), 2.03 (d, J = 12.6 Hz, 2H), 2.56 - 2.66 (m, 3H), 3.11 - 3.35 (m, 5H), 3.36 - 3.48 (m, 1H), 4.50 (q, J = 4.8 Hz, 1H), 5.31 (d, J = 4.8 Hz, 1H), 6.61 (d, J = 9.0 Hz, 1H), 6.99 (d, J = 8.2 Hz, 2H), 7.09 (dd, J = 8.9, 2.4 Hz, 1H), 7.17 (d, J = 8.0 Hz, 2H), 7.31 (d, J = 2.4 Hz, 1H). |
| | Composé 35 | MS : [M+H] =510 |
| **Exemple 36** | 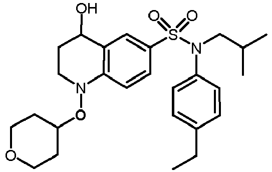 | **Acide 4-hydroxy-1-(tetrahydro- pyran-4-yloxy)-1,2,3,4-tetrahydro- quinoline-6-sulfonique (4-ethyl- phenyl)-isobutyl-amide** |
| | | ¹H NMR (DMSO) δ: 0.84 (d, J = 6.7 Hz, 3H), 0.84 (d, J = 6.2 Hz, 3H), 1.19 (t, J = 7.6 Hz, 3H), 1.41 (hept, J = 7.0 Hz, 1H), 1.51 - 1.66 (m, 2H), 1.88 - 2.05 (m, 3H), 2.07 - 2.20 (m, 1H), 2.61 (q, J = 7.5 Hz, 2H), 3.20 - 3.29 (m, 2H), 3.29 - 3.47 (m, 4H), 3.85 - 3.96 (m, 2H), 4.03 - 4.16 (m, 1H), 4.54 (q, J = 5.6 Hz, 1H), 5.49 (d, J = 5.4 Hz, 1H), 6.99 (d, J = 7.9 Hz, 2H), 7.11 (d, J = 8.7 Hz, 1H), 7.19 (d, J = 7.9 Hz, 2H), 7.28 (dd, J = 8.8, 2.3 Hz, 1H), 7.44 (d, J = 2.3 Hz, 1H). |
| | Composé 36 | MS : [M+H] =489 |
| **Exemple 37** | 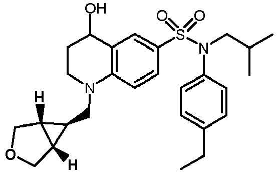 | **Acide 4-hydroxy-1-[(1S,5R,6S)-1- (3-oxa-bicyclo-[3.1.0]hex-6- yl)methyl]-1,2,3,4-tetrahydro- quinoline-6-sulfonique (4-ethyl- phenyl)-isobutyl-amide** |
| | | ¹H NMR (DMSO-d6) δ: 0.78 - 0.91 (m, 7H), 1.18 (t, J = 7.6 Hz, 3H), 1.33 - 1.48 (m, 1H), 1.60 - 1.70 (m, 2H), 1.75 - 1.93 (m, 2H), 2.60 (q, J = 7.5 Hz, 2H), 3.15 - 3.29 (m, 3H), 3.29 - 3.51 (m, 3H), 3.56 (dd, J = 8.4, 2.7 Hz, 2H), 3.72 (dd, J = 8.3, 2.0 Hz, 2H), 4.50 (q, J = 4.9 Hz, 1H), 5.31 (d, J = 5.0 Hz, 1H), 6.71 (d, J = 9.0 Hz, 1H), 6.99 (d, J = 8.0 Hz, 2H), 7.11 (dd, J = 8.9, 2.4 Hz, 1H), 7.17 (d, J = 7.9 Hz, 2H), 7.34 (d, J = 2.4 Hz, 1H). |
| | Composé 37 | MS : [M+H] =465 |
| **Exemple 38** | 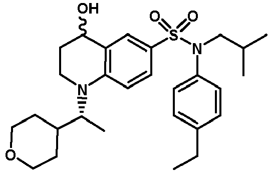 | **Acide 4-hydroxy-1-[(R)-1- (tetrahydro-pyran-4-yl)-ethyl]- 1,2,3,4-tetrahydro-quinoline-6- sulfonique (4-ethyl-phenyl)- isobutyl-amide** |
| | | ¹H NMR (DMSO-d6) δ: 0.83 (d, J = 6.2 Hz, 3H), 0.83 (d, J = 6.2 Hz, 3H), 1.05 - 1.15 (m, 4H), 1.19 (t, J = 7.6 Hz, 3H), 1.22 - 1.56 (m, 3H), 1.55 - 1.65 (m, 1H), 1.71 - 1.94 (m, 3H), 2.61 (q, J = 7.5 Hz, 2H), 3.11 - 3.30 (m, 6H), 3.76 - 3.85 (m, 2H), 3.86 - 3.95 (m, 1H), 4.47 (q, J = 4.8 Hz, 1H), 5.30 (d, J = 4.8 Hz, 1H), 6.86 (d, J = 9.1 Hz, 1H), 7.01 (d, J = 8.1 Hz, 2H), 7.08 (dd, J = 9.1, 2.5 Hz, 1H), 7.18 (d, J = 8.0 Hz, 2H), 7.33 (d, J = 2.4 Hz, 1H). |
| | Composé 38 | MS : [M+H] =501 |
| **Exemple 39** | 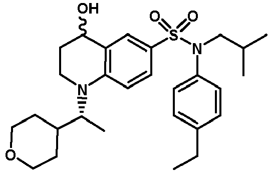 | **Acide 4-hydroxy-1-[(R)-1- (tetrahydro-pyran-4-yl)-ethyl]- 1,2,3,4-tetrahydro-quinoline-6- sulfonique (4-ethyl-phenyl)- isobutyl-amide** |
| | | ¹H NMR (DMSO-d6) δ: 0.83 (d, J = 6.6 Hz, 3H), 0.83 (d, J = 6.7 Hz, 3H), 1.03 - 1.23 (m, 7H), 1.26 - 1.49 (m, 3H), 1.54 - 1.64 (m, 1H), 1.72 - 1.88 (m, 3H), 2.60 (q, J = 7.6 Hz, 2H), 3.12 - 3.40 (m, 6H), 3.72 - 3.97 (m, 3H), 4.49 (q, J = 5.0 Hz, 1H), 5.29 (d, J = 4.9 Hz, 1H), 6.86 (d, J = 9.2 Hz, 1H), 7.00 (d, J = 8.3 Hz, 2H), 7.06 (dd, J = 9.0, 2.5 Hz, 1H), 7.17 (d, J = 8.3 Hz, 2H), 7.33 (d, J = 2.4 Hz, 1H). |
| | Composé 39 | MS : [M+H] =501 |
| **Exemple 40** | 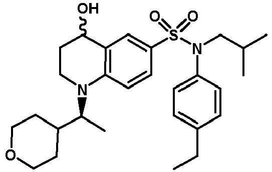 | **Acide 4-hydroxy-1-[(S)-1- (tetrahydro-pyran-4-yl)-ethyl]- 1,2,3,4-tetrahydro-quinoline-6- sulfonique (4-ethyl-phenyl)- isobutyl-amide** |
| | | ¹H NMR (DMSO-d6) δ: 0.83 (d, J = 6.7 Hz, 3H), 0.83 (d, J = 6.3 Hz, 3H), 1.12 (d, J = 6.5 Hz, 4H), 1.19 (t, J = 7.6 Hz, 3H), 1.26 - 1.55 (m, 3H), 1.56 - 1.67 (m, 1H), 1.72 - 1.91 (m, 3H), 2.61 (q, J = 7.5 Hz, 2H), 3.11 - 3.31 (m, 6H), 3.74 - 3.87 (m, 2H), 3.86 - 3.95 (m, 1H), 4.47 (q, J = 4.7 Hz, 1H), 5.30 (d, J = 4.8 Hz, 1H), 6.86 (d, J = 9.1 Hz, 1H), 7.01 (d, J = 8.1 Hz, 2H), 7.08 (dd, J = 9.1, 2.5 Hz, 1H), 7.18 (d, J = 8.0 Hz, 2H), 7.33 (d, J = 2.4 Hz, 1H). |
| | Composé 40 | MS : [M+H] =501 |
| **Exemple 41** | 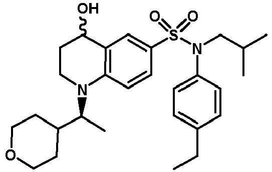 | **Acide 4-hydroxy-1-[(S)-1- (tetrahydro-pyran-4-yl)-ethyl]- 1,2,3,4-tetrahydro-quinoline-6- sulfonique (4-ethyl-phenyl)- isobutyl-amide** |
| | | ¹H NMR (DMSO-d6) δ: 0.83 (d, J = 6.7 Hz, 3H), 0.83 (d, J = 6.7 Hz, 3H), 1.04 - 1.22 (m, 7H), 1.26 - 1.50 (m, 3H), 1.55 - 1.63 (m, 1H), 1.71 - 1.88 (m, 3H), 2.60 (q, J = 7.6 Hz, 2H), 3.13 - 3.35 (m, 6H), 3.76 - 3.94 (m, 3H), 4.49 (q, J = 5.1 Hz, 1H), 5.29 (d, J = 4.9 Hz, 1H), 6.86 (d, J = 9.2 Hz, 1H), 6.98 - 7.02 (m, 2H), 7.06 (dd, J = 9.0, 2.5 Hz, 1H), 7.17 (d,J = 8.3 Hz, 2H), 7.33 (d, J = 2.5 Hz, 1H). |
| | Composé 41 | MS : [M+H] =501 |
| **Exemple 42** | 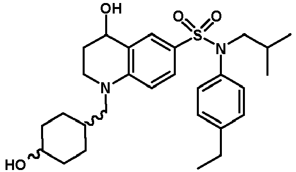 | **Acide 4-hydroxy-1-(4-hydroxy- cyclohexylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 6.2 Hz, 3H), 0.83 (d, J = 6.8 Hz, 3H), 0.95 - 1.15 (m, 4H), 1.18 (t, J = 7.6 Hz, 3H), 1.41 (hept, J = 6.7 Hz, 1H), 1.56 - 1.74 (m, 3H), 1.74 - 1.91 (m, 4H), 2.60 (q, J = 7.6 Hz, 2H), 3.11 - 3.38 (m, 6H), 3.37 - 3.50 (m, 1H), 4.44 - 4.55 (m, 2H), 5.30 (d, J = 4.8 Hz, 1H), 6.60 (d, J = 9.0 Hz, 1H), 6.99 (d, J = 7.9 Hz, 2H), 7.09 (dd, J = 8.9, 2.5 Hz, 1H), 7.17 (d, J = 7.9 Hz, 2H), 7.31 (d, J = 2.5 Hz, 1H). |
| | Composé 42 | MS : [M+H] =501 |
| | | Temps de rétention : 8.257 min |
| **Exemple 43** | 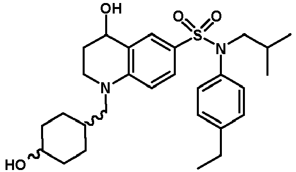 | **Acide 4-hydroxy-1-(4-hydroxy- cyclohexylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 6.3 Hz, 3H), 0.83 (d, J = 6.4 Hz, 3H), 1.18 (t, J = 7.6 Hz, 3H), 1.31 - 1.49 (m, 7H), 1.55 - 1.68 (m, 2H), 1.69 - 1.89 (m, 3H), 2.60 (q, J = 7.6 Hz, 2H), 3.12 - 3.37 (m, 5H), 3.40 - 3.50 (m, 1H), 3.78 (s, 1H), 4.29 (d, J = 3.2 Hz, 1H), 4.50 (q, J = 4.8 Hz, 1H), 5.30 (d, J = 4.8 Hz, 1H), 6.60 (d, J = 9.0 Hz, 1H), 6.99 (d, J = 7.9 Hz, 2H), 7.11 (dd, J = 8.9, 2.4 Hz, 1H), 7.17 (d, J = 8.1 Hz, 2H), 7.30 (d, J = 2.4 Hz, 1H). |
| | Composé 43 | MS : [M+H] =501 |
| | | Temps de rétention : 8.430 min |
| **Exemple 44** | 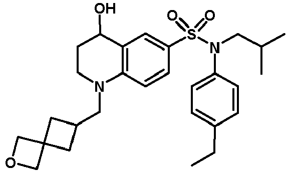 | **Acide 4-hydroxy-1-(2-oxa- spiro[3.3]hept-6-ylmethyl)-1,2,3,4- tetrahydro-quinoline-6-sulfonique (4-ethyl-phenyl)-isobutyl-amide** |
| | | ¹H NMR (DMSO-d6) δ: 0.83 (d, J = 6.8 Hz, 3H), 0.83 (d, J = 6.3 Hz, 3H), 1.18 (t, J = 7.6 Hz, 3H), 1.34 - 1.47 (m, 1H), 1.74 - 1.85 (m, 2H), 1.89 - 2.02 (m, 2H), 2.23 - 2.54 (m, 4H), 2.60 (q, J = 7.7 Hz, 2H), 3.15 - 3.25 (m, 2H), 3.25 - 3.46 (m, 3H), 4.43 - 4.53 (m, 3H), 4.58 (s, 2H), 5.29 (d, J = 4.8 Hz, 1H), 6.61 (d, J = 8.9 Hz, 1H), 6.99 (d, J = 7.9 Hz, 2H), 7.08 (dd, J = 8.8, 2.5 Hz, 1H), 7.17 (d, J = 8.1 Hz, 2H), 7.31 (d, J = 2.5 Hz, 1H). ). |
| | Composé 44 | MS : [M+H] =499 |
| **Exemple 46** | 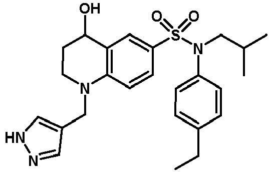 | **Acide 4-hydroxy-1-(1H-pyrazol-4- ylmethyl)-1,2,3,4-tetrahydro- quinoline-6-sulfonique (4-ethyl- phenyl)-isobutyl-amide** |
| | | ¹H NMR (DMSO-d6) δ: 0.82 (d, J = 6.5 Hz, 3H), 0.83 (d, J = 6.5 Hz, 3H), 1.18 (t, J = 7.6 Hz, 3H), 1.32 - 1.48 (m, 1H), 1.79 - 1.94 (m, 2H), 2.60 (q, J = 7.6 Hz, 2H), 3.15 - 3.28 (m, 2H), 3.28 - 3.54 (m, 2H), 4.33 - 4.54 (m, 3H), 5.31 (d, J = 4.8 Hz, 1H), 6.80 (d, J = 8.9 Hz, 1H), 6.97 (d, J = 7.8 Hz, 2H), 7.09 (dd, J = 8.8, 2.4 Hz, 1H), 7.17 (d, J = 8.0 Hz, 2H), 7.33 (d, J = 2.5 Hz, 1H), 7.43 - 7.74 (m, 2H), 12.71 (s, 1H). |
| | Composé 45 | MS : [M+H] =469 |
| **Exemple 47** | 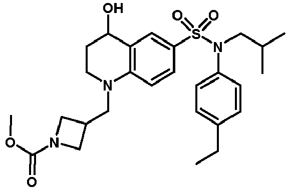 | **3-{6-[(4-Ethyl-phenyl)-isobutyl- sulfamoyl]-4-hydroxy-3,4-dihydro- 2H-quinolin-1-ylmethyl}-azetidine- 1-carboxylate de méthyle** |
| | | ¹H NMR (DMSO-d6) δ: 0.83 (d, J = 6.7 Hz, 3H), 0.83 (d, J = 6.4 Hz, 3H), 1.18 (t, J = 7.5 Hz, 3H), 1.41 (hept, J = 6.7 Hz, 1H), 1.81 (p, J = 5.7, 4.9 Hz, 2H), 2.60 (q, J = 7.5 Hz, 2H), 2.85 - 3.00 (m, 1H), 3.15 - 3.27 (m, 2H), 3.27 - 3.47 (m, 2H), 3.56 (s, 3H), 3.58 - 3.65 (m, 2H), 3.65 - 3.78 (m, 2H), 3.89 - 4.06 (m, 2H), 4.49 (q, J = 4.9 Hz, 1H), 5.32 (d, J = 4.8 Hz, 1H), 6.72 (d, J = 8.9 Hz, 1H), 6.99 (d, J = 8.1 Hz, 2H), 7.08 (dd, J = 8.8, 2.4 Hz, 1H), 7.18 (d, J = 8.0 Hz, 2H), 7.34 (d, J = 2.4 Hz, 1H). |
| | Composé 46 | MS : [M+H] =516 |
| **Exemple 48** | 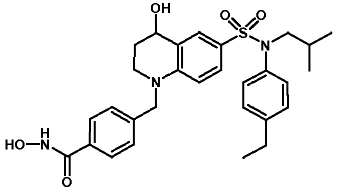 | **4-{6-[(4-Ethyl-phenyl)-isobutyl- sulfamoyl]-4-hydroxy-3,4-dihydro- 2H-quinolin-1-ylmethyl}-N- hydroxy-benzamide** |
| | | 1H NMR (DMSO-d6) δ: 0.82 (d, J = 6.6 Hz, 3H), 0.83 (d, J = 6.3 Hz, 3H), 1.17 (t, J = 7.6 Hz, 3H), 1.33 - 1.48 (m, 1H), 1.84 - 2.02 (m, 2H), 2.59 (q, J = 7.7 Hz, 2H), 3.15 - 3.28 (m, 2H), 3.39 - 3.49 (m, 1H), 3.50 - 3.65 (m, 1H), 4.52 - 4.77 (m, 3H), 5.41 (d, J = 4.8 Hz, 1H), 6.52 (d, J = 8.9 Hz, 1H), 6.97 (d, J = 8.0 Hz, 2H), 7.05 (dd, J = 8.8, 2.4 Hz, 1H), 7.16 (d, J = 7.9 Hz, 2H), 7.30 (d, J = 8.0 Hz, 2H), 7.38 (d, J = 2.4 Hz, 1H), 7.72 (d, J = 7.9 Hz, 2H), 9.00 (s, 1H), 11.17 (s, 1H). |
| | Composé 47 | MS : [M+H] =538 |
| **Exemple 49** | 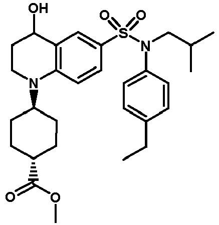 | **(1r,4r)-4-(6-(N-(4-ethylphenyl)-N- isobutyl-sulfamoyl)-4-hydroxy-3,4- dihydroquinolin-1 (2H)- yl)cyclohexane-1-carboxylate de méthyle** |
| | | ¹H NMR (DMSO-d6) δ: 0.83 (d, J = 6.7 Hz, 3H), 0.83 (d, J = 6.6 Hz, 3H), 1.19 (t, J = 7.6 Hz, 3H), 1.40 (hept, J = 6.7 Hz, 1H), 1.50 - 1.85 (m, 8H), 1.94 - 2.03 (m, 2H), 2.29 - 2.41 (m, 1H), 2.60 (q, J = 7.6 Hz, 2H), 3.15 - 3.35 (m, 4H), 3.61 (s, 3H), 3.68 - 3.82 (m, 1H), 4.46 (q, J = 5.0 Hz, 1H), 5.30 (d, J = 5.0 Hz, 1H), 6.79 (d, J = 9.2 Hz, 1H), 7.00 (d, J = 8.3 Hz, 2H), 7.10 (dd, J = 8.9, 2.4 Hz, 1H), 7.18 (d, J = 8.4 Hz, 2H), 7.34 (d, J = 2.3 Hz, 1H). |
| | Composé 48 | MS : [M+H] =529 |
| **Exemple 50** | 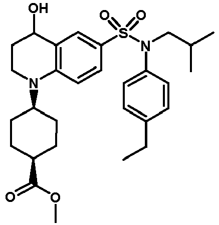 | **(1s,4s)-4-(6-(N-(4-ethylphenyl)-N- isobutyl-sulfamoyl)-4-hydroxy-3,4- dihydroquinolin-1 (2H)- yl)cyclohexane-1-carboxylate de méthyle** |
| | | ¹H NMR (DMSO-d6) δ: 0.83 (d, J = 6.6 Hz, 3H), 0.83 (d, J = 6.6 Hz, 3H), 1.19 (t, J = 7.6 Hz, 3H), 1.34 - 1.47 (m, 1H), 1.52 - 1.86 (m, 8H), 2.14 (d, J = 11.4 Hz, 2H), 2.60 (q, J = 7.6 Hz, 2H), 2.69 - 2.75 (m, 1H), 3.16 - 3.25 (m, 4H), 3.67 (s, 3H), 3.70 - 3.83 (m, 1H), 4.46 (q, J = 5.1 Hz, 1H), 5.30 (d, J = 5.0 Hz, 1H), 6.77 (d, J = 9.2 Hz, 1H), 7.00 (d, J = 8.4 Hz, 2H), 7.10 (dd, J = 9.0, 2.5 Hz, 1H), 7.18 (d, J = 8.3 Hz, 2H), 7.34 (d, J = 2.5 Hz, 1H). |
| | Composé 49 | MS : [M+H] =529 |
| **Exemple 51** | 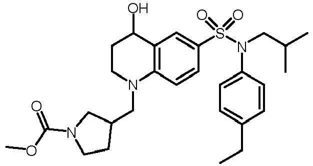 | **3-{6-[(4-Ethyl-phenyl)-isobutyl- sulfamoyl]-4-hydroxy-3,4-dihydro- 2H-quinolin-1-ylmethyl}- pyrrolidine-1-carboxylate de méthyle** |
| | | ¹H NMR (DMSO-d6) δ: 0.83 (d, J = 6.1 Hz, 3H), 0.83 (d, J = 6.1 Hz, 3H), 1.18 (t, J = 7.6 Hz, 3H), 1.41 (hept, J = 6.7 Hz, 1H), 1.56 - 1.74 (m, 1H), 1.76 - 1.90 (m, 2H), 1.90 - 2.04 (m, 1H), 2.55 - 2.66 (m, 3H), 3.03 (t, J = 9.0 Hz, 1H), 3.16 - 3.29 (m, 3H), 3.29 - 3.50 (m, 6H), 3.57 (d, J = 5.4 Hz, 3H), 4.51 (q, J = 4.9 Hz, 1H), 5.30 - 5.35 (m, 1H), 6.71 (d, J = 9.0 Hz, 1H), 6.99 (d, J = 8.0 Hz, 2H), 7.06 - 7.11 (m, 1H), 7.18 (d, J = 8.1 Hz, 2H), 7.31 - 7.36 (m, 1H). |
| | Composé 50 | MS : [M+H] =530 |
| **Exemple 52** | 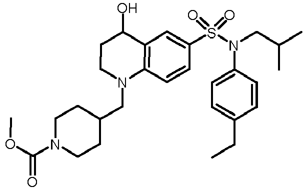 | **4-{6-[(4-Ethyl-phenyl)-isobutyl- sulfamoyl]-4-hydroxy-3,4-dihydro- 2H-quinolin-1-ylmethyl}- piperidine-1-carboxylate de méthyle** |
| | | 1H NMR (DMSO-d6) δ: 0.83 (d, J = 6.2 Hz, 3H), 0.83 (d, J = 6.1 Hz, 3H), 1.04 - 1.24 (m, 5H), 1.34 - 1.48 (m, J = 6.6 Hz, 1H), 1.64 (d, J = 12.8 Hz, 2H), 1.77 - 1.87 (m, 2H), 1.85 - 2.01 (m, 1H), 2.60 (q, J = 7.6 Hz, 2H), 2.66 - 2.82 (m, 2H), 3.15 - 3.38 (m, 5H), 3.38 - 3.49 (m, 1H), 3.58 (s, 3H), 3.90 - 4.09 (m, 2H), 4.51 (q, J = 4.7 Hz, 1H), 5.31 (d, J = 4.7 Hz, 1H), 6.66 (d, J = 9.0 Hz, 1H), 6.99 (d, J = 8.1 Hz, 2H), 7.08 (dd, J = 9.1, 2.4 Hz, 1H), 7.17 (d, J = 7.9 Hz, 2H), 7.32 (d, J = 2.4 Hz, 1H). |
| | Composé 51 | MS : [M+H] =544 |
| **Exemple 53** | 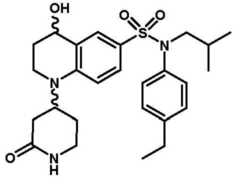 | **Diastéréoisomère 1 Acide 4-hydroxy-1-(2-oxo- piperidin-4-yl)-1,2,3,4-tetrahydro- quinoline-6-sulfonique (4-ethyl- phenyl)-isobutyl-amide** |
| | | ¹H NMR (DMSO-d6) δ: 0.83 (d, J = 6.4 Hz, 6H), 1.19 (t, J = 7.6 Hz, 3H), 1.41 (hept, J = 6.9 Hz, 1H), 1.73 - 2.00 (m, 4H), 2.23 - 2.36 (m, 1H), 2.40 - 2.54 (m, 1H), 2.61 (q, J = 7.6 Hz, 2H), 3.14 - 3.40 (m, 6H), 4.22 - 4.35 (m, 1H), 4.49 (q, J = 5.0 Hz, 1H), 5.33 (d, J = 4.8 Hz, 1H), 6.87 (d, J = 9.0 Hz, 1H), 7.00 (d, J = 8.1 Hz, 2H), 7.10 (dd, J = 8.8, 2.5 Hz, 1H), 7.18 (d, J = 7.9 Hz, 2H), 7.35 (d, J = 2.4 Hz, 1H), 7.57 - 7.62 (m, 1H). |
| | Composé 52 | MS : [M+H] =486 |
| | | Temps de rétention : 7.671min |
| **Exemple 54** | 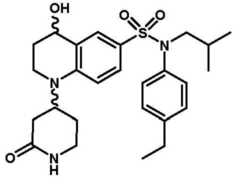 | **Diastéréoisomère 2 Acide 4-hydroxy-1-(2-oxo- piperidin-4-yl)-1,2,3,4-tetrahydro- quinoline-6-sulfonique (4-ethyl- phenyl)-isobutyl-amide** |
| | | ¹H NMR (DMSO-d6) δ: 0.83 (d, J = 6.6 Hz, 3H), 0.84 (d, J = 6.1 Hz, 3H), 1.19 (t, J = 7.6 Hz, 3H), 1.34 - 1.47 (m, 1H), 1.71 - 1.90 (m, 3H), 1.91 - 2.05 (m, 1H), 2.23 - 2.44 (m, 2H), 2.61 (q, J = 7.6 Hz, 2H), 3.14 - 3.37 (m, 6H), 4.22 - 4.34 (m, 1H), 4.48 (q, J = 5.2 Hz, 1H), 5.34 (d, J = 5.0 Hz, 1H), 6.88 (d, J = 9.1 Hz, 1H), 7.00 (d, J = 8.0 Hz, 2H), 7.10 (dd, J = 8.9, 2.4 Hz, 1H), 7.18 (d, J = 8.0 Hz, 2H), 7.37 (d, J = 2.4 Hz, 1H), 7.57 - 7.65 (m, 1H). |
| | Composé 53 | MS : [M+H] =486 |
| | | Temps de rétention : 7.961min |

### Exemple 55 : Acide 4-oxo-1-piperidin-4-ylmethyl-1,2,3,4-tetrahydro-quinoline-6-sulfonique (4-ethyl-phenyl)-isobutyl-amide

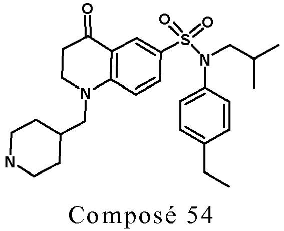

A une solution d'ester tert-butyl d'acide 4-{6-[(4-éthyl-phényl)-isobutyl-sulfamoyl]-4-oxo-3,4-dihydro-2H-quinolin-1-ylméthyl}-piperidine-1-carboxylique (0.14 g; 0.24 mmol) dans du dichlorométhane (2ml) est ajouté de l'acide trifluoroacétique (270µl; 3.54 mmol).

Le milieu réactionnel est agité pendant 12 heures, hydrolysé avec une solution aqueuse à 1 M de soude et extrait au dichlorométhane. La phase organique est lavée à l'eau, séchée (NaSO₄), filtrée et concentrée.

L'acide 4-oxo-1-piperidin-4-ylmethyl-1,2,3,4-tetrahydro-quinoline-6-sulfonique (4-ethyl-phenyl)-isobutyl-amide (0.12 g; 100%) est obtenu sous la forme d'un solide jaune.
¹H NMR (DMSO-d6) δ: 0.83 (d, J = 6.7 Hz, 6H), 1.14 - 1.29 (m, 5H), 1.41 (hept, J = 6.8 Hz, 1H), 1.63 - 1.76 (m, 2H), 1.78 - 1.91 (m, 1H), 2.42 - 2.72 (m, 6H), 2.93 - 3.09 (m, 2H), 3.23 (d, J = 7.2 Hz, 2H), 3.25 - 3.46 (m, 3H), 3.62 - 3.67 (m, 2H), 6.97 - 7.03 (m, 3H), 7.16 - 7.21 (m, 2H), 7.31 - 7.38 (m, 1H), 7.77 (d, J = 2.5 Hz, 1H)
MS : [M+H] =483

### Exemple 56 : Synthèse de l'acide 4-oxo-1-piperidin-4-yl-1,2,3,4-tetrahydro-quinoline-6-sulfonique (4-ethyl-phenyl)-isobutyl-amide

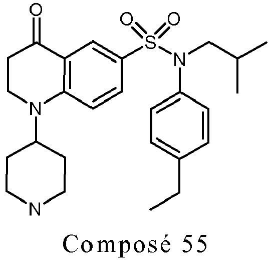

### 1. Synthèse de l'intermédiaire 56.1

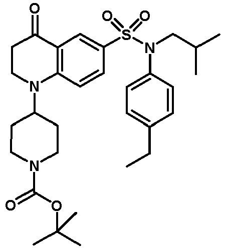

### 4-{6-[(4-éthyl-phényl)-isobutyl-sulfamoyl]-4-oxo-3,4-dihydro-2H-quinolin-1-yl}-pipéridine-1-carboxylate de méthyle

Avec un mode opératoire analogue à celui décrit dans l'exemple 1, le 4-{6-[(4-éthyl-phényl)-isobutyl-sulfamoyl]-4-oxo-3,4-dihydro-2H-quinolin-1-yl}-piperidine-1-carboxylate de méthyle (230 mg; 58%) obtenu sous la forme d'un solide jaune incolore avec une RMN¹H conforme.
MS : [M+H] = 570

### 2. Synthèse de l'acide 4-oxo-1-piperidin-4-yl-1,2,3,4-tetrahydro-quinoline-6-sulfonique (4-ethyl-phenyl)-isobutyl-amide

En suivant le même mode opératoire que pour l'exemple 55, l'acide 4-oxo-1-piperidin-4-yl-1,2,3,4-tetrahydro-quinoline-6-sulfonique (4-éthyl-phényl)-isobutyl-amide (0.15 g; 90%) est obtenu sous la forme d'un solide jaune.
¹H NMR (DMSO-d6) δ: 0.83 (d, J = 6.7 Hz, 6H), 1.18 (t, J = 7.6 Hz, 3H), 1.40 (hept, J = 6.6 Hz, 1H), 1.56 - 1.72 (m, 4H), 2.00 - 2.23 (m, 1H), 2.54 - 2.71 (m, 6H), 2.97 - 3.06 (m, 2H), 3.23 (d, J = 7.2 Hz, 2H), 3.54 (t, J = 7.0 Hz, 2H), 3.96 (p, J = 8.0 Hz, 1H), 7.01 (d, J = 8.3 Hz, 2H), 7.16 - 7.22 (m, 3H), 7.36 (dd, J = 9.1, 2.5 Hz, 1H), 7.81 (d, J = 2.5 Hz, 1H).
MS : [M+H] =470

### Exemple 57: Acide 4-hydroxy-1-piperidin-4-ylmethyl-1,2,3,4-tetrahydro-quinoline-6-sulfonique (4-ethyl-phenyl)-isobutyl-amide

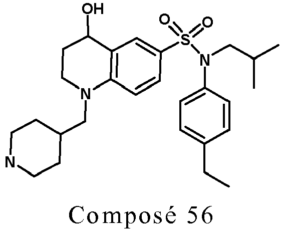

Avec le même mode opératoire que pour l'exemple 2, l'acide 4-hydroxy-1-piperidin-4-ylméthyl-1,2,3,4-tétrahydro-quinoline-6-sulfonique (4-éthyl-phényl)-isobutyl-amide (4.5 mg; 25%) est obtenu sous la forme d'un solide blanc.
^{1H} NMR (DMSO) δ: 0.83 (dd, J = 6.5, 2.8 Hz, 6H), 1.13 - 1.33 (m, 5H), 1.33 - 1.48 (m, 1H), 1.69 (d, J = 12.9 Hz, 2H), 1.75 - 1.96 (m, 3H), 2.46 - 2.66 (m, 5H), 3.11 (d, J = 12.2 Hz, 2H), 3.15 - 3.50 (m, 5H), 4.47 - 4.56 (m, 1H), 5.26 - 5.43 (m, 1H), 6.64 (d, J = 8.9 Hz, 1H), 6.99 (d, J = 8.2 Hz, 2H), 7.10 (dd, J = 8.8, 2.5 Hz, 1H), 7.18 (d, J = 7.9 Hz, 2H), 7.32 (d, J = 2.5 Hz, 1H), 8.35 (s, 1H).
MS : [M+H] =485

### Exemple 58 : Acide 4-hydroxy-1-piperidin-4-yl-1,2,3,4-tetrahydro-quinoline-6-sulfonique (4-ethyl-phenyl)-isobutyl-amide

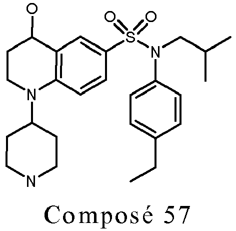

Avec le même mode opératoire que pour l'exemple 2, l'acide 4-hydroxy-1-pipéridin-4-yl-1,2,3,4-tetrahydro-quinoline-6-sulfonique (4-ethyl-phenyl)-isobutyl-amide (27 mg; 95%) est obtenu sous la forme d'un solide blanc.
¹H NMR (DMSO-d6) δ: 0.83 (d, J = 6.7 Hz, 6H), 1.19 (t, J = 7.6 Hz, 3H), 1.22 - 1.26 (m, 1H), 1.33 - 1.47 (m, 1H), 1.59 - 2.06 (m, 5H), 2.61 (q, J = 7.6 Hz, 2H), 2.91 - 3.10 (m, 2H), 3.12 - 3.68 (m, 7H), 3.95 - 4.15 (m, 1H), 4.44 - 4.54 (m, 1H), 5.13 - 5.60 (m, 1H), 6.82 - 6.92 (m, 1H), 7.00 (d, J = 8.1 Hz, 2H), 7.05 - 7.14 (m, 1H), 7.18 (d, J = 7.8 Hz, 2H), 7.30 - 7.43 (m, 1H).
MS : [M+H] =472

### Exemple 59 : Acide 4-hydroxy-1-piperidin-4-ylméthyl-1,2,3,4-tétrahydro-quinoline-6-sulfonique (4-éthyl-phényl)-isobutyl-amide

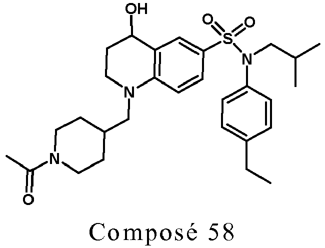

### 1. Synthèse de l'intermédiaire 59.1

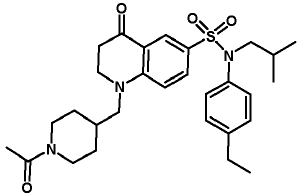

### Acide 1-(1-acétyl-piperidin-4-ylméthyl)-4-oxo-1,2,3,4-tetrahydro-quinoline-6-sulfonique (4-ethyl-phenyl)-isobutyl-amide

Un mélange d'acide 4-oxo-1-piperidin-4-ylméthyl-1,2,3,4-tetrahydro-quinoline-6-sulfonique (4-éthyl-phenyl)-isobutyl-amide (40m g; 0.08 mmol) dans du dichlorométhane (1.5ml) de chlorure d'acetyle (20µl; 0.28 mmol) et de triméthylamine (20.00 µl; 0.14 mmol) est agité pendant 16 heures et concentré sous azote. Le résidu est repris dans du N,N'-diméthylformamide et filtré. Le filtrat est purifié par HPLC préparative (colonne C18, éluant : acétonitrile dans eau/0.1% d'acide formique).

L'acide 1-(1-acétyl-piperidin-4-ylmethyl)-4-oxo-1,2,3,4-tetrahydro-quinoline-6-sulfonique (4-éthyl-phényl)-isobutyl-amide (33mg; 76 %) est obtenu sous la forme d'un solide jaune avec une RMN¹H conforme.
MS : [M+H] = 526

### 2. Synthèse de l'acide 4-hydroxy-1-piperidin-4-ylmethyl-1,2,3,4-tetrahydro-quinoline-6-sulfonique (4-ethyl-phenyl)-isobutyl-amide

Avec le même mode opératoire que pour l'exemple 2, l'acide 1-(1-acétyl-piperidin-4-ylméthyl)-4-hydroxy-1,2,3,4-tétrahydro-quinoline-6-sulfonique (4-éthyl-phényl)-isobutyl-amide (27mg; 86%) est obtenu sous la forme d'un solide blanc.
1H NMR (DMSO-d6) δ: 0.83 (dd, J = 6.6, 2.7 Hz, 6H), 0.98 - 1.11 (m, 1H), 1.18 (t, J = 7.6 Hz, 3H), 1.34 - 1.46 (m, 1H), 1.66 (t, J = 14.9 Hz, 2H), 1.74 - 1.86 (m, 3H), 1.89 - 2.05 (m, 4H), 2.39 - 2.48 (m, 1H), 2.60 (q, J = 7.6 Hz, 2H), 2.96 (t, J = 12.7 Hz, 1H), 3.13 - 3.37 (m, 4H), 3.39 - 3.49 (m, 1H), 3.57 - 3.65 (m, 1H), 3.82 (d, J = 13.5 Hz, 1H), 4.40 (d, J = 13.0 Hz, 1H), 4.51 (q, J = 4.9 Hz, 1H), 5.32 (d, J = 4.9 Hz, 1H), 6.67 (d, J = 9.0 Hz, 1H), 6.99 (d, J = 8.3 Hz, 2H), 7.09 (dd, J = 8.9, 2.4 Hz, 1H), 7.18 (d, J = 8.3 Hz, 2H), 7.32 (d, J = 2.4 Hz, 1H).
MS : [M+H] = 528

### Exemple 60 : Synthèse de l'acide 4-hydroxy-1-pipéridin-4-ylméthyl-1,2,3,4-tétrahydro-quinoline-6-sulfonique (4-éthyl-phényl)-isobutyl-amide

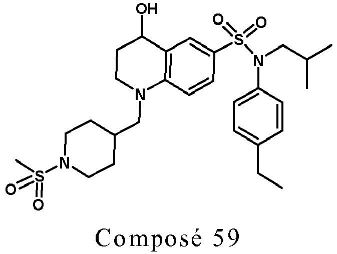

### 1. Synthèse de l'intermédiaire 60.1

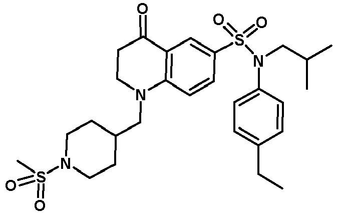

### Acide 1-(1-acétyl-piperidin-4-ylméthyl)-4-oxo-1,2,3,4-tétrahydro-quinoline-6-sulfonique (4-éthyl-phényl)-isobutyl-amide

Avec le même mode opératoire que pour l'intermédiaire 59.1, l'acide 1-(1-méthanesulfonyl-piperidin-4-ylméthyl)-4-oxo-1,2,3,4-tétrahydro-quinoline-6-sulfonique (4-éthyl-phényl)-isobutyl-amide (6 mg; 13 %) est obtenu sous la forme d'un solide beige avec une RMN¹H conforme.
MS : [M+H] = 562

### 2. Synthèse de l'acide 4-hydroxy-1-piperidin-4-ylmethyl-1,2,3,4-tetrahydro-quinoline-6-sulfonique (4-ethyl-phenyl)-isobutyl-amide

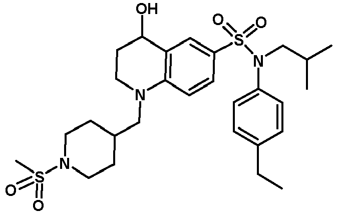

Avec le même mode opératoire que pour l'exemple 2, l'acide 4-hydroxy-1-(1-méthanesulfonyl-piperidin-4-ylméthyl)-1,2,3,4-tétrahydro-quinoline-6-sulfonique (4-éthyl-phényl)-isobutyl-amide (2.1 mg; 34%) est obtenu sous la forme d'un solide blanc.
1H NMR (DMSO-d6) δ: 0.83 (dd, J = 6.6, 2.6 Hz, 6H), 1.18 (t, J = 7.6 Hz, 3H), 1.22 - 1.36 (m, 2H), 1.36 - 1.47 (m, 1H), 1.68 - 1.90 (m, 5H), 2.57 - 2.66 (m, 4H), 2.84 (s, 3H), 3.16 - 3.29 (m, 4H), 3.29 - 3.37 (m, 1H), 3.40 - 3.49 (m, 1H), 3.57 (d, J = 11.7 Hz, 2H), 4.51 (q, J = 4.9 Hz, 1H), 5.32 (d, J = 4.9 Hz, 1H), 6.68 (d, J = 9.0 Hz, 1H), 6.97 - 7.02 (m, 2H), 7.09 (dd, J = 8.9, 2.4 Hz, 1H), 7.17 (d, J = 8.4 Hz, 2H), 7.32 (d, J = 2.2 Hz, 1H).
MS : [M+H] =564

### Exemple 61: Synthèse de l'acide 1-(1-Acetyl-piperidin-4-yl)-4-oxo-1,2,3,4-tetrahydro-quinoline-6-sulfonique (4-ethyl-phenyl)-isobutyl-amide

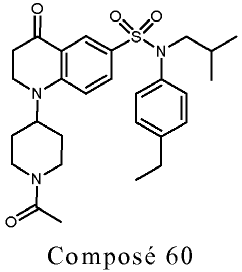

Avec le même mode opératoire que pour l'intermédiaire 59.1, l'acide 1-(1-acétyl-pipéridin-4-yl)-4-oxo-1,2,3,4-tetrahydro-quinoline-6-sulfonique (4-éthyl-phényl)-isobutyl-amide (45 mg; 68%) est obtenu sous la forme d'un solide jaune.
1H NMR (DMSO-d6) δ: 0.84 (d, J = 6.5 Hz, 6H), 1.19 (t, J = 7.6 Hz, 3H), 1.34 - 1.47 (m, 1H), 1.51 - 1.86 (m, 4H), 2.04 (s, 3H), 2.57 - 2.72 (m, 5H), 3.15 - 3.28 (m, 3H), 3.52 (t, J = 6.9 Hz, 2H), 3.92 (d, J = 13.7 Hz, 1H), 4.15 - 4.30 (m, 1H), 4.53 (d, J = 12.8 Hz, 1H), 7.02 (d, J = 8.1 Hz, 2H), 7.20 (d, J = 8.0 Hz, 2H), 7.28 (d, J = 9.3 Hz, 1H), 7.37 (dd, J = 9.3, 2.6 Hz, 1H), 7.82 (d, J = 2.6 Hz, 1H)
MS : [M+H] =512

### Exemple 62 : Synthèse de l'acide 1-(1-Acetyl-piperidin-4-yl)-4-hydroxy-1,2,3,4-tetrahydro-quinoline-6-sulfonique (4-ethyl-phenyl)-isobutyl-amide

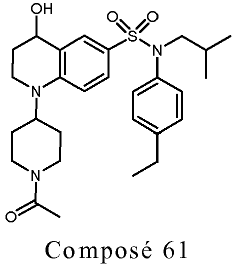

Avec le même mode opératoire que pour l'exemple 2, l'acide 1-(1-acétyl-pipéridin-4-yl)-4-hydroxy-1,2,3,4-tétrahydro-quinoline-6-sulfonique (4-éthyl-phényl)-isobutyl-amide (33mg; 87%) est obtenu sous la forme d'un solide blanc.
¹H NMR (DMSO-d6) δ: 0.83 (d, J = 6.5 Hz, 6H), 1.19 (t, J = 7.6 Hz, 3H), 1.34 - 1.47 (m, 1H), 1.47 - 1.86 (m, 6H), 2.03 (s, 3H), 2.56 - 2.65 (m, 3H), 3.13 - 3.30 (m, 5H), 3.90 (d, J = 13.4 Hz, 1H), 3.96 - 4.10 (m, 1H), 4.41 - 4.58 (m, 2H), 5.33 (d, J = 4.8 Hz, 1H), 6.87 (d, J = 9.0 Hz, 1H), 7.00 (d, J = 8.1 Hz, 2H), 7.11 (dd, J = 8.8, 2.5 Hz, 1H), 7.18 (d, J = 7.9 Hz, 2H), 7.36 (d, J = 2.4 Hz, 1H)).
MS : [M+H] = 514

### Exemple 63 : Acide 1-(1-acetyl-piperidin-4-yl)-4-oxo-1,2,3,4-tetrahydro-quinoline-6-sulfonique (4-ethyl-phenyl)-isobutyl-amide

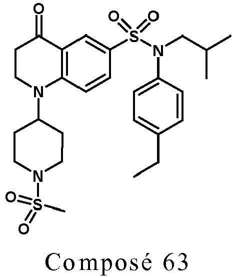

Avec le même mode opératoire que pour l'intermédiaire 59.1, l'acide 1-(1-méthanesulfonyl-piperidin-4-yl)-4-oxo-1,2,3,4-tetrahydro-quinoline-6-sulfonique (4-ethyl-phenyl)-isobutyl-amide (51 mg; 72 %) est obtenu sous la forme d'un solide jaune.
1H NMR (DMSO-d6) δ: 0.84 (d, J = 6.6 Hz, 6H), 1.18 (t, J = 7.6 Hz, 3H), 1.41 (hept, J = 6.7 Hz, 1H), 1.74 - 1.95 (m, 4H), 2.56 - 2.67 (m, 4H), 2.87 - 3.03 (m, 5H), 3.23 (d, J = 7.3 Hz, 2H), 3.56 (t, J = 6.8 Hz, 2H), 3.70 (d, J = 11.7 Hz, 2H), 4.05 - 4.17 (m, 1H), 7.01 (d, J = 8.0 Hz, 2H), 7.16 - 7.26 (m, 3H), 7.37 (dd, J = 9.2, 2.6 Hz, 1H), 7.82 (d, J = 2.7 Hz, 1H)
MS : [M+H] = 548

### Exemple 64 : Synthèse de l'acide 4-hydroxy-1-(1-methanesulfonyl-piperidin-4-yl)-1,2,3,4-tetrahydro-quinoline-6-sulfonique (4-éthyl-phényl)-isobutyl-amide

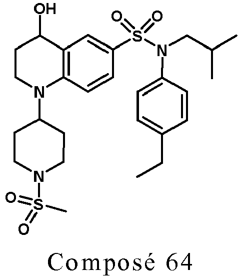

Avec le même mode opératoire que pour l'exemple 2, l'acide 4-hydroxy-1-(1-methanesulfonyl-piperidin-4-yl)-1,2,3,4-tétrahydro-quinoline-6-sulfonique (4-éthyl-phényl)-isobutyl-amide (2.1 mg; 34%) est obtenu sous la forme d'un solide blanc.
¹H NMR (DMSO-d6) δ: 0.83 (d, J = 6.7 Hz, 6H), 1.19 (t, J = 7.6 Hz, 3H), 1.32 - 1.48 (m, 1H), 1.70 - 1.94 (m, 6H), 2.61 (q, J = 7.6 Hz, 2H), 2.87 - 3.00 (m, 5H), 3.14 - 3.37 (m, 4H), 3.67 (d, J = 11.7 Hz, 2H), 3.86 - 3.99 (m, 1H), 4.48 (q, J = 5.0 Hz, 1H), 5.34 (d, J = 4.9 Hz, 1H), 6.82 (d, J = 9.3 Hz, 1H), 7.00 (d, J = 7.9 Hz, 2H), 7.10 (dd, J = 8.8, 2.4 Hz, 1H), 7.18 (d, J = 8.0 Hz, 2H), 7.37 (d, J = 2.4 Hz, 1H).
MS : [M+H] = 550

### Exemple 65: Synthèse de l'acide 4-Hydroxy-1-(1H-pyrazol-4-ylmethyl)-1,2,3,4-tetrahydro-quinoline-6-sulfonique isopropyl-(4-methoxy-2-methyl-phenyl)-amide

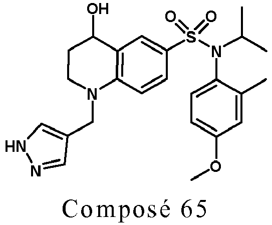

### 1. Synthèse de l'intermédiaire 65.1

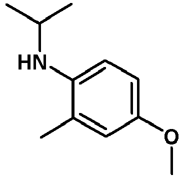

### Isopropyl-(4-méthoxy-2-méthyl-phenyl)-amine

A une solution de 4-méthoxy-2-methylaniline (2.0 g; 14.6 mmol) dans de l'acétone (20 ml) est ajouté le triacétoxy-borohydrure de sodium (4.63 g; 22 mmol). Le milieu réactionnel est chauffé 10 minutes au micro-onde à 70°C. Le milieu réactionnel est versé sur de la glace. La phase aqueuse est extraite au dichlorométhane. Les phases organiques sont rassemblées, lavées à la saumure, séchées (MgSO₄), filtrées et évaporées.

Le produit brut est purifié par chromatographié sur gel de silice (éluant heptane/acétate d'éthyle de 0 à 15% d'acétate d'éthyle). L'isopropyl-(4-methoxy-2-methyl-phenyl)-amine (1.43 g; 55%) est obtenue sous forme d'huile jaune avec une RMN¹H conforme.
MS : [M+H] = 180

### 2. Synthèse de l'intermédiaire 65.2

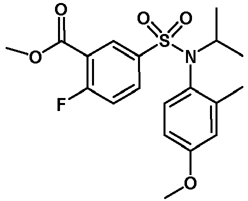

### 2-fluoro-5-[isopropyl-(4-méthoxy-2-méthyl-phényl)-sulfamoyl]-benzoate de méthyle

Dans un tube scellé, l'isopropyl-(4-methoxy-2-methyl-phenyl)-amine (1.0 g; 5.6 mmol) est ajoutée à une solution de 5-chlorosulfonyl-2-fluoro-benzoate de méthyle (1.41; 5.6 mmol) dans de la pyridine (4.0 ml; 0.05 mol. Le milieu réactionnel est chauffé pendant 40 minutes au micro-onde à une température de 100°C. Le milieu réactionnel est dilué avec de l'acétate d'éthyle.

La phase organique est lavée 3 fois avec une solution d'acide chlorhydrique 1N, puis une solution d'hydrogénocarbonate de sodium saturée et à l'eau, séchées sur sulfate de magnésium, filtrées et évaporées.

Le 2-fluoro-5-[isopropyl-(4-méthoxy-2-méthyl-phényl)-sulfamoyl]-benzoate de méthyle (1.41 g; 64 %) est obtenue sous forme d'huile brune avec une RMN¹H conforme.
MS : [M-H] = 396

### 3. Synthèse de l'intermédiaire 65.3

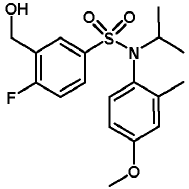

### 4-fluoro-3-hydroxymethyl-N-isopropyl-N-(4-methoxy-2-methyl-phenyl)-benzenesulfonamide

Avec le même mode opératoire que pour l'intermédiaire 1.5, le 4-fluoro-3-hydroxyméthyl-N-isopropyl-N-(4-méthoxy-2-méthyl-phényl)-benzenesulfonamide (0.47 g; 99%) est obtenu sous la forme d'une huile jaune avec une RMN¹H conforme.
MS : [M-H] = 366

### 4. Synthèse de l'intermédiaire 65.4

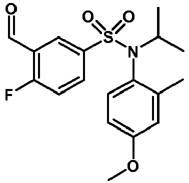

### fluoro-3-formyl-N-isopropyl-N-(4-methoxy-2-methyl-phenyl)-benzenesulfonamide

Avec le même mode opératoire que pour l'intermédiaire 1.6, le 4-fluoro-3-formyl-N-isopropyl-N-(4-méthoxy-2-méthyl-phényl)-benzenesulfonamide (0.42 g; 88%) est obtenu sous la forme d'une huile jaune avec une RMN¹H conforme.
MS : [M-H] =366

### 5. Synthèse de l'intermédiaire 65.5

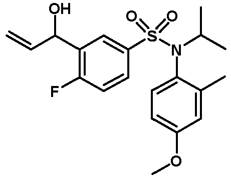

### 4-fluoro-3-(1-hydroxy-allyl)-N-isopropyl-N-(4-methoxy-2-methyl-phenyl)-benzenesulfonamide

Avec le même mode opératoire que pour l'intermédiaire 1.7, le 4-fluoro-3-(1-hydroxy-allyl)-N-isopropyl-N-(4-méthoxy-2-methyl-phenyl)-benzenesulfonamide (0.17 g; 42%) est obtenu sous la forme d'une huile jaune avec une RMN¹H conforme.
MS : [M-H] =394

### 6. Synthèse de l'intermédiaire 65.6

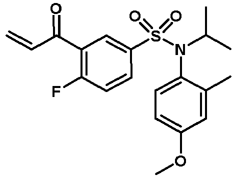

### 3-acryloyl-4-fluoro-N-isopropyl-N-(4-méthoxy-2-methyl-phenyl)-benzenesulfonamide

Avec le même mode opératoire que pour l'intermédiaire 1.6, le 3-acryloyl-4-fluoro-N-isopropyl-N-(4-méthoxy-2-méthyl-phényl)-benzenesulfonamide (0.16 g; 100%) est obtenu sous la forme d'une huile jaune avec une RMN¹H conforme.
MS : [M-H] =392

### 7. Synthèse de l'acide 4-hydroxy-1-(1H-pyrazol-4-ylmethyl)-1,2,3,4-tetrahydro-quinoline-6-sulfonique isopropyl-(4-methoxy-2-methyl-phenyl)-amide

A une solution de 3-acryloyl-4-fluoro-N-isopropyl-N-(4-méthoxy-2-méthyl-phényl)-benzenesulfonamide (0.17 g; 0.43 mmol) dans du N,N-diméthylformamide (850µl) sont ajoutées le dihydrochlorure de 1H-pyrazol-4-ylméthan-amine (0.11 g; 0.65 mmol) et la triéthylamine (270µl; 1.95 mmol) Le milieu réactionnel est agité pendant 20 minutes puis du borohydrure de sodium (0.11 g; 3 mmol) et du méthanol (680 µl) sont ajoutés.

Le milieu réactionnel est agité pendant 25 minutes puis est hydrolysé avec une solution aqueuse à 1 M de dihydrogénophosphate de sodium et extrait avec du dichlorométhane. Les phases organiques sont réunies, séchées sur sulfate de sodium anhydre, filtrées et concentrées.

Le produit brut est purifié par HPLC préparative (colonne C18, éluant : acétonitrile dans eau/0.1% d'acide formique).

L'acide 4-hydroxy-1-(1H-pyrazol-4-ylméthyl)-1,2,3,4-tétrahydro-quinoline-6-sulfonique isopropyl-(4-méthoxy-2-méthyl-phényl)-amide (15.00 mg; 7.31 %) est obtenu sous la forme d'un solide beige.
¹H NMR (DMSO-d6) δ: 0.87 (dd, J = 6.7, 3.1 Hz, 3H), 0.94 (dd, J = 6.7, 2.2 Hz, 3H), 1.15 (s, 2H), 1.79 - 1.96 (m, 2H), 2.24 (d, J = 8.9 Hz, 3H), 3.28 - 3.53 (m, 2H), 3.76 (s, 3H), 4.23 - 4.60 (m, 4H), 5.35 (dd, J = 5.0, 2.5 Hz, 1H), 6.64 - 6.78 (m, 2H), 6.84 (d, J = 8.9 Hz, 1H), 6.88 (t, J = 3.2 Hz, 1H), 7.27 (ddd, J = 8.2, 5.3, 2.4 Hz, 1H), 7.38 - 7.77 (m, 1H), 12.71 (s, 1H)
MS : [M-H] = 471

### Exemple 66 : Acide 4-Hydroxy-1-(tetrahydro-pyran-4-ylmethyl)-1,2,3,4-tetrahydro-quinoline-6-sulfonique (4,6-dimethyl-pyridin-3-yl)-isobutyl-amide

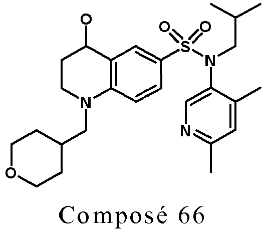

### 1. Synthèse de l'intermédiaire 66.1

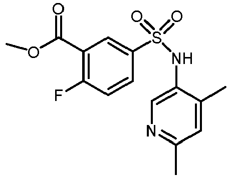

### 5-(4,6-diméthyl-pyridin-3-ylsulfamoyl)-2-fluoro-benzoate de méthyle

Avec le même mode opératoire que pour l'intermédiaire 65.2, le 5-(4,6-dimethyl-pyridin-3-ylsulfamoyl)-2-fluoro-benzoate de méthyle (1.40 g; 78 %) est obtenu sous la forme d'un solide blanc avec une RMN¹H conforme.
MS : [M+H] = 339

### 2. Synthèse de l'intermédiaire 66.2

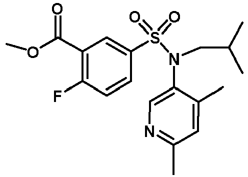

### 5-[(4,6-diméthyl-pyridin-3-yl)-isobutyl-sulfamoyl]-2-fluoro-benzoate de méthyle

Du 1-iodo-2-methyl-propane (1.30 ml; 11.3 mmol) est ajouté à un mélange de 5-(4,6-dimethyl-pyridin-3-ylsulfamoyl)-2-fluoro-benzoate de méthyle (1.24 g; 3.7 mmol) et de carbonate de césium (1.79 g; 5.5 mmol) dans de la 1-methyl-2-pyrrolidone (12 ml).

Le milieu réactionnel est chauffé pendant 1 heure à une température de 100°C, hydrolysé avec de l'eau.

La phase organique est séparée, séchée (MgSO₄), filtrée et concentrée. Le produit brut est chromatographié sur gel de silice (éluant heptane/acétate d'éthyle de 40 à 80% d'acétate d'éthyle). Le 5-[(4,6-dimethyl-pyridin-3-yl)-isobutyl-sulfamoyl]-2-fluoro- benzoate de méthyle (1.15 g; 80%) est obtenu sous la forme d'une huile orangeavec une RMN¹H conforme.
MS : [M+H] = 395

### 3. Synthèse de l'intermédiaire 66.3

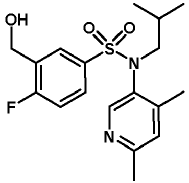

### N-(4,6-diméthyl-pyridin-3-yl)-4-fluoro-3-hydroxyméthyl-N-isobutyl-benzene-sulfonamide

Avec le même mode opératoire que pour l'intermédiaire 1.5, le N-(4,6-diméthyl-pyridin-3-yl)-4-fluoro-3-hydroxyméthyl-N-isobutyl-benzenesulfonamide (0.65 g; 100%) est obtenu sous la forme d'une huile jaune avec une RMN¹H conforme.
MS : [M+H] = 367

### 4. Synthèse de l'intermédiaire 66.4

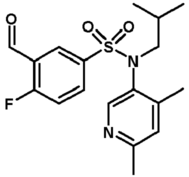

### N-(4,6-Dimethyl-pyridin-3-yl)-4-fluoro-3-formyl-N-isobutyl-benzenesulfonamide

Avec le même mode opératoire que pour l'intermédiaire 1.6, le N-(4,6-dimethyl-pyridin-3-yl)-4-fluoro-3-formyl-N-isobutyl-benzenesulfonamide (0.43 g; 67%) est obtenu sous la forme d'une huile incolore avec une RMN¹H conforme.
MS : [M+H] =365

### 5. Synthèse de l'intermédiaire 66.5

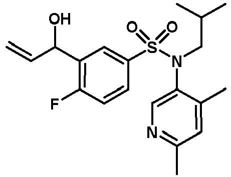

### N-(4,6-diméthyl-pyridin-3-yl)-4-fluoro-3-(1-hydroxy-allyl)-N-isobutyl-benzen-esulfonamide

Avec le même mode opératoire que pour l'intermédiaire 1.7, le N-(4,6-diméthyl-pyridin-3-yl)-4-fluoro-3-(1-hydroxy-allyl)-N-isobutyl-benzenesulfonamide (0.28 g; 60 %) est obtenu sous la forme d'une huile incolore. avec une RMN¹H conforme.
MS : [M+H] =393

### 6. Synthèse de l'intermédiaire 66.6

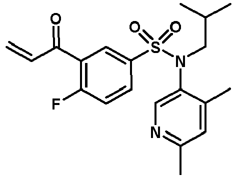

### 3-acryloyl-N-(4,6-diméthyl-pyridin-3-yl)-4-fluoro-N-isobutyl-benzenesulfonamide

Avec le même mode opératoire que pour l'intermédiaire 1.6, le 3-acryloyl-N-(4,6-diméthyl-pyridin-3-yl)-4-fluoro-N-isobutyl-benzenesulfonamide (0.08 g; 29%) est obtenu sous la forme d'une huile jaune avec une RMN¹H conforme.
MS : [M+H] =391

### 7. Synthèse de l'acide 4-hydroxy-1-(tetrahydro-pyran-4-ylmethyl)-1,2,3,4-tetrahydro-quinoline-6-sulfonique (4,6-dimethyl-pyridin-3-yl)-isobutyl-amide

Avec le même mode opératoire que pour l'exemple 65, l'acide 4-hydroxy-1-(tetrahydro-pyran-4-ylméthyl)-1,2,3,4-tétrahydro-quinoline-6-sulfonique (4,6-dimethyl-pyridin-3-yl)-isobutyl-amide (16 mg; 16%) est obtenu sous la forme d'un solide blanc cassé.
¹H NMR (DMSO-d6, 80°C) δ: 0.74 - 0.99 (m, 6H), 1.23 - 1.40 (m, 2H), 1.46 - 1.55 (m, 1H), 1.55 - 1.65 (m, 2H), 1.80 - 1.93 (m, 2H), 1.95 - 2.09 (m, 1H), 2.23 (s, 3H), 2.42 (s, 3H), 3.20 - 3.41 (m, 7H), 3.43 - 3.52 (m, 1H), 3.82 - 3.92 (m, 2H), 4.52 - 4.59 (m, 1H), 5.10 (d, J = 4.9 Hz, 1H), 6.71 (d, J = 8.9 Hz, 1H), 7.14 (s, 1H), 7.21 (d, J = 8.8 Hz, 1H), 7.44 (s, 1H), 7.78 - 7.93 (m, 1H)
MS : [M+H] = 488

## Revendications

1. Composé de formule (I), ses sels d'addition pharmaceutiquement acceptables, ses hydrates et/ou ses solvates : 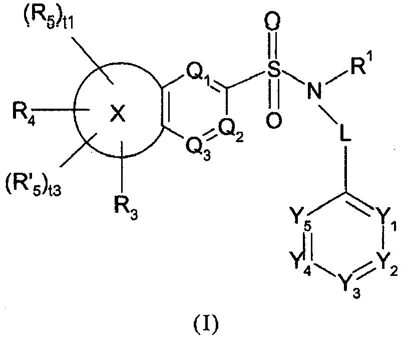 Formule (I) dans laquelle :
• L représente une liaison simple ou un groupe méthylène CH₂,
• X représente le radical cyclique suivant : 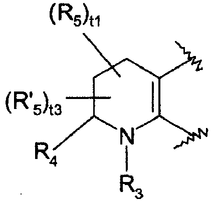
• un ou deux des éléments Y¹, Y², Y³, Y⁴ et Y⁵ représente(nt) un atome d'azote et les autres éléments correspondent à un groupement -CR², ou chacun des éléments Y¹, Y², Y³, Y⁴ et Y⁵ correspond à un groupement -CR²,
• un ou deux des éléments Q¹, Q² et Q³ représente(nt) un atome d'azote et le ou les autres éléments corresponde(nt) à un groupement -CR^{2a}, ou chacun des éléments Q¹, Q² et Q³ correspond à un groupement -CR^{2a},
• R¹ représente un radical alkyle, linéaire ou ramifié, en C₃-C₅, un radical cycloalkyle en C₃-C₅, un radical alkényle, linéaire ou ramifié, en C₂-C₅, un radical CH₂-cycloalkyle en C₃-C₅, un radical hétérocycloalkyle en C₄-C₅, un radical CH₂-hétérocycloalkyle en C₄-C₆,
• R² représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement cyano -CN, un radical -C(=O)R'² avec R'² désignant un radical alcoxy en C₁-C₃, un radical -CF₃ ; lesdits radicaux alkyle, alkényle et alcoxy pouvant être substitués par un ou plusieurs atomes d'halogène ;
• R^{2a} représente un atome d'hydrogène ou un atome d'halogène, un radical alkyle en C₁-C₅, linéaire ou ramifié, un radical alkényle en C₂-C₄, linéaire ou ramifié, un radical alcoxy en C₁-C₄, un groupement -CN, un groupement hydroxy -OH, un groupement-CH(R^{3a})OH, un groupement carboxylique -COOH, un groupement carbamoyle -CONR^{2c}R^{2d}, un groupement amido -NR^{2c}COR^{2d}, un groupement -SO₂R^{2c}, un groupement -SOR^{2c}, un groupement-S(=O)(=NH-R^{2c}), lesdits radicaux alkyle, alkényle et alcoxy pouvant être substitués par un ou plusieurs atomes d'halogène,
• R^{2c} et R^{2d}, identiques ou différents, représentent un atome d'hydrogène ou un radical alkyle, linéaire ou ramifié, en C₁-C₅ ;
• R^{3a} représente un atome d'hydrogène ou un radical alkyle, linéaire ou ramifié, en C₁-C₅ ;
• t₁ et t₃, identiques ou différents, désignent un entier naturel 0 ou 1,
• R³ et R⁴, identiques ou différents, représentent un atome d'hydrogène ou un groupement (CHR⁶)ₙ-(Z)ₒ-(CHR'⁶)ₚ-R⁷,,
• n, o et p, identiques ou différents, représentent zéro ou un entier naturel allant de 1 à 3,
• lorsque R³ représente un groupement (CHR⁶)ₙ-(Z)ₒ-(CHR'⁶)ₚ-R⁷, alors R⁴ représente un atome d'hydrogène,
• lorsque R⁴ représente un groupement (CHR⁶)ₙ-(Z)ₒ-(CHR'⁶)ₚ-R⁷ alors R³ représente un atome d'hydrogène,
• Z représente un groupement divalent choisi parmi un groupement méthylène -CH₂-, un groupement amino -NH- et un atome d'oxygène - O-,
• R⁶ et R'⁶, identiques ou différents, représentent un atome d'hydrogène, un groupement méthyle -CH₃, un groupement -OH, un groupement hydroxyméthyle, une fonction carboxylique -COOH,
• R⁷ représente :
- un atome d'hydrogène ou un atome d'halogène,
- un groupement COOR'⁷ avec R'⁷ désignant alkyl (C₁)hétérocycle(C₆),
- un radical hétérocycloalkyle non cationique éventuellement substitué par un ou plusieurs atomes d'halogène, un ou plusieurs groupements alkyle en C₁-C₃, linéaire ou ramifié, un ou plusieurs groupements -OH, une ou plusieurs fonctions carbonyles, un ou plusieurs groupements hydroxyalkyle en C₁-C₄, linéaire ou ramifié, un ou plusieurs groupements amino, un ou plusieurs groupements -C(=O)R^{7a}, un ou plusieurs groupements S(=O)₂R^{7a} ; R^{7a} représentant un radical alkyle, linéaire ou ramifié, en C₁-C₃, un radical alcoxy en C₁-C₃, linéaire ou ramifié, ou un radical amino N(R^{8a})(R^{9a}),
- un radical cycloalkyle non cationique en C₃-C₆ éventuellement substitué par un ou plusieurs radicaux méthyle, un ou plusieurs atomes d'halogène, un groupement cyano -CN ou un ou plusieurs groupements -COR¹³ ; R¹³ désignant un radical alcoxy en C₁-C₃, linéaire ou ramifié, ou un groupement hydroxy,
- un radical aromatique ou hétéroaromatique, non cationique, éventuellement substitué par un ou plusieurs atomes d'halogène, un ou plusieurs groupements alkyle en C₁-C₃, linéaire ou ramifié, éventuellement substitué par un ou plusieurs atomes d'halogène, un ou plusieurs groupements alcoxy en C₁-C₃, un ou plusieurs groupements amino -NR¹¹R¹², un ou plusieurs groupements -COR¹¹, un ou plusieurs groupements -COOR¹¹, un ou plusieurs groupements amido-CONR¹¹R¹², un ou plusieurs groupements -SOR¹¹, un ou plusieurs groupements -SO₂R¹¹, un ou plusieurs groupements-NHCOR¹¹, un ou plusieurs groupements -NHCOOR¹¹, un ou plusieurs groupements -SO₂NR¹¹R¹² ou un ou plusieurs groupements -CN ; R¹¹ et R¹², identiques ou différents, représentant un atome d'hydrogène, un radical hydroxy -OH, un radical alkyle, linéaire ou ramifié, en C₁-C₃, éventuellement substitué par un ou plusieurs atomes d'halogène,
• R⁵ représente un atome d'hydrogène ou un atome d'halogène, un radical alkyle en C₁-C₃, linéaire ou ramifié éventuellement substitué par un ou plusieurs atomes d'halogène; un radical amino-NH₂, un radical CH₂R'^{7a} avec R'^{7a} désignant un radical méthoxy, un groupement hydroxy -OH, un groupement -CH₂COOH, un groupement -CH(R^{5b})OH, un groupement carboxylique -COOH, un groupement-CN, une fonction thioxo,
• R'₅ représente une fonction carbonyle (C=O) ou une fonction thioxo (C=S),
• R^{8a} et R^{8b} identiques ou différents, désignent un atome d'hydrogène, un radical alkyle, linéaire ou ramifié, en C₁-C₃ ou un radical cyclopropyle.

2. Composé de formule (I) selon la revendication 1, **caractérisé en ce que** R⁷ représente un radical hétérocyclique choisi parmi les hétérocycles suivants : 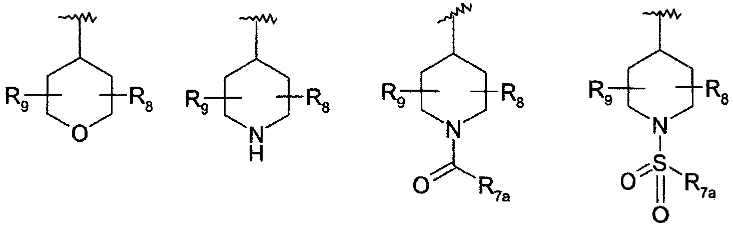 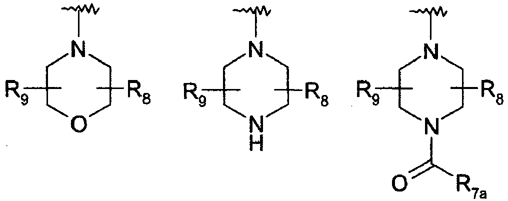 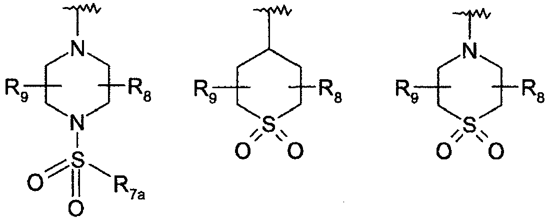 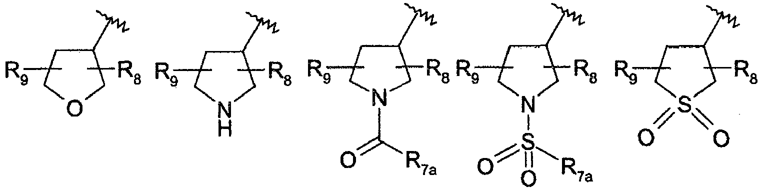 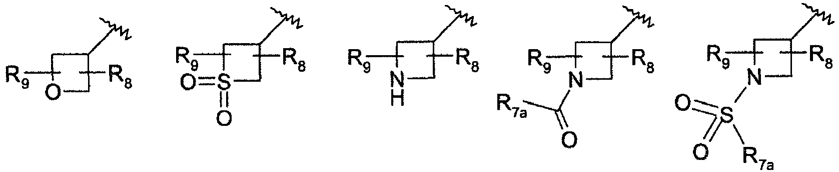 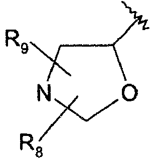 dans lesquels :
- R₇ₐ représente un radical alkyle, linéaire ou ramifié, en C₁-C₃, un radical alcoxy, linéaire ou ramifié, en C₁-C₃ ou un radical amino en N(R^{8a})(R^{8b}),
- R^{8a} et R^{8b}, identiques ou différents, désignent un atome d'hydrogène, un radical alkyle, linéaire ou ramifié, en C₁-C₃ ou un radical cyclopropyle,
- R₈ et R₉, identiques ou différents, représentent un atome d'hydrogène, un radical alkyle, linéaire ou ramifié, en C₁-C₃, un groupement hydroxy -OH, une fonction carbonyle =O, un radical hydroxyalkyle en C₁ (-CH₂OH), un groupe amino NH₂,
- R₈ et R₉ peuvent former ensemble avec les atomes de carbone auxquels ils sont attachés un cycle carbocyclique comportant de 5 à 7 chaînons,

3. Composé de formule (I) selon la revendication 1, **caractérisé en ce que** R⁷ représente un radical aromatique ou hétéroaromatique choisi parmi : 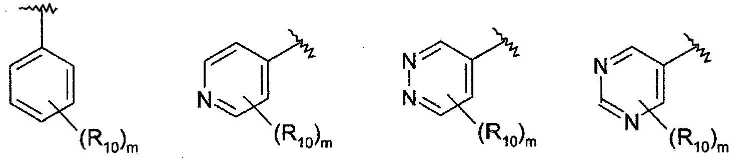 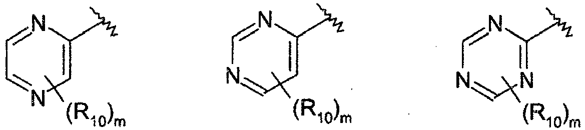 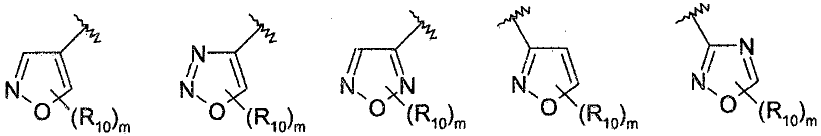 dans lesquels :
- R₁₀ représente un atome d'hydrogène ou un atome d'halogène, un groupement alkyle en C₁-C₃, linéaire ou ramifié, éventuellement substitué par un ou plusieurs atomes d'halogène ; un groupement alcoxy en C₁-C₃, un groupement amino -NR¹¹R¹², un groupement -COR¹¹, un groupement -COOR¹¹, un groupement amido-CONR¹¹R¹², un groupement -SOR¹¹, un groupement -SO₂R¹¹, un groupement -NHCOR¹¹, un groupement -NHCOOR¹¹, un groupement-SO₂NR¹¹R¹² ou un groupement -CN ; R¹¹ et R¹², identiques ou différents, représentant un atome d'hydrogène ou un radical alkyle, linéaire ou ramifié, en C₁-C₃ éventuellement substitué par un ou plusieurs atomes d'halogène,
m désigne zéro ou un entier naturel allant de 1 à 3.

4. Composé de formule (I) selon l'une quelconque des revendications précédentes, **caractérisé en ce que** lorsque t₁ est égal à zéro alors t₃ est égal à 1 et réciproquement lorsque t₁ est égal à 1 alors t₃ désigne zéro.

5. Composé de formule (I) selon l'une quelconque des revendications 1 à 4, **caractérisé en ce que** R'⁵ désigne une fonction carbonyle C=O.

6. Composé de formule (I) selon l'une quelconque des revendications précédentes, **caractérisé en ce que** R⁵ représente un groupement hydroxy -OH.

7. Composé de formule (I) selon la revendication 1, **caractérisé en ce qu'**il est choisi parmi le ou les composés de formule (V) ainsi que leurs sels d'addition pharmaceutiquement acceptables, leurs hydrates et/ou leurs solvates : 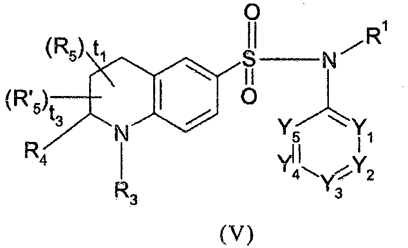 Formule (V) dans laquelle R¹, R³, R⁴, R⁵, R'⁵ et Y¹ à Y⁵, les indices t1 et t3 ont les mêmes significations que dans la formule (I) telle que définie selon l'une quelconque des reendications précédentes.

8. Composé de formule (I) selon la revendication 7, **caractérisé en ce qu'**il est choisi parmi le ou les composés de formule (V') ainsi que leurs sels d'addition pharmaceutiquement acceptables, leurs hydrates et/ou leurs solvates : 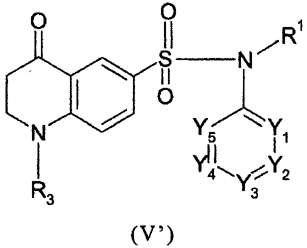 Formule (V') dans laquelle R¹, R³ et Y¹ à Y⁵ ont les mêmes significations que dans la formule (I) telle que définie selon l'une quelconque des revendications précédentes.

9. Composé de formule (I) selon la revendication 7, **caractérisé en ce qu'**il est choisi parmi le ou les composés de formule (V") ainsi que leurs sels d'addition pharmaceutiquement acceptables, leurs hydrates et/ou leurs solvates : 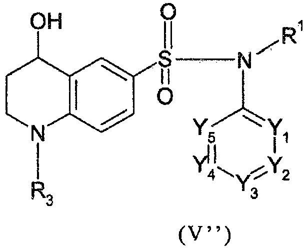 Formule (V") dans laquelle R¹, R³ et Y¹ à Y⁵ ont les mêmes significations que dans la formule (I) telle que définie selon l'une quelconque des revendications précédentes.

10. Composé de formule (I) selon l'une quelconque des revendications précédentes, **caractérisé en ce qu'**il est choisi parmi les composés suivants :
| | |
|---|---|
| 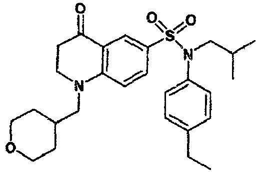 | Acide 4-oxo-1-(tétrahydro-pyran-4-ylméthyl)-1,2,3,4-tétrahydro-quinoline-6-sulfonique (4-éthyl-phényl)-isobutyl-amide |
| | Composé 1 |
| 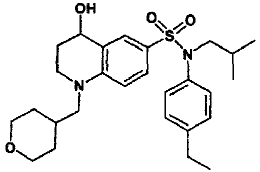 | Acide 4-hydroxy-1-(tetrahydro-pyran-4-ylmethyl)-1,2,3,4-tetrahydro-quinoline-6-sulfonique (4-éthyl-phényl)-isobutyl-amide |
| | Composé 2 |
| 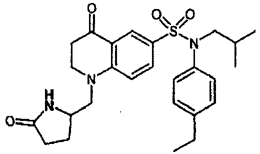 | Acide 4-oxo-1-(5-oxo-pyrrolidin-2-ylméthyl)-1,2,3,4-tétrahydro-quinoline-6-sulfonique (4-éthyl-phényl)-isobutyl-amide |
| | Composé 3 |
| 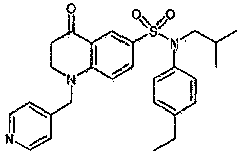 | Acide 4-oxo-1-pyridin-4-ylméthyl-1,2,3,4-tétrahydro-quinoline-6-sulfonique (4-éthyl-phényl)-isobutyl-amide |
| | Composé 4 |
| 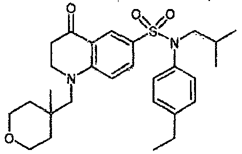 | Acide 1-(4-méthyl-tétrahydro-pyran-4-ylméthyl)-4-oxo-1,2,3,4-tétrahydro-quinoline-6-sulfonique (4-éthyl-phényl)-isobutyl-amide |
| | Composé 6 |
| 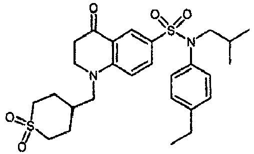 | Acide 1-(1,1-dioxo-hexahydro-1λ⁶-thiopyran-4-ylmethyl)-4-oxo-1,2,3,4-tetrahydro-quinoline-6-sulfonique (4-éthyl- phényl)-isobutyl-amide |
| | Composé 7 |
| 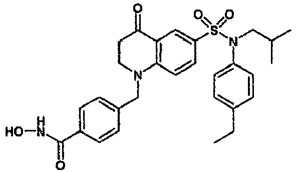 | 4-{6-[(4-éthyl-phényl)-isobutyl-sulfamoyl]-4-oxo-3,4-dihydro-2H-quinolin-1-ylméthyl}-N-hydroxy-benzamide |
| | Composé 8 |
| 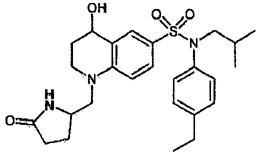 | Acide 4-hydroxy-1-(5-oxo-pyrrolidin-2-ylméthyl)-1,2,3,4-tetrahydro-quinoline-6-sulfonique (4-éthyl-phényl)-isobutyl-amide |
| | Composé 9 |
| 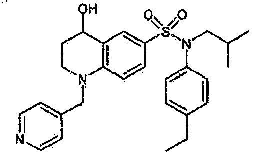 | Acide 4-hydroxy-1-pyridin-4-ylméthyl-1,2,3,4-tétrahydro-quinoline-6-sulfonique (4-éthyl-phényl)-isobutyl-amide |
| | Composé 10 |
| 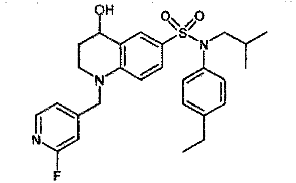 | Acide 1-(2-fluoro-pyridin-4-ylmethyl)-4-hydroxy-1,2,3,4-tetrahydro-quinoline-6-sulfonique (4-éthyl- phényl)-isobutyl-amide |
| | Composé 11 |
| 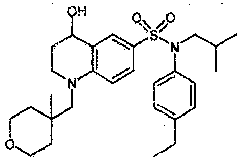 | Acide 1-(4-méthyl-tétrahydro-pyran-4-ylméthyl)-4-hydroxy-1,2,3,4-tétrahydro-quinoline-6-sulfonique (4-éthyl-phényl)-isobutyl-amide |
| | Composé 14 (Composé 218) |
| 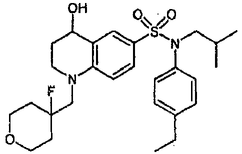 | Acide 1-(4-fluoro-tétrahydro-pyran-4-ylméthyl)-4-hydroxy-1,2,3,4-tétrahydro-quinoline-6-sulfonique (4-éthyl-phényl)-isobutyl-amide |
| | Composé 15 (Composé 219) |
| 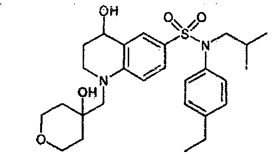 | Acide 4-hydroxy-1-(4-hydroxy-tétrahydro-pyran-4-ylméthyl)-1,2,3,4-tétrahydro-quinoline-6-sulfonique (4-éthyl-phényl)-isobutyl-amide |
| | Composé 16 |
| 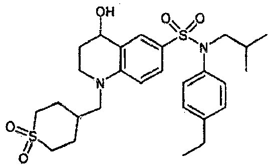 | Acide 1-(1,1-dioxo-hexahydro-1λ⁶-thiopyran-4-ylméthyl)-4-hydroxy-1,2,3,4-tetrahydro-quinoline-6-sulfonique (4-éthyl-phényl)-isobutyl-amide |
| | Composé 17 |
| 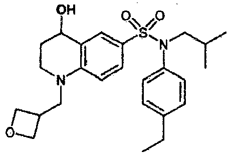 | Acide 4-hydroxy-1-oxetan-3-ylméthyl-1,2,3,4-tétrahydro-quinoline-6-sulfonique (4-éthyl-phényl)-isobutyl-amide |
| | Composé 18 |
| 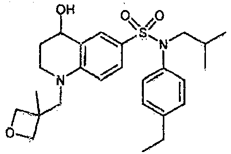 | Acide 4-hydroxy-1-(3-méthyl-oxetan-3-ylméthyl)-1,2,3,4-tetrahydro-quinoline-6-sulfonique (4-éthyl-phényl)-isobutyl-amide |
| | Composé 19 |
| 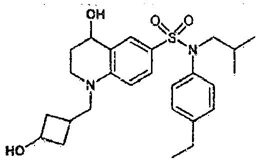 | Acide 4-hydroxy-1-(3-hydroxy-cyclobutylmethyl)-1,2,3,4-tetrahydro-quinoline-6-sulfonique (4-éthyl-phényl)-isobutyl-amide mélange de diastéréoisomères : |
| | Composé 20 |
| 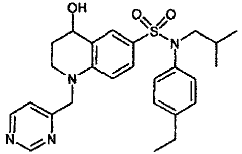 | Acide 4-hydroxy-1-pyrimidin-4-ylméthyl-1,2,3,4-tétrahydro-quinoline-6-sulfonique (4- éthyl-phényl)-isobutyl-amide |
| | Composé 21 |
| 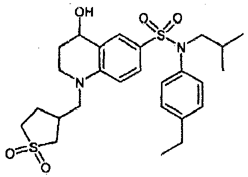 | Acide 1-(1,1-Dioxo-tétrahydro-1λ⁶-thiophen-3-ylméthyl)-4-hydroxy-1,2,3,4-tétrahydro-quinoline-6-sulfonique (4- éthyl-phényl)-isobutyl-amide |
| | Composé 22 |
| 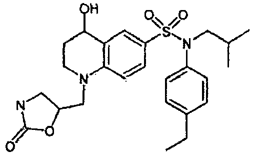 | Acide 4-hydroxy-1-(2-oxo-oxazolidin-5-ylméthyl)-1,2,3,4-tétrahydro-quinoline-6-sulfonique (4-éthyl-phényl)-isobutyl-amide |
| | Composé 23 |
| 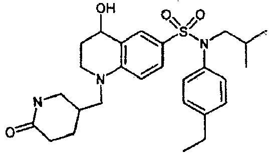 | Acide 4-hydroxy-1-(6-oxo-piperidin-3-ylméthyl)-1,2,3,4-tétrahydro-quinoline-6-sulfonique (4-éthyl-phényl)-isobutyl-amide |
| | Composé 24 |
| 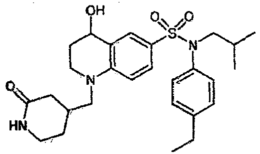 | Acide 4-hydroxy-1-(2-oxo-piperidin-4-ylmethyl)-1,2,3,4-tétrahydro-quinoline-6-sulfonique (4-éthyl-phényl)-isobutyl-amide |
| | Composé 25 |
| 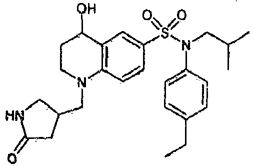 | Acide 4-hydroxy-1-(5-oxo-pyrrolidin-3-ylméthyl)-1,2,3,4-tétrahydro-quinoline-6-sulfonique (4-éthyl-phényl)-isobutyl-amide |
| | Composé 26 |
| 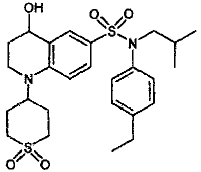 | Acide 1-(1,1-dioxo-hexahydro-1λ⁶-thiopyran-4-yl)-4-hydroxy-1,2,3,4-tétrahydro-quinoline-6-sulfonique (4-éthyl-phényl)-isobutyl-amide |
| | Composé 27 |
| 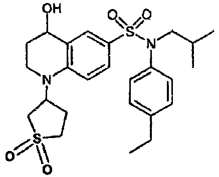 | Acide 1-(1,1-dioxo-tétrahydro-1lambda*6*-thiophen-3-yl)-4-hydroxy-1,2,3,4-tétrahydro-quinoline-6-sulfonique (4-éthyl-phényl)-isobutyl-amide |
| | Composé 28 |
| 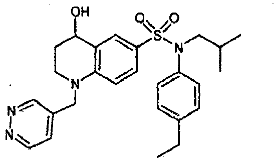 | Acide 4-hydroxy-1-pyridazin-4-ylmethyl-1,2,3,4-tetrahydro-quinoline-6-sulfonique (4 éthyl-phényl)-isobutyl-amide |
| | Composé 29 |
| 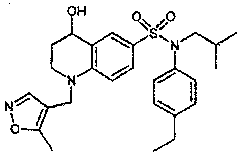 | Acide 4-hydroxy-1-(5-methyl-isoxazol-4-ylméthyl)-1,2,3,4-tetrahydro-quinoline-6-sulfonique (4-éthyl-phényl)-isobutyl-amide |
| | Composé 30 |
| 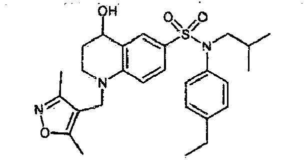 | Acide 1-(3,5-Diméthyl-isoxazol-4-ylmethyl)-4-hydroxy-1,2,3,4-tetrahydro-quinoline-6-sulfonique (4-éthyl-phényl)-isobutyl-amide |
| | Composé 31 |
| 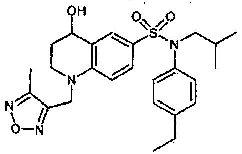 | Acide 4-hydroxy-1-(4-methyl-furazan-3-ylmethyl)-1,2,3,4-tétrahydro-quinoline-6-sulfonique (4-éthyl-phényl)- isobutyl-amide |
| | Composé 32 |
| 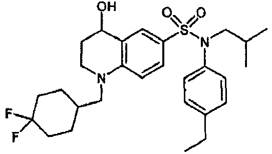 | Acide 1-(4,4-difluoro-cyclohexylmethyl)-4-hydroxy-1,2,3,4-tetrahydro-quinoline-6-sulfonique (4-éthyl-phényl)-isobutyl-amide |
| | Composé 33 |
| 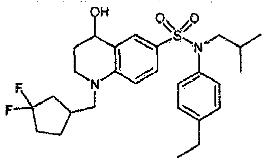 | Acide 1-(3,3-difluoro-cyclopentylmethyl)-4-hydroxy-1,2,3,4-tetrahydro-quinoline-6-sulfonique (4- éthyl-phényl)-isobutyl-amide |
| | Composé 34 |
| 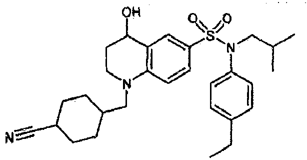 | Acide 1-(4-cyano-cyclohexylmethyl)-4-hydroxy-1,2,3,4-tetrahydro-quinoline-6-sulfonique (4-éthyl-phényl)-isobutyl-amide |
| | Composé 35 |
| 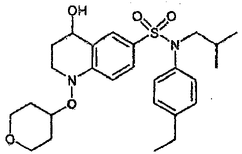 | Acide 4-hydroxy-1-(tétrahydro-pyran-4-yloxy)-1,2,3,4-tétrahydro-quinoline-6-sulfonique (4-éthyl-phényl)-isobutyl-amide |
| | Composé 36 |
| 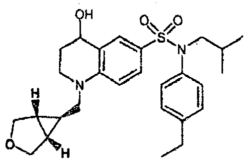 | Acide 4-hydroxy-1-[(1S,5R,6S)-1-(3-oxa-bicyclo-[3.1.0]hex-6-yl)methyl]-1,2,3,4-tetrahydro-quinoline-6-sulfonique (4-éthyl-phényl)-isobutyl-amide |
| | Composé 37 |
| 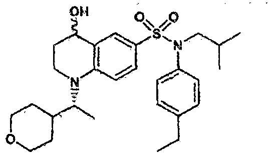 | Acide 4-hydroxy-1-[(R)-1-(tétrahydro-pyran-4-yl)-ethyl]-1,2,3,4-tétrahydro-quinoline-6-sulfonique (4-éthyl-phényl)-isobutyl-amide |
| | Composé 38 |
| 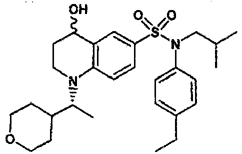 | Acide 4-hydroxy-1-[(R)-1-(tétrahydro-pyran-4-yl)-éthyl]-1,2,3,4-tétrahydro-quinoline-6-sulfonique (4-éthyl-phényl)-isobutyl-amide |
| | Composé 39 |
| 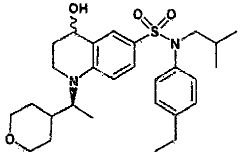 | Acide 4-hydroxy-1-[(S)-1-(tétrahydro-pyran-4-yl)-éthyl]-1,2,3,4-tétrahydro-quinoline-6-sulfonique (4- éthyl-phényl)-isobutyl-amide |
| | Composé 40 |
| 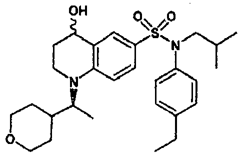 | 4-hydroxy-1-[(S)-1-(tétrahydro-pyran-4-yl)-éthyl]-1,2,3,4-tetrahydro-quinoline-6-sulfonique (4-éthyl-phényl)-isobutyl-amide |
| | Composé 41 |
| 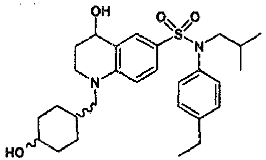 | Acide 4-hydroxy-1-(4-hydroxy-cyclohexylmethyl)-1,2,3,4-tetrahydro-quinoline-6-sulfonique (4-éthyl-phényl)-isobutyl-amide |
| | Composé 42 |
| 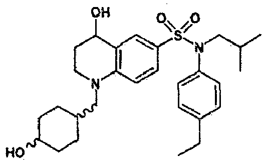 | Acide 4-hydroxy-1-(4-hydroxy-cyclohexylmethyl)-1,2,3,4-tetrahydro-quinoline-6-sulfonique (4-éthyl-phényl)-isobutyl-amide |
| | Composé 43 |
| 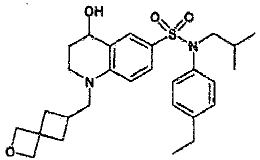 | Acide 4-hydroxy-1-(2-oxa-spiro[3.3]hept-6-ylmethyl)-1,2,3,4-tetrahydro-quinoline-6-sulfonique (4-éthyl-phényl)-isobutyl-amide |
| | Composé 44 |
| 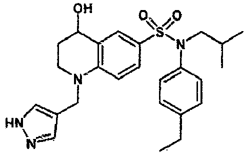 | Acide 4-hydroxy-1-(1H-pyrazol-4-ylméthyl)-1,2,3,4-tétrahydro-quinoline-6-sulfonique (4-éthyl-phényl)-isobutyl-amide |
| | Composé 45 |
| 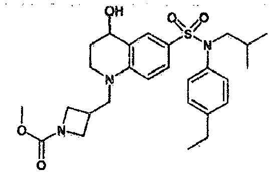 | Ester de l'acide méthyl 3-{6-[(4-éthyl-phényl)-isobutyl-sulfamoyl]-4-hydroxy-3,4-dihydro-2H-quinolin-1-ylméthyl}-azetidine-1-carboxylique |
| | Composé 46 |
| 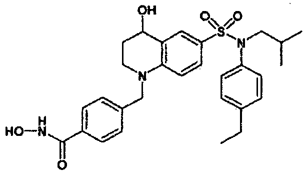 | 4-{6-[(4-éthyl-phényl)-isobutyl-sulfamoyl]-4-hydroxy-3,4-dihydro-2H-quinolin-1-ylméthyl}-N-hydroxy-benzamide |
| | Composé 47 |
| 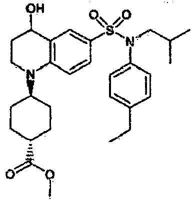 | méthyl (1r,4r)-4-(6-(N-(4-éthylphényl)-N-isobutylsulfamoyl)-4-hydroxy-3,4-dihydroquinolin-1(2H)-yl)cyclohexane-1-carboxylate |
| | Composé 48 |
| 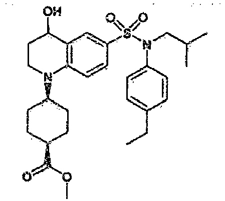 | Méthyl (1s,4s)-4-(6-(N-(4-ethylphenyl)-N-isobutylsulfamoyl)-4-hydroxy-3,4-dihydroquinolin-1(2H)-yl)cyclohexane-1-carboxylate |
| | Composé 49 |
| 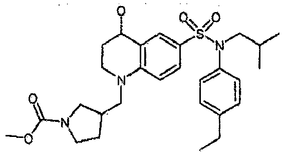 | Ester de l'acide méthyl carboxylique 3-{6-[(4-éthyl-phényl)-isobutyl-sulfamoyl]-4-hydroxy-3,4-dihydro-2H-quinolin-1-ylméthyl}-pyrrolidine-1 |
| | Composé 50 |
| 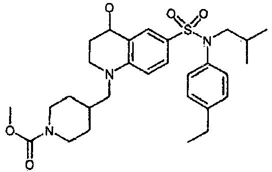 | Ester de l'acide méthyl carboxylique 4-{6-[(4-Ethyl-phenyl)-isobutyl-sulfamoyl]-4-hydroxy-3,4-dihydro-2H-quinolin-1-ylmethyl}-piperidine-1 |
| | Composé 51 (Composé 255) |
| 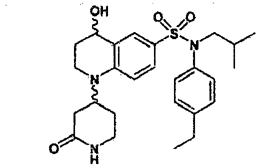 | **Diastéréoisomère 1** Acide 4-hydroxy-1-(2-oxo-pipéridin-4-yl)-1,2,3,4-tétrahydro-quinoline-6-sulfonique (4-éthyl-phényl)-isobutyl-amide |
| | Composé 52 |
| 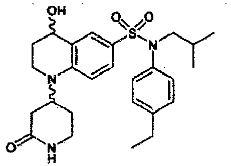 | **Diastéréoisomère 2** Acide 4-Hydroxy-1-(2-oxo-piperidin-4-yl)-1,2,3,4-tétrahydro-quinoline-6-sulfonique (4-éthyl-phényl)-isobutyl-amide |
| | Composé 53 |
| 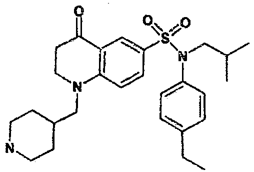 | Acide 4-oxo-1-piperidin-4-ylméthyl-1,2,3,4-tétrahydro-quinoline-6-sulfonique (4-éthyl-phényl)-isobutyl-amide |
| | Composé 54 |
| 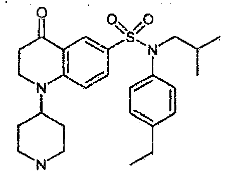 | Acide 4-oxo-1-piperidin-4-yl-1,2,3,4-tétrahydro-quinoline-6-sulfonique (4-éthyl-phényl)-isobutyl-amide |
| | Composé 55 |
| 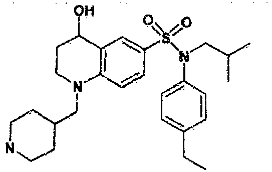 | Acide 4-hydroxy-1-piperidin-4-ylmethyl-1,2,3,4-tetrahydro-quinoline-6-sulfonique (4-éthyl-phényl)-isobutyl-amide |
| | Composé 56 |
| 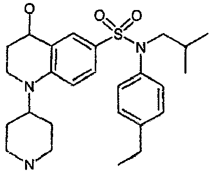 | Acide 4-hydroxy-1-piperidin-4-yl-1,2,3,4-tetrahydro-quinoline-6-sulfonique (4-éthyl-phényl)-isobutyl-amide |
| | Composé 57 |
| 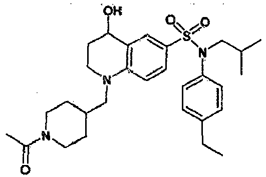 | Acide 4-hydroxy-1-piperidin-4-ylméthyl-1,2,3,4-tetrahydro-quinoline-6-sulfonique (4-éthyl-phényl)-isobutyl-amide |
| | Composé 58 |
| 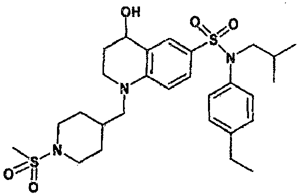 | Acide 4-hydroxy-1-pipéridin-4-ylméthyl-1,2,3,4-tétrahydro-quinoline-6-sulfonique (4-éthyl-phényl)-isobutyl-amide |
| | Composé 59 |
| 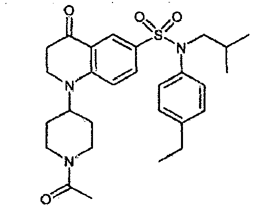 | Acide 1-(1-acétyl-pipéridin-4-yl)-4-oxo-1,2,3,4-tétrahydro-quinoline-6-sulfonique (4-éthyl-phényl)-isobutyl-amide |
| | Composé 60 |
| 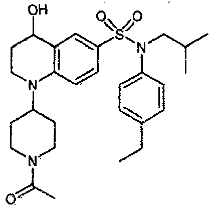 | Acide 1-(1-acétyl-piperidin-4-yl)-4-hydroxy-1,2,3,4-tetrahydro-quinoline-6-sulfonique (4-éthyl-phényl)-isobutyl-amide |
| | Composé 61 |
| 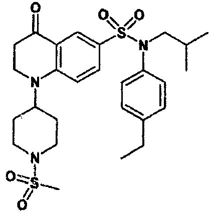 | Acide 1-(1-acétyl-piperidin-4-yl)-4-oxo-1,2,3,4-tetrahydro-quinoline-6-sulfonique (4-éthyl-phényl)-isobutyl-amide |
| | Composé 62 |
| 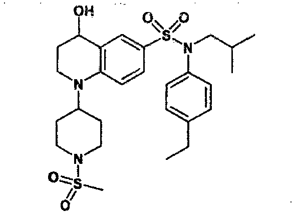 | Acide 4-hydroxy-1-(1-methanesulfonyl-piperidin-4-yl)-1,2,3,4-tétrahydro-quinoline-6-sulfonique (4-éthyl-phényl)-isobutyl-amide |
| | Composé 64 |
| 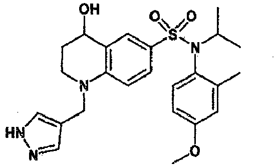 | Acide 4-hydroxy-1-(1H-pyrazol-4-ylmethyl)-1,2,3,4-tetrahydro-quinoline-6-sulfonique isopropyl-(4-méthoxy-2-methyl-phényl)-amide |
| | Composé 65 |
| 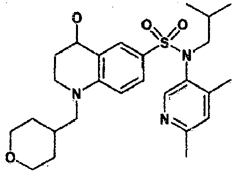 | Acide 4-Hydroxy-1-(tétrahydro-pyran-4-ylméthyl)-1,2,3,4-tétrahydro-quinoline-6-sulfonique (4,6-diméthyl-pyridin-3-yl)-isobutyl-amide |
| | Composé 66 |

11. Composé selon l'une quelconque des revendications précédentes pour son utilisation en tant que médicament.

12. Composé selon l'une quelconque des revendications 1 à 10 pour son utilisation dans le traitement de l'acné.

13. Composé(s) selon l'une quelconque des revendications 1 à 10 pour son utilisation dans le traitement du psoriasis.

14. Composition pharmaceutique comprenant un ou plusieurs composés tels que définis selon l'une quelconque des revendications 1 à 10.

15. Composition pharmaceutique telle que définie selon la revendication 14 pour son utilisation dans le traitement de l'acné, la dermatite atopique et/ou le psoriasis.

## Patentansprüche

1. Verbindung der Formel (I), pharmazeutisch unbedenkliche Additionssalze davon, Hydrate davon und/oder Solvate davon: 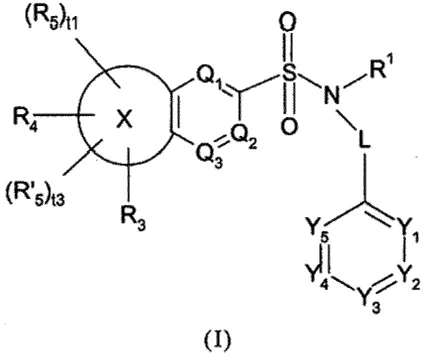 wobei in der Formel (I):
• L für eine Einfachbindung oder eine Methylengruppe CH₂ steht,
• X für den folgenden cyclischen Rest steht: 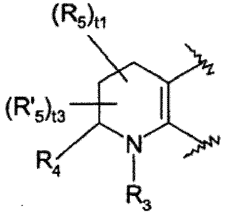
• ein oder zwei der Elemente Y¹, Y², Y³, Y⁴ und Y⁵ für ein Stickstoffatom steht bzw. stehen und die anderen Elemente einer Gruppe -CR² entsprechen oder jedes der Elemente Y¹, Y², Y³, Y⁴ und Y⁵ einer Gruppe -CR² entspricht,
• ein oder zwei der Elemente Q¹, Q² und Q³ für ein Stickstoffatom steht bzw. stehen und das bzw. die anderen Elemente einer Gruppe -CR^{2a} entspricht bzw. entsprechen oder jedes der Elemente Q¹, Q² und Q³ einer Gruppe -CR^{2a} entsprechen,
• R¹ für einen linearen oder verzweigten C₃-C₅-Alkylrest, einen C₃-C₅-Cycloalkylrest, einen linearen oder verzweigten C₂-C₅-Alkenylrest, einen CH₂-C₃-C₅-Cycloalkylrest, einen C₄-C₅-Heterocycloalkylrest oder einen CH₂-C₄-C₆-Heterocycloalkylrest steht,
• R² für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest, eine Cyanogruppe-CN, einen Rest -C(=O)R'², wobei R'² einen C₁-C₃-Alkoxyrest bedeutet, oder einen -CF₃-Rest steht; wobei die Alkyl-, Alkenyl- und Alkoxyreste durch ein oder mehrere Halogenatome substituiert sein können;
• R^{2a} für ein Wasserstoffatom oder ein Halogenatom, einen linearen oder verzweigten C₁-C₅-Alkylrest, einen linearen oder verzweigten C₂-C₄-Alkenylrest, einen C₁-C₄-Alkoxyrest, eine -CN-Gruppe, eine Hydroxygruppe -OH, eine Gruppe -CH(R^{3a})OH, eine Carboxylgruppe -COOH, eine Carbamoylgruppe -CONR^{2c}R^{2d}, eine Amidogruppe -NR^{2c}COR^{2d}, eine Gruppe -SO₂R^{2c}, eine Gruppe -SOR^{2c} oder eine Gruppe -S(=O)(=NH-R^{2c}) steht, wobei die Alkyl-, Alkenyl- und Alkoxyreste durch ein oder mehrere Halogenatome substituiert sein können,
• R^{2c} und R^{2d} gleich oder verschieden sind und für ein Wasserstoffatom oder einen linearen oder verzweigten C₁-C₅-Alkylrest stehen;
• R^{3a} für ein Wasserstoffatom oder einen linearen oder verzweigten C₁-C₅-Alkylrest steht;
• t₁ und t₃ gleich oder verschieden sind und eine natürliche Zahl mit einem Wert von 0 oder 1 bedeuten,
• R³ und R⁴ gleich oder verschieden sind und für ein Wasserstoffatom oder eine Gruppe (CHR⁶)ₙ-(Z)ₒ-(CHR'⁶)ₚ-R⁷ stehen,
• n, o und p gleich oder verschieden sind und für null oder eine natürliche ganze Zahl im Bereich von 1 bis 3 stehen,
• dann, wenn R³ für eine Gruppe (CHR⁶)ₙ-(Z)ₒ-(CHR'⁶)ₚ-R⁷ steht, R⁴ für ein Wasserstoffatom steht,
• dann, wenn R⁴ für eine Gruppe (CHR⁶)ₙ-(Z)ₒ-(CHR'⁶)ₚ-R⁷ steht, R³ für ein Wasserstoffatom steht,
• Z für eine zweiwertige Gruppe steht, die aus einer Methylengruppe -CH₂-, einer Aminogruppe -NH- und einem Sauerstoffatom -O- ausgewählt ist,
• R⁶ und R'⁶ gleich oder verschieden sind und für ein Wasserstoffatom, eine Methylgruppe -CH₃, eine -OH-Gruppe, eine Hydroxymethylgruppe oder eine Carboxylfunktion -COOH stehen,
• R⁷ für:
- ein Wasserstoffatom oder ein Halogenatom,
- eine Gruppe COOR'⁷, wobei R'⁷ (C₁)Alkyl(C₆)-heterocyclus bedeutet,
- einen nichtkationischen Heterocycloalkylrest, der gegebenenfalls durch ein oder mehrere Halogenatome, eine oder mehrere lineare oder verzweigte C₁-C₃-Alkylgruppen, eine oder mehrere -OH-Gruppen, eine oder mehrere Carbonylfunktionen, eine oder mehrere lineare oder verzweigte C₁-C₄-Hydroxyalkylgruppen, ein oder mehrere Amino, eine oder mehrere Gruppen -C(=O)R^{7a} oder eine oder mehrere Gruppen S(=O)₂R^{7a} substituiert ist; wobei R^{7a} für einen linearen oder verzweigten C₁-C₃-Alkylrest, einen linearen oder verzweigten C₁-C₃-Alkoxyrest oder einen Aminorest N(R^{8a})(R^{9a}) steht,
- einen nichtkationischen C₃-C₆-Cycloalkylrest, der gegebenenfalls durch einen oder mehrere Methylreste, ein oder mehrere Halogenatome, eine Cyanogruppe -CN oder eine oder mehrere Gruppen COR¹³ substituiert ist; wobei R¹³ für einen linearen oder verzweigten C₁-C₃-Alkoxyrest oder eine Hydroxygruppe steht,
- einen nichtkationischen aromatischen oder heteroaromatischen Rest, der gegebenenfalls durch ein oder mehrere Halogenatome, eine oder mehrere C₁-C₃-Alkylgruppen, die gegebenenfalls durch ein oder mehrere Halogenatome substituiert sind, eine oder mehrere C₁-C₃-Alkoxygruppen, eine oder mehrere Aminogruppen -NR¹¹R¹², eine oder mehrere Grippen -COR¹¹, eine oder mehrere Gruppen -COOR¹¹, eine oder mehrere Amidogruppen -CONR¹¹R¹², eine oder mehrere Gruppen -SOR¹¹, eine oder mehrere Gruppen -SO₂R¹¹, eine oder mehrere Gruppen -NHCOR¹¹, eine oder mehrere Gruppen -NHCOOR¹¹, eine oder mehrere Gruppen -SO₂NR¹¹R¹² oder eine oder mehrere -CN-Gruppen substiuiert ist; wobei R¹¹ und R¹² gleich oder verschieden sind und für ein Wasserstoffatom, einen Hydroxyrest -OH oder einen linearen oder verzweigten C₁-C₃-Alkylrest, der gegebenenfalls durch ein oder mehrere Halogenatome substituiert ist, stehen, steht,
• R⁵ für ein Wasserstoffatom oder ein Halogenatom, einen linearen oder verzweigten C₁-C₃-Alkylrest, der gegebenenfalls durch ein oder mehrere Halogenatome substituiert ist, einen Aminorest -NH₂ oder einen Rest CH₂R'^{7a} steht, wobei R'^{7a} einen Methoxyrest, eine Hydroxygruppe -OH, eine Gruppe -CH₂COOH, eine Gruppe -CH(R^{5b})OH, eine Carboxylgruppe -COOH, eine -CN-Gruppe oder eine Thioxofunktion bedeutet,
• R'₅ für eine Carbonylfunktion (C=O) oder eine Thioxofunktion (C=S) steht,
• R^{8a} und R^{8b} gleich oder verschieden sind und ein Wasserstoffatom, einen linearen oder verzweigten C₁-C₃-Alkylrest oder einen Cyclopropylrest bedeuten.

2. Verbindung der Formel (I) nach Anspruch 1, **dadurch gekennzeichnet, dass** R⁷ für einen heterocyclischen Rest steht, der aus den folgenden Heterocyclen ausgewählt ist: 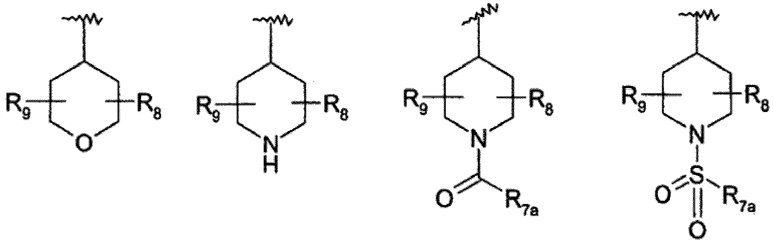 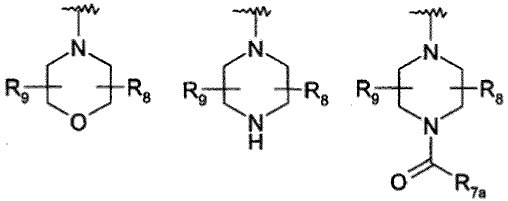 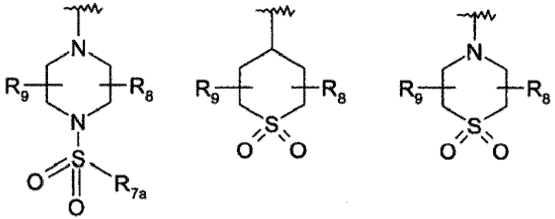 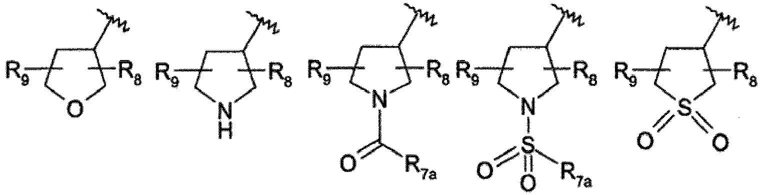 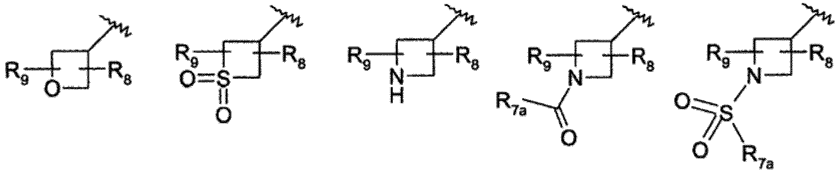 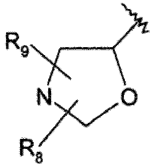 in denen:
- R₇ₐ für einen linearen oder verzweigten C₁-C₃-Alkylrest, einen linearen oder verzweigten C₁-C₃-Alkoxyrest oder einen Aminorest N(R^{8a})(R^{8b}) steht,
- R^{8a} und R^{8b} gleich oder verschieden sind und ein Wasserstoffatom, einen linearen oder verzweigten C₁-C₃-Alkylrest oder einen Cyclopropylrest bedeuten,
- R₈ und R₉ gleich oder verschieden sind und für ein Wasserstoffatom, einen linearen oder verzweigten C₁-C₃-Alkylrest, eine Hydroxygruppe -OH, eine Carbonylfunktion =O, einen C₁-Hydroxyalkylrest (-CH₂OH) oder eine Aminogruppe NH₂ stehen,
- R₈ und R₉ zusammen mit den Kohlenstoffatomen, an die sie gebunden sind, einen 5- bis 7-gliedrigen heterocyclischen Ring bilden können.

3. Verbindung der Formel (I) nach Anspruch 1, **dadurch gekennzeichnet, dass** R⁷ für einen aromatischen oder heteroaromatischen Rest steht, der aus den folgenden Resten ausgewählt ist: 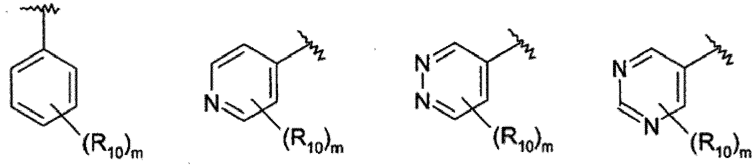 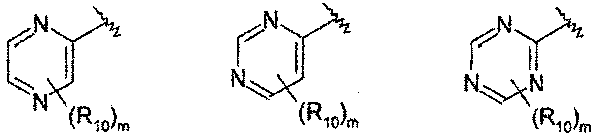 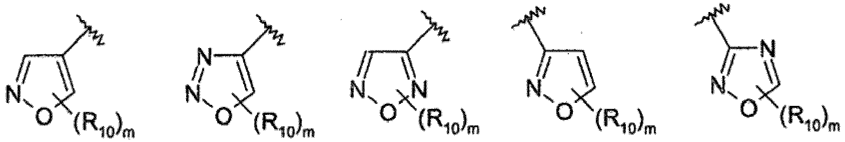 in denen:
- R₁₀ für ein Wasserstoffatom oder ein Halogenatom, eine C₁-C₃-Alkylgruppe, die gegebenenfalls durch ein oder mehrere Halogenatome substituiert ist, eine C₁-C₃-Alkoxygruppe, eine Aminogruppe -NR¹¹R¹², eine Grippe -COR¹¹, eine Gruppe -COOR¹¹, eine Amidogruppe -CONR¹¹R¹², eine Gruppe -SOR¹¹, eine Gruppe -SO₂R¹¹, eine Gruppe -NHCOR¹¹, eine Gruppe -NHCOOR¹¹, eine Gruppe -SO₂NR¹¹R¹² oder eine -CN-Gruppe substiuiert ist; wobei R¹¹ und R¹² gleich oder verschieden sind und für ein Wasserstoffatom oder einen linearen oder verzweigten C₁-C₃-Alkylrest, der gegebenenfalls durch ein oder mehrere Halogenatome substituiert ist, stehen,
- m für null oder eine natürliche ganze Zahl im Bereich von 1 bis 3 steht.

4. Verbindung der Formel (I) nach einem der vorhergehenden Ansprüche, **dadurch gekennzeichnet, dass** dann, wenn t₁ gleich null ist, t₃ gleich 1 ist, und umgekehrt dann, wenn t₁ gleich 1 ist, t₃ gleich null ist.

5. Verbindung der Formel (I) nach einem der Ansprüche 1 bis 4, **dadurch gekennzeichnet, dass** R'⁵ für eine Carbonylfunktion C=O steht.

6. Verbindung der Formel (I) nach einem der vorhergehenden Ansprüche, **dadurch gekennzeichnet, dass** R⁵ für eine Hydroxygruppe -OH steht.

7. Verbindung der Formel (I) nach Anspruch 1, **dadurch gekennzeichnet, dass** sie aus der Verbindung bzw. den Verbindungen der Formel (V) sowie den pharmazeutisch unbedenklichen Additionssalzen davon, Hydraten davon und/oder Solvaten davon ausgewählt ist: 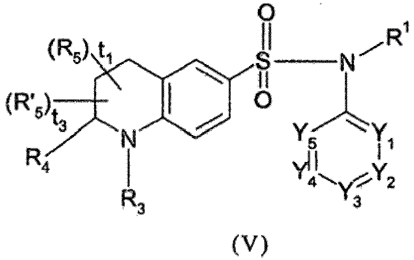 wobei in der Formel (V) R¹, R³, R⁴, R⁵, R'⁵ und Y¹ bis Y⁵ und die Indices t1 und t3 die gleichen Bedeutungen wie in der Formel (I) gemäß einem der vorhergehenden Ansprüche haben.

8. Verbindung der Formel (I) nach Anspruch 7, **dadurch gekennzeichnet, dass** sie aus der Verbindung bzw. den Verbindungen der Formel (V') sowie den pharmazeutisch unbedenklichen Additionssalzen davon, Hydraten davon und/oder Solvaten davon ausgewählt ist: 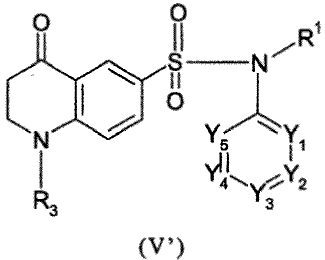 wobei in der Formel (V') R¹, R³ und Y¹ bis Y⁵ die gleichen Bedeutungen wie in der Formel (I) gemäß einem der vorhergehenden Ansprüche haben.

9. Verbindung der Formel (I) nach Anspruch 7, **dadurch gekennzeichnet, dass** sie aus der Verbindung bzw. den Verbindungen der Formel (V") sowie den pharmazeutisch unbedenklichen Additionssalzen davon, Hydraten davon und/oder Solvaten davon ausgewählt ist: 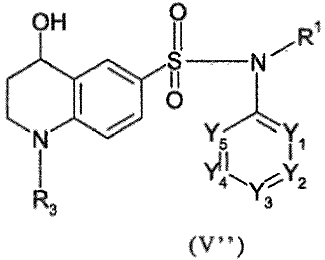 wobei in der Formel (V") R¹, R³ und Y¹ bis Y⁵ die gleichen Bedeutungen wie in der Formel (I) gemäß einem der vorhergehenden Ansprüche haben.

10. Verbindung der Formel (I) nach einem der vorhergehenden Ansprüche, **dadurch gekennzeichnet, dass** sie aus den folgenden Verbindungen ausgewählt ist:
| | |
|---|---|
| 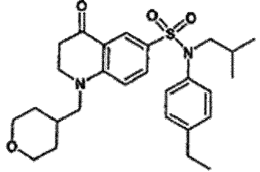 | 4-Oxo-1-(tetrahydropyran-4-ylmethyl)-1,2,3,4-tetrahydrochinolin-6-sulfonsäure(4-ethylphenyl)isobutylamid |
| | Verbindung 1 |
| 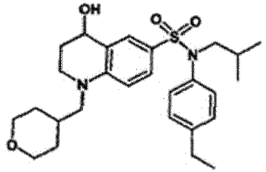 | 4-Hydroxy-1-(tetrahydropyran-4-ylmethyl)-1,2,3,4-tetrahydrochinolin-6-sulfonsäure(4-ethylphenyl)isobutylamid |
| | Verbindung 2 |
| 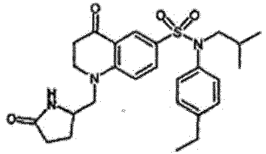 | 4-Oxo-1-(5-oxopyrrolidin-2-ylmethyl)-1,2,3,4-tetrahydrochinolin-6-sulfonsäure(4-ethylphenyl)isobutylamid |
| | Verbindung 3 |
| 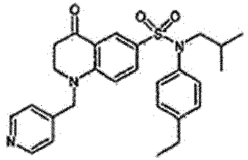 | 4-Oxo-1-pyridin-4-ylmethyl-1,2,3,4-tetrahydrochinolin-6-sulfonsäure(4-ethylphenyl)isobutylamid |
| | Verbindung 4 |
| 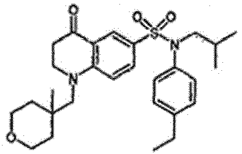 | 1-(4-Methyltetrahydropyran-4-ylmethyl)-4-oxo-1,2,3,4-tetrahydrochinolin-6-sulfonsäure(4-ethylphenyl)isobutylamid |
| | Verbindung 6 |
| 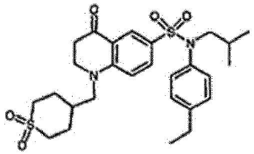 | 1-(1,1-Dioxohexahydro-1λ⁶-thiopyran-4-ylmethyl)-4-oxo-1,2,3,4-tetrahydrochinolin-6-sulfonsäure(4-ethylphenyl)isobutylamid |
| | Verbindung 7 |
| 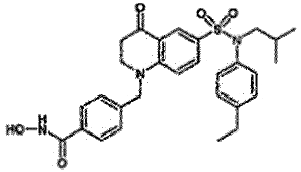 | 4-{6-[(4-Ethylphenyl)isobutyl-sulfamoyl]-4-oxo-3,4-dihydro-2H-chinolin-1-ylmethyl}-N-hydroxybenzamid |
| | Verbindung 8 |
| 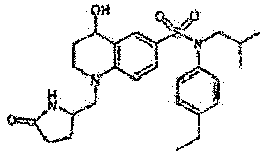 | 4-Hydroxy-1-(5-oxopyrrolidin-2-ylmethyl)-1,2,3,4-tetrahydrochinolin-6-sulfonsäure(4-ethylphenyl)isobutylamid |
| | Verbindung 9 |
| 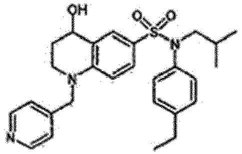 | 4-Hydroxy-1-pyridin-4-ylmethyl-1,2,3,4-tetrahydrochinolin-6-sulfonsäure(4-ethylphenyl)isobutylamid |
| | Verbindung 10 |
| 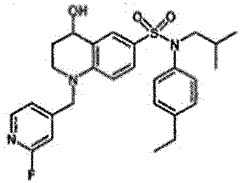 | 1-(2-Fluorpyridin-4-ylmethyl)-4-hydroxy-1,2,3,4-tetrahydrochinolin-6-sulfonsäure(4-ethylphenyl)isobutylamid |
| | Verbindung 11 |
| 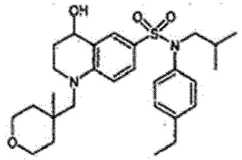 | 1-(4-Methyltetrahydropyran-4-ylmethyl)-4-hydroxy-1,2,3,4-tetrahydrochinolin-6-sulfonsäure(4-ethylphenyl)isobutylamid |
| | Verbindung 14 (Verbindung 218) |
| 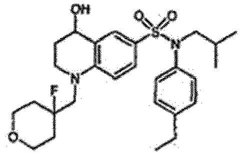 | 1-(4-Fluortetrahydropyran-4-ylmethyl)-4-hydroxy-1,2,3,4-tetrahydrochinolin-6-sulfonsäure(4-ethylphenyl)isobutylamid Verbindung 15 |
| | (Verbindung 219) |
| 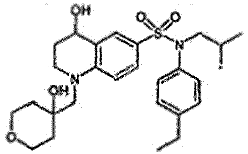 | 4-Hydroxy-1-(4-hydroxytetrahydropyran-4-ylmethyl)-1,2,3,4-tetrahydrochinolin-6-sulfonsäure(4-ethylphenyl)isobutylamid |
| | Verbindung 16 |
| 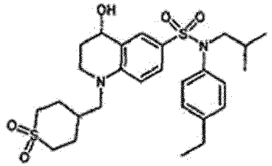 | 1-(1,1-Dioxohexahydro-1λ⁶-thiopyran-4-ylmethyl)-4-hydroxy-1,2,3,4-tetrahydrochinolin-6-sulfonsäure(4-ethylphenyl)isobutylamid |
| | Verbindung 17 |
| 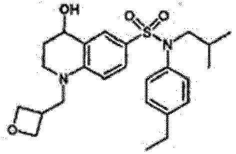 | 4-Hydroxy-1-oxetan-3-ylmethyl-1,2,3,4-tetrahydrochinolin-6-sulfonsäure(4-ethylphenyl)isobutylamid |
| | Verbindung 18 |
| 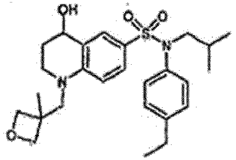 | 4-Hydroxy-1-(3-methyloxetan-3-ylmethyl)-1,2,3,4-tetrahydrochinolin-6-sulfonsäure(4-ethylphenyl)isobutylamid |
| | Verbindung 19 |
| 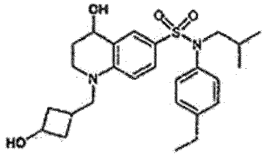 | 4-Hydroxy-1-(3-hydroxycyclobutylmethyl)-1,2,3,4-tetrahydrochinolin-6-sulfonsäure(4-ethylphenyl)isobutylamid Gemisch von Diastereoisomeren: |
| | Verbindung 20 |
| 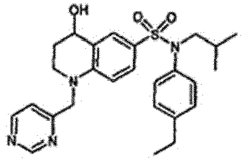 | 4-Hydroxy-1-pyrimidin-4-ylmethyl-1,2,3,4-tetrahydrochinolin-6-sulfonsäure(4-ethylphenyl)isobutylamid |
| | Verbindung 21 |
| 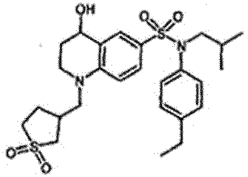 | 1-(1,1-Dioxotetrahydro-1λ⁶-thiophen-3-ylmethyl)-4-hydroxy-1,2,3,4-tetrahydrochinolin-6-sulfonsäure(4-ethylphenyl)isobutylamid |
| | Verbindung 22 |
| 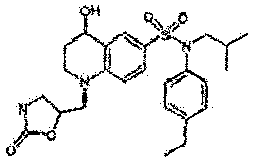 | 4-Hydroxy-1-(2-oxooxazolidin-5-ylmethyl)-1,2,3,4-tetrahydrochinolin-6-sulfonsäure(4-ethylphenyl)isobutylamid |
| | Verbindung 23 |
| 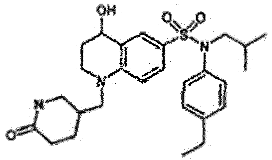 | 4-Hydroxy-1-(6-oxopiperidin-3-ylmethyl)-1,2,3,4-tetrahydrochinolin-6-sulfonsäure(4-ethylphenyl)isobutylamid |
| | Verbindung 24 |
| 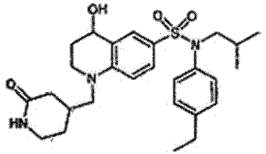 | 4-Hydroxy-1-(2-oxopiperidin-4-ylmethyl)-1,2,3,4-tetrahydrochinolin-6-sulfonsäure(4-ethylphenyl)isobutylamid |
| | Verbindung 25 |
| 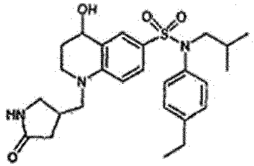 | 4-Hydroxy-1-(5-oxopyrrolidin-3-ylmethyl)-1,2,3,4-tetrahydrochinolin-6-sulfonsäure(4-ethylphenyl)isobutylamid |
| | Verbindung 26 |
| 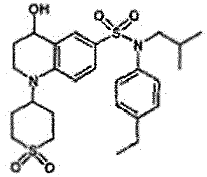 | 1-(1,1-Dioxohexahydro-1λ⁶-thiopyran-4-yl)-4-hydroxy-1,2,3,4-tetrahydrochinolin-6-sulfonsäure(4-ethylphenyl)isobutylamid |
| | Verbindung 27 |
| 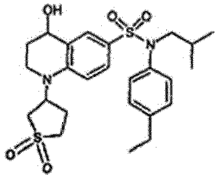 | 1-(1,1-Dioxotetrahydro-1λ⁶-thiophen-3-yl)-4-hydroxy-1,2,3,4-tetrahydrochinolin-6-sulfonsäure(4-ethylphenyl)isobutylamid |
| | Verbindung 28 |
| 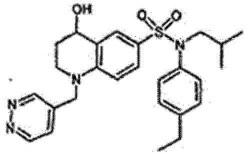 | 4-Hydroxy-1-pyridazin-4-ylmethyl-1,2,3,4-tetrahydrochinolin-6-sulfonsäure(4 ethylphenyl)isobutylamid |
| | Verbindung 29 |
| 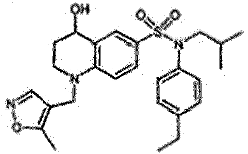 | 4-Hydroxy-1-(5-methylisoxazol-4-ylmethyl)-1,2,3,4-tetrahydrochinolin-6-sulfonsäure(4-ethylphenyl)isobutylamid |
| | Verbindung 30 |
| 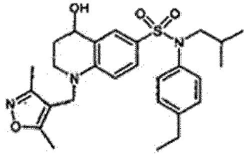 | 1-(3,5-Dimethylisoxazol-4-ylmethyl)-4-hydroxy-1,2,3,4-tetrahydrochinolin-6-sulfonsäure(4-ethylphenyl)isobutylamid |
| | Verbindung 31 |
| 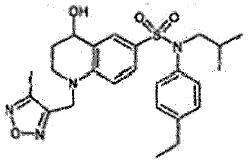 | 4-Hydroxy-1-(4-methylfurazan-3-ylmethyl)-1,2,3,4-tetrahydrochinolin-6-sulfonsäure(4-ethylphenyl)isobutylamid |
| | Verbindung 32 |
| 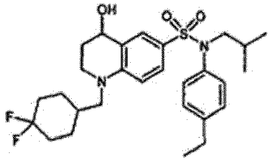 | 1-(4,4-Difluorcyclohexylmethyl)-4-hydroxy-1,2,3,4-tetrahydrochinolin-6-sulfonsäure(4-ethylphenyl)isobutylamid |
| | Verbindung 33 |
| 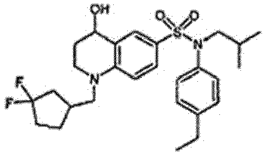 | 1-(3,3-Difluorcyclopentylmethyl)-4-hydroxy-1,2,3,4-tetrahydrochinolin-6-sulfonsäure(4-ethylphenyl)isobutylamid |
| | Verbindung 34 |
| 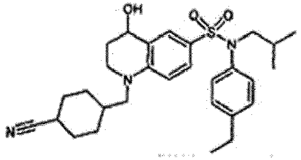 | 1-(4-Cyanocyclohexylmethyl)-4-hydroxy-1,2,3,4-tetrahydrochinolin-6-sulfonsäure(4-ethylphenyl)isobutylamid |
| | Verbindung 35 |
| 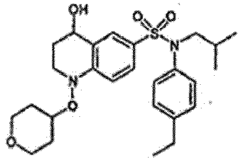 | 4-Hydroxy-1-(tetrahydropyran-4-yloxy)-1,2,3,4-tetrahydrochinolin-6-sulfonsäure(4-ethylphenyl)isobutylamid |
| | Verbindung 36 |
| 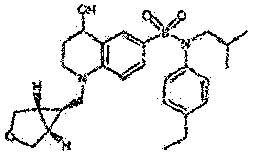 | 4-Hydroxy-1-[(1S,5R,6S)-1-(3-oxabicyclo[3.1.0]hex-6-yl)methyl]-1,2,3,4-tetrahydrochinolin-6-sulfonsäure(4-ethylphenyl)isobutylamid |
| | Verbindung 37 |
| 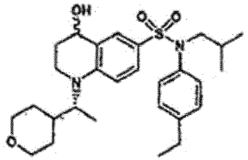 | 4-Hydroxy-1-[(R)-1-(tetrahydropyran-4-yl)ethyl]-1,2,3,4-tetrahydrochinolin-6-sulfonsäure(4-ethylphenyl)isobutylamid |
| | Verbindung 38 |
| 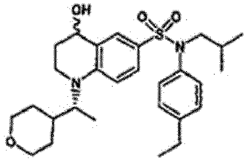 | 4-Hydroxy-1-[(R)-1-(tetrahydropyran-4-yl)ethyl]-1,2,3,4-tetrahydrochinolin-6-sulfonsäure(4-ethylphenyl)isobutylamid |
| | Verbindung 39 |
| 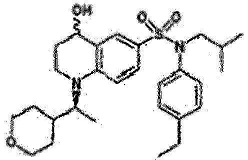 | 4-Hydroxy-1-[(S)-1-(tetrahydropyran-4-yl)ethyl]-1,2,3,4-tetrahydrochinolin-6-sulfonsäure(4-ethylphenyl)isobutylamid |
| | Verbindung 40 |
| 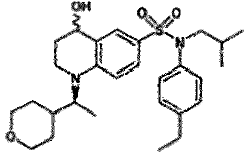 | 4-Hydroxy-1-[(S)-1-(tetrahydropyran-4-yl)ethyl]-1,2,3,4-tetrahydrochinolin-6-sulfonsäure(4-ethylphenyl)isobutylamid |
| | Verbindung 41 |
| 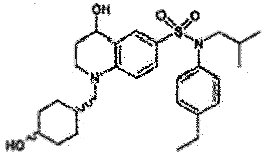 | 4-Hydroxy-1-(4-hydroxycyclohexylmethyl)-1,2,3,4-tetrahydrochinolin-6-sulfonsäure(4-ethylphenyl)isobutylamid |
| | Verbindung 42 |
| 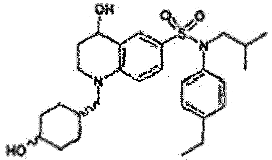 | 4-Hydroxy-1-(4-hydroxycyclohexylmethyl)-1,2,3,4-tetrahydrochinolin-6-sulfonsäure(4-ethylphenyl)isobutylamid |
| | Verbindung 43 |
| 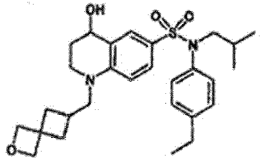 | 4-Hydroxy-1-(2-oxaspiro[3.3]hept-6-ylmethyl)-1,2,3,4-tetrahydrochinolin-6-sulfonsäure(4-ethylphenyl)isobutylamid |
| | Verbindung 44 |
| 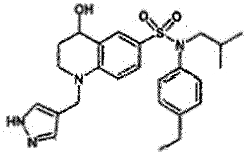 | 4-Hydroxy-1-(1H-pyrazol-4-ylmethyl)-1,2,3,4-tetrahydrochinolin-6-sulfonsäure(4-ethylphenyl)isobutylamid |
| | Verbindung 45 |
| 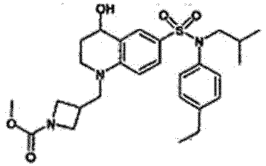 | 3-{6-[(4-Ethylphenyl)isobutyl-sulfamoyl]-4-hydroxy-3,4-dihydro-2H-chinolin-1-ylmethyl}azetidin-1-carbonsäuremethylester |
| | Verbindung 46 |
| 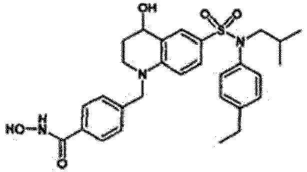 | 4-{6-[(4-Ethylphenyl)isobutyl-sulfamoyl]-4-hydroxy-3,4-dihydro-2H-chinolin-1-ylmethyl}-N-hydroxybenzamid |
| | Verbindung 47 |
| 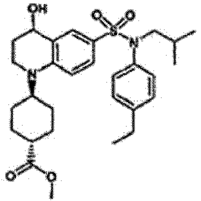 | (1R,4R)-4-(6-(N-(4-Ethylphenyl)-N-isobutylsulfamoyl)-4-hydroxy-3,4-dihydrochinolin-1(2H)-yl)cyclohexan-1-carbonsäuremethylester |
| | Verbindung 48 |
| 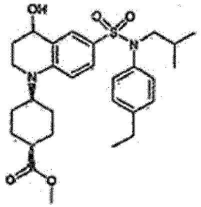 | (1S,4S)-4-(6-(N-(4-Ethylphenyl)-N-isobutylsulfamoyl)-4-hydroxy-3,4-dihydrochinolin-1(2H)-yl)cyclohexan-1-carbonsäuremethylester |
| | Verbindung 49 |
| 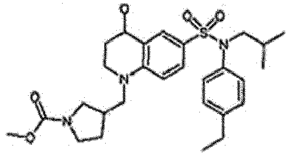 | 3-{6-[(4-Ethylphenyl)isobutyl-sulfamoyl]-4-hydroxy-3,4-dihydro-2H-chinolin-1-ylmethyl}pyrrolidin-1-carbonsäuremethylester Verbindung 50 |
| 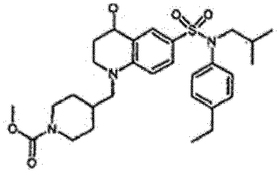 | 4-{6-[(4-Ethylphenyl)isobutyl-sulfamoyl]-4-hydroxy-3,4-dihydro-2H-chinolin-1-ylmethyl}piperidin-1-carbonsäuremethylester |
| | Verbindung 51 (Verbindung 255) |
| 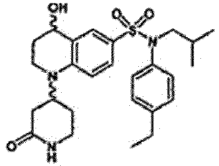 | **Diastereoisomer 1** |
| | 4-Hydroxy-1-(2-oxopiperidin-4-yl)-1,2,3,4-tetrahydrochinolin-6-sulfonsäure(4-ethylphenyl)isobutylamid |
| | Verbindung 52 |
| 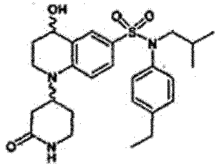 | **Diastereoisomer** 2 |
| | 4-Hydroxy-1-(2-oxopiperidin-4-yl)-1,2,3,4-tetrahydrochinolin-6-sulfonsäure(4-ethylphenyl)isobutylamid |
| | Verbindung 53 |
| 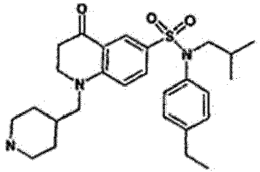 | 4-Oxo-1-piperidin-4-ylmethyl-1,2,3,4-tetrahydrochinolin-6-sulfonsäure(4-ethylphenyl)isobutylamid |
| | Verbindung 54 |
| 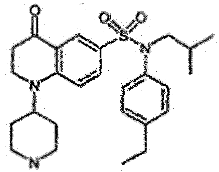 | 4-Oxo-1-piperidin-4-yl-1,2,3,4-tetrahydrochinolin-6-sulfonsäure(4-ethylphenyl)isobutylamid |
| | Verbindung 55 |
| 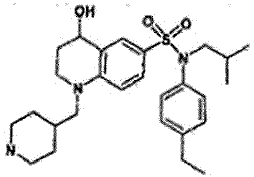 | 4-Hydroxy-1-piperidin-4-ylmethyl-1,2,3,4-tetrahydrochinolin-6-sulfonsäure(4-ethylphenyl)isobutylamid |
| | Verbindung 56 |
| 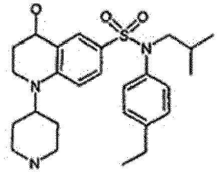 | 4-Hydroxy-1-piperidin-4-yl-1,2,3,4-tetrahydrochinolin-6-sulfonsäure(4-ethylphenyl)isobutylamid |
| | Verbindung 57 |
| 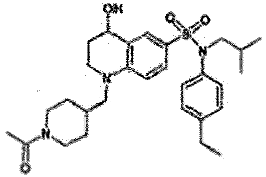 | 4-Hydroxy-1-piperidin-4-ylmethyl-1,2,3,4-tetrahydrochinolin-6-sulfonsäure(4-ethylphenyl)isobutylamid |
| | Verbindung 58 |
| 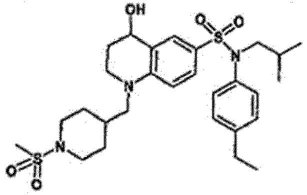 | 4-Hydroxy-1-piperidin-4-ylmethyl-1,2,3,4-tetrahydrochinolin-6-sulfonsäure(4-ethylphenyl)isobutylamid |
| | Verbindung 59 |
| 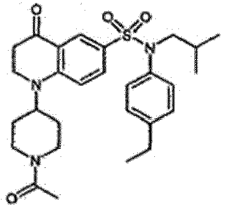 | 1-(1-Acetylpiperidin-4-yl)-4-oxo-1,2,3,4-tetrahydrochinolin-6-sulfonsäure(4-ethylphenyl)isobutylamid |
| | Verbindung 60 |
| 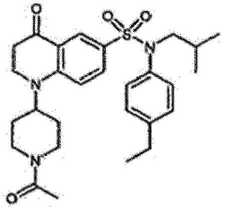 | 1-(1-Acetylpiperidin-4-yl)-4-hydroxy-1,2,3,4-tetrahydrochinolin-6-sulfonsäure(4-ethylphenyl)isobutylamid |
| | Verbindung 61 |
| 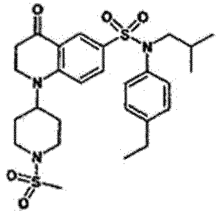 | 1-(1-Acetylpiperidin-4-yl)-4-oxo-1,2,3,4-tetrahydrochinolin-6-sulfonsäure(4-ethylphenyl)isobutylamid |
| | Verbindung 62 |
| 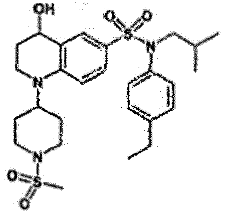 | 4-Hydroxy-1-(1-methansulfonylpiperidin-4-yl)-1,2,3,4-tetrahydrochinolin-6-sulfonsäure(4-ethylphenyl)isobutylamid |
| | Verbindung 64 |
| 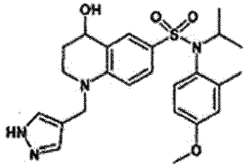 | 4-Hydroxy-1-(1H-pyrazol-4-ylmethyl)-1,2,3,4-tetrahydrochinolin-6-sulfonsäureisopropyl(4-methoxy-2-methylphenyl) amid |
| | Verbindung 65 |
| 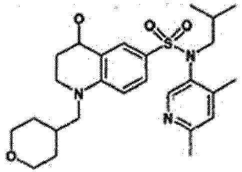 | 4-Hydroxy-1-(tetrahydropyran-4-ylmethyl)-1,2,3,4-tetrahydrochinolin-6-sulfonsäure(4,6-dimethylpyridin-3-yl)isobutylamid |
| | Verbindung 66 |

11. Verbindung nach einem der vorhergehenden Ansprüche zur Verwendung als Medikament.

12. Verbindung nach einem der Ansprüche 1 bis 10 zur Verwendung bei der Behandlung von Akne.

13. Verbindung (en) nach einem der Ansprüche 1 bis 10 zur Verwendung bei der Behandlung von Psoriasis.

14. Pharmazeutische Zusammensetzung, umfassend eine oder mehrere Verbindungen gemäß einem der Ansprüche 1 bis 10.

15. Pharmazeutische Zusammensetzung gemäß Anspruch 14 zur Verwendung bei der Behandlung von Akne, atopischer Dermatitis und/oder Psoriasis.

## Claims

1. Compound of formula (I), pharmaceutically acceptable addition salts thereof, hydrates thereof and/or solvates thereof: 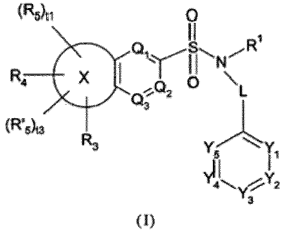 in which formula (I):
• L represents a single bond or a methylene group CH₂,
• X represents the following cyclic radical: 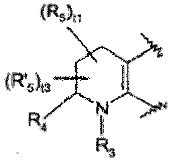
• one or two of the elements Y¹, Y², Y³, Y⁴ and Y⁵ represent (s) a nitrogen atom and the other elements correspond to a -CR² group, or each of the elements Y¹, Y², Y³, Y⁴ and Y⁵ corresponds to a -CR² group,
• one or two of the elements Q¹, Q² and Q³ represent (s) a nitrogen atom and the other element(s) correspond(s) to a -CR^{2a} group, or each of the elements Q¹, Q² and Q³ corresponds to a -CR^{2a} group,
• R¹ represents a linear or branched C₃-C₅ alkyl radical, a C₃-C₅ cycloalkyl radical, a linear or branched C₂-C₅ alkenyl radical, a CH₂-(C₃-C₅)cycloalkyl radical, a C₄-C₅ heterocycloalkyl radical, or a CH₂-(C₄-C₆)heterocycloalkyl radical,
• R² represents a hydrogen atom or a halogen atom, a linear or branched C₁-C₅ alkyl radical, a linear or branched C₂-C₄ alkenyl radical, a C₁-C₄ alkoxy radical, a cyano group -CN, a -C(=O)R'² radical with R'² denoting a C₁-C₃ alkoxy radical, or a -CF₃ radical; said alkyl, alkenyl and alkoxy radicals possibly being substituted with one or more halogen atoms;
• R^{2a} represents a hydrogen atom or a halogen atom, a linear or branched C₁-C₅ alkyl radical, a linear or branched C₂-C₄ alkenyl radical, a C₁-C₄ alkoxy radical, a -CN group, a hydroxyl group -OH, a -CH(R^{3a})OH group, a carboxylic group -COOH, a carbamoyl group -CONR^{2c}R^{2d}, an amido group -NR^{2c}COR^{2d}, an SO₂R^{2c} group, an -SOR^{2c} group, or an-S(=O)(=NH-R^{2c}) group, said alkyl, alkenyl and alkoxy radicals possibly being substituted with one or more halogen atoms,
• R^{2c} and R^{2d}, which may be identical or different, represent a hydrogen atom or a linear or branched C₁-C₅ alkyl radical;
• R^{3a} represents a hydrogen atom or a linear or branched C₁-C₅ alkyl radical;
• t₁ and t₃, which may be identical or different, denote a natural integer 0 or 1,
• R³ and R⁴, which may be identical or different, represent a hydrogen atom or a (CHR⁶)ₙ-(Z)ₒ-(CHR'⁶)ₚ-R⁷ group,
• n, o and p, which may be identical or different, represent zero or a natural integer ranging from 1 to 3,
• when R³ represents a (CHR⁶)ₙ-(Z)ₒ-(CHR'⁶)ₚ-R⁷ group, then R⁴ represents a hydrogen atom,
• when R⁴ represents a (CHR⁶)ₙ-(Z)ₒ-(CHR'⁶)ₚ-R⁷ group, then R³ represents a hydrogen atom,
• Z represents a divalent group chosen from a methylene group -CH₂-, an amino group -NH- and an oxygen atom -0-,
• R⁶ and R'⁶, which may be identical or different, represent a hydrogen atom, a methyl group -CH₃, an -OH group, a hydroxymethyl group, or a carboxylic function -COOH,
• R⁷ represents:
- a hydrogen atom or a halogen atom,
- a COOR'⁷ group with R'⁷ denoting (C₁)alkyl(C₆)heterocycle,
- a non-cationic heterocycloalkyl radical optionally substituted with one or more halogen atoms, one or more linear or branched C₁-C₃ alkyl groups, one or more -OH groups, one or more carbonyl functions, one or more linear or branched C₁-C₄ hydroxyalkyl groups, one or more amino groups, one or more -C(=O)R^{7a} groups, or one or more S(=O)₂R^{7a} groups; R^{7a} representing a linear or branched C₁-C₃ alkyl radical, a linear or branched C₁-C₃ alkoxy radical, or an amino radical N(R^{8a})(R^{9a}),
- a non-cationic C₃-C₆ cycloalkyl radical optionally substituted with one or more methyl radicals, one or more halogen atoms, a cyano group -CN, or one or more -COR¹³ groups; R¹³ denoting a linear or branched C₁-C₃ alkoxy radical, or a hydroxyl group,
- a non-cationic aromatic or heteroaromatic radical optionally substituted with one or more halogen atoms, one or more linear or branched C₁-C₃ alkyl groups, optionally substituted with one or more halogen atoms, one or more C₁-C₃ alkoxy groups, one or more amino groups -NR¹¹R¹², one or more -COR¹¹ groups, one or more -COOR¹¹ groups, one or more amido groups -CONR¹¹R¹², one or more -SOR¹¹ groups, one or more -SO₂R¹¹ groups, one or more -NHCOR¹¹ groups, one or more -NHCOOR¹¹ groups, one or more -SO₂NR¹¹R¹² groups or one or more -CN groups; R¹¹ and R¹², which may be identical or different, representing a hydrogen atom, a hydroxyl radical -OH, or a linear or branched C₁-C₃ alkyl radical optionally substituted with one or more halogen atoms,
• R⁵ represents a hydrogen atom or a halogen atom, a linear or branched C₁-C₃ alkyl radical optionally substituted with one or more halogen atoms; an amino radical -NH₂, a CH₂R'^{7a} radical with R'^{7a} denoting a methoxy radical, a hydroxyl group -OH, a -CH₂COOH group, a -CH(R^{5b})OH group, a carboxylic group -COOH, a -CN group, or a thioxo function,
• R'₅ represents a carbonyl function (C=O) or a thioxo function (C=S),
• R^{8a} and R^{8b}, which may be identical or different, denote a hydrogen atom, a linear or branched C₁-C₃ alkyl radical or a cyclopropyl radical.

2. Compound of formula (I) according to Claim 1, **characterized in that** R⁷ represents a heterocyclic radical chosen from the following heterocycles: 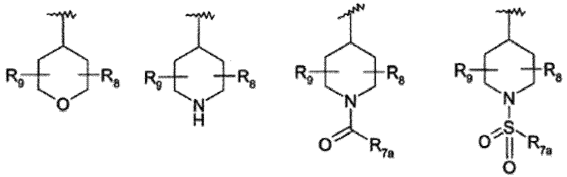 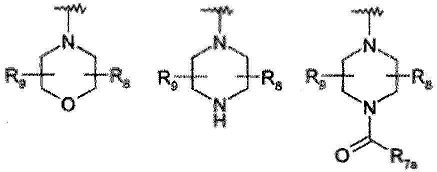 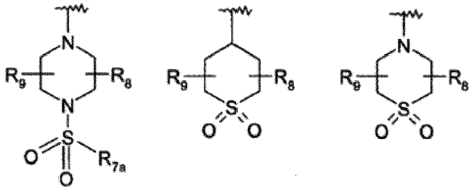 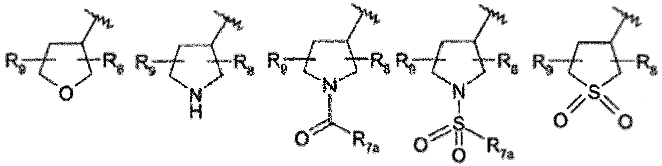 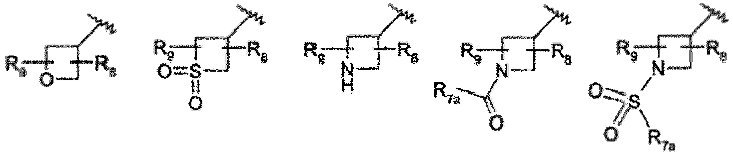 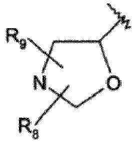 in which:
- R₇ₐ represents a linear or branched C₁-C₃ alkyl radical, a linear or branched C₁-C₃ alkoxy radical or an N(R^{8a})(R^{8b}) amino radical,
- R^{8a} and R^{8b}, which may be identical or different, denote a hydrogen atom, a linear or branched C₁-C₃ alkyl radical or a cyclopropyl radical,
- R₈ and R₉, which may be identical or different, represent a hydrogen atom, a linear or branched C₁-C₃ alkyl radical, a hydroxyl group -OH, a carbonyl function =O, a C₁ hydroxyalkyl radical (-CH₂OH), or an amino group NH₂,
- R₈ and R₉ can form, together with the carbon atoms to which they are attached, a carbocyclic ring comprising from 5 to 7 ring members.

3. Compound of formula (I) according to Claim 1, **characterized in that** R⁷ represents an aromatic or heteroaromatic radical chosen from: 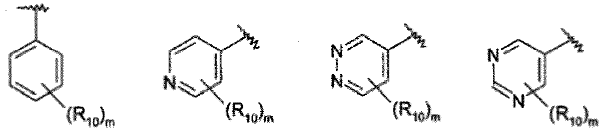 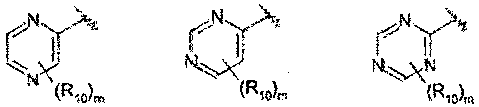 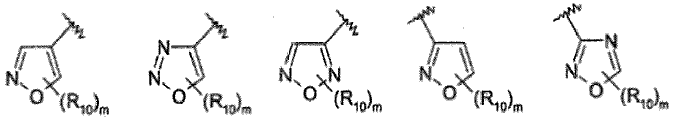 in which:
- R₁₀ represents a hydrogen atom or a halogen atom, a linear or branched C₁-C₃ alkyl group optionally substituted with one or more halogen atoms; a C₁-C₃ alkoxy group, an amino group -NR¹¹R¹², a -COR¹¹ group, a -COOR¹¹ group, an amido group -CONR¹¹R¹², an -SOR¹¹ group, an -SO₂R¹¹ group, an -NHCOR¹¹ group, an -NHCOOR¹¹ group, an -SO₂NR¹¹R¹² group or a -CN group; R¹¹ and R¹², which may be identical or different, representing a hydrogen atom or a linear or branched C₁-C₃ alkyl radical optionally substituted with one or more halogen atoms,
m denotes zero or a natural integer ranging from 1 to 3.

4. Compound of formula (I) according to any one of the preceding claims, **characterized in that**, when t₁ is equal to zero, then t₃ is equal to 1 and, reciprocally, when t₁ is equal to 1, then t₃ denotes zero.

5. Compound of formula (I) according to any one of Claims 1 to 4, **characterized in that** R'⁵ denotes a carbonyl function C=O.

6. Compound of formula (I) according to any one of the preceding claims, **characterized in that** R⁵ represents a hydroxyl group -OH.

7. Compound of formula (I) according to Claim 1, **characterized in that** it is chosen from the compound(s) of formula (V) and also pharmaceutically acceptable addition salts thereof, hydrates thereof and/or solvates thereof: 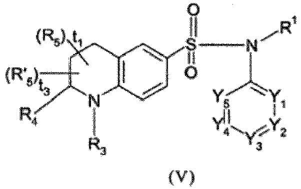 in which formula (V), R¹, R³, R⁴, R⁵, R'⁵ and Y¹ to Y⁵, and the indices t₁ and t₃ have the same meanings as in formula (I) as defined according to any one of the preceding claims.

8. Compound of formula (I) according to Claim 7, **characterized in that** it is chosen from the compound(s) of formula (V') and also pharmaceutically acceptable addition salts thereof, hydrates thereof and/or solvates thereof: 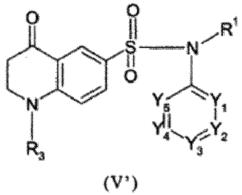 in which formula (V'), R¹, R³ and Y¹ to Y⁵ have the same meanings as in formula (I) as defined according to any one of the preceding claims.

9. Compound of formula (I) according to Claim 7, **characterized in that** it is chosen from the compound(s) of formula (V") and also pharmaceutically acceptable addition salts thereof, hydrates thereof and/or solvates thereof: 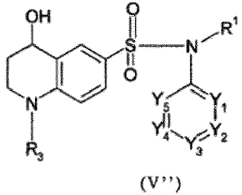 in which formula (V"), R¹, R³ and Y¹ to Y⁵ have the same meanings as in formula (I) as defined according to any one of the preceding claims.

10. Compound of formula (I) according to any one of the preceding claims, **characterized in that** it is chosen from the following compounds:
| | |
|---|---|
| 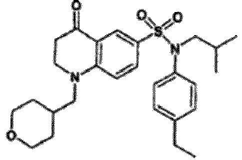 | 4-Oxo-1-(tetrahydropyran-4-ylmethyl)-1,2,3,4-tetrahydroquinoline-6-sulfonic acid (4-ethylphenyl)isobutylamide |
| | Compound 1 |
| 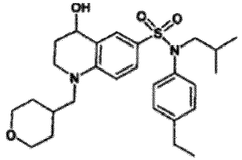 | 4-Hydroxy-1-(tetrahydropyran-4-ylmethyl)θ1,2,3,4-tetrahydroquinoline-6-sulfonic acid (4-ethylphenyl)isobutylamide |
| | Compound 2 |
| 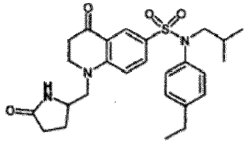 | 4-Oxo-1-(5-oxopyrrolidin-2-ylmethyl)-1,2,3,4-tetrahydroquinoline-6-sulfonic acid (4-ethylphenyl)isobutylamide |
| | Compound 3 |
| 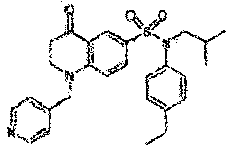 | 4-Oxo-1-pyridin-4-ylmethyl-1,2,3,4-tetrahydroquinoline-6-sulfonic acid (4-ethylphenyl)isobutylamide Compound 4 |
| | |
| 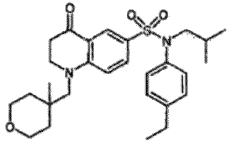 | 1-(4-Methyltetrahydropyran-4-ylmethyl)-4-oxo-1,2,3,4-tetrahydroquinoline-6-sulfonic acid (4-ethylphenyl)isobutylamide |
| | Compound 6 |
| 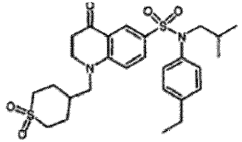 | 1-(1,1-Dioxohexahydro-1λ⁶-thiopyran-4-ylmethyl)-4-oxo-1,2,3,4-tetrahydroquinoline-6-sulfonic acid (4-ethylphenyl)isobutylamide |
| | Compound 7 |
| 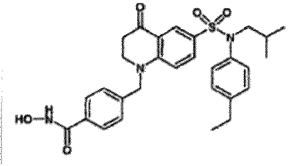 | 4-{6-[(4-Ethylphenyl)isobutylsulfamoyl]-4-oxo-3,4-dihydro-2H-quinolin-1-ylmethyl}-N-hydroxybenzamide |
| | Compound 8 |
| 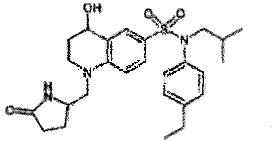 | 4-Hydroxy-1-(5-oxopyrrolidin-2-ylmethyl)-1,2,3,4-tetrahydroquinoline-6-sulfonic acid (4-ethylphenyl)isobutylamide Compound 9 |
| | |
| 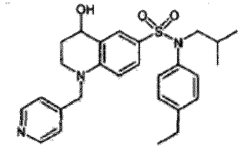 | 4-Hydroxy-1-pyridin-4-ylmethyl-1,2,3,4-tetrahydroquinoline-6-sulfonic acid (4-ethylphenyl)isobutylamide |
| | Compound 10 |
| 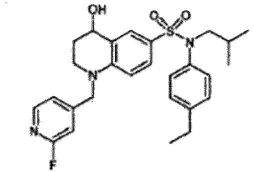 | 1-(2-Fluoropyridin-4-ylmethyl)-4-hydroxy-1,2,3,4-tetrahydroquinoline-6-sulfonic acid (4-ethylphenyl)isobutylamide |
| | Compound 11 |
| 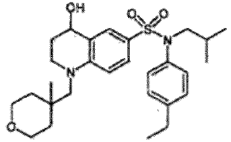 | 1-(4-Methyltetrahydropyran-4-ylmethyl)-4-hydroxy-1,2,3,4-tetrahydroquinoline-6-sulfonic acid (4-ethylphenyl)isobutylamide |
| | Compound 14 (Compound 218) |
| 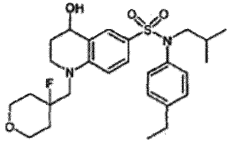 | 1-(4-Fluorotetrahydropyran-4-ylmethyl)-4-hydroxy-1,2,3,4-tetrahydroquinoline-6-sulfonic acid (4-ethylphenyl)isobutylamide |
| | Compound 15 (Compound 219) |
| 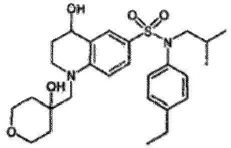 | 4-Hydroxy-1-(4-hydroxytetrahydropyran-4-ylmethyl)-1,2,3,4-tetrahydroquinoline-6-sulfonic acid (4-ethylphenyl)isobutylamide |
| | Compound 16 |
| 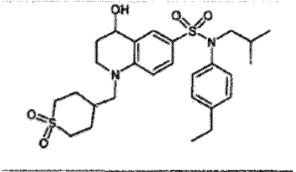 | 1-(1,1-Dioxohexahydro-1λ⁶-thiopyran-4-ylmethyl)-4-hydroxy-1,2,3,4-tetrahydroquinoline-6-sulfonic acid (4-ethylphenyl)isobutylamide |
| | Compound 17 |
| 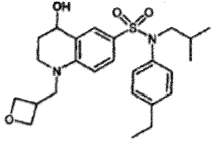 | 4-Hydroxy-1-oxetan-3-ylmethyl-1,2,3,4-tetrahydroquinoline-6-sulfonic acid (4-ethylphenyl)isobutylamide |
| | Compound 18 |
| 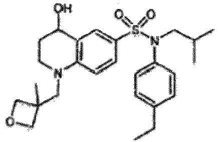 | 4-Hydroxy-1-(3-methyloxetan-3-ylmethyl)-1,2,3,4-tetrahydroquinoline-6-sulfonic acid (4-ethylphenyl)isobutylamide |
| | Compound 19 |
| 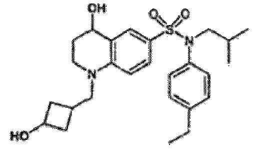 | 4-Hydroxy-1-(3-hydroxycyclobutylmethyl)-1,2,3,4-tetrahydroquinoline-6-sulfonic acid (4-ethylphenyl)isobutylamide mixture of diastereasomers: |
| | Compound 20 |
| 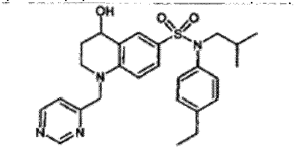 | 4-Hydroxy-1-pyrimidin-4-ylmethyl-1,2,3,4-tetrahydroquinoline-6-sulfonic acid (4-ethylphenyl)isobutylamide |
| | Compound 21 |
| 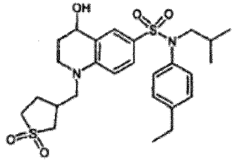 | 1-(1,1-Dioxotetrahydro-1λ⁶-thiophen-3-ylmethyl)-4-hydroxy-1,2,3,4-tetrahydroquinoline-6-sulfonic acid (4-ethylphenyl)isobutylamide |
| | Compound 22 |
| 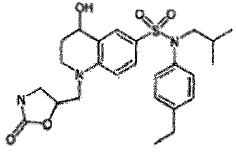 | 4-Hydroxy-1-(2-oxooxazolidin-5-ylmethyl)-1,2,3,4-tetrahydroquinoline-6-sulfonic acid (4-ethylphenyl)isobutylamide |
| | Compound 23 |
| 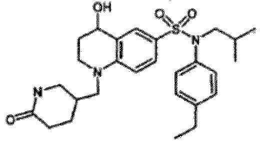 | 4-Hydroxy-1-(6-oxopiperidin-3-ylmethyl)-1,2,3,4-tetrahydroquinoline-6-sulfonic acid (4-ethylphenyl)isobutylamide |
| | Compound 24 |
| 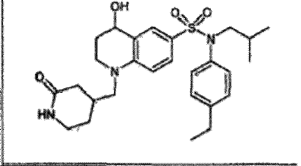 | 4-Hydroxy-1-(2-oxopiperidin-4-ylmethyl)-1,2,3,4-tetrahydroquinoline-6-sulfonic acid (4-ethylphenyl)isobutylamide |
| | Compound 25 |
| 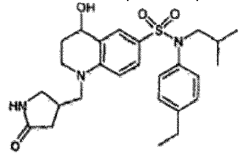 | 4-Hydroxy-1-(5-oxopyrrolidin-3-ylmethyl)-1,2,3,4-tetrahydroquinoline-6-sulfonic acid (4-ethylphenyl)isobutylamide |
| | Compound 26 |
| 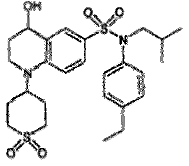 | 1-(1,1-Dioxohexahydro-1λ⁶-thiopyran-4-yl)-4-hydroxy-1,2,3,4-tetrahydroquinoline-6-sulfonic acid (4-ethylphenyl)isobutylamide |
| | Compound 27 |
| 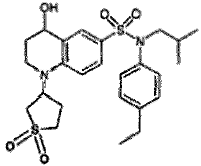 | 1-(1,1-Dioxotetrahydro1 lambda*6*-thiophen-3-yl)-4-hydroxy-1,2,3,4-tetrahydroquinoline-6-sulfonic acid (4-ethylphenyl)isobutylamide Compound 28 |
| | |
| 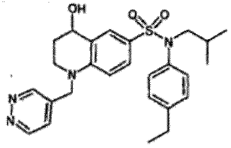 | 4-Hydroxy-1-pyridazin-4-ylmethyl-1,2,3,4-tetrahydroquinoline-6-sulfonic acid (4-ethylphenyl)isobutylamide |
| | Compound 29 |
| 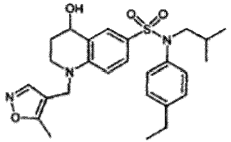 | 4-Hydroxy-1-(5-methylisoxazol-4-ylmethyl)-1,2,3,4-tetrahydroquinoline-6-sulfonic acid (4-ethylphenyl)isobutylamide |
| | Compound 30 |
| 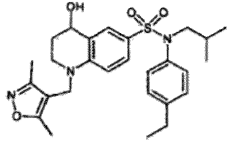 | 1-(3,5-Dimethylisoxazol-4-ylmethyl)-4-hydroxy-1,2,3,4-tetrahydroquinoline-6-sulfonic acid (4-ethylphenyl)isobutylamide |
| | Compound 31 |
| 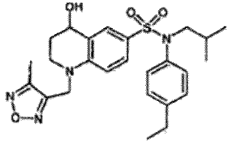 | 4-Hydroxy-1-(4-methylfurazan-3-ylmethyl)-1,2,3,4-tetrahydroquinoline-6-sulfonic acid (4-ethylphenyl)isobutylamide |
| | Compound 32 |
| 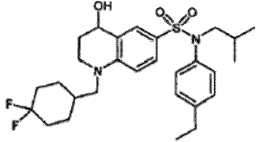 | 1-(4,4-Difluorocyclohexylmethyl)-4-hydroxy-1,2,3,4-tetrahydroquinoline-6-sulfonic acid (4-ethylphenyl)isobutylamide |
| | Compound 33 |
| 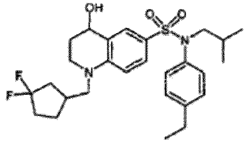 | 1-(3,3-Difluorocyclopentylmethyl)-4-hydroxy-1,2,3,4-tetrahydroquinoline-6-sulfonic acid (4-ethylphenyl)isobutylamide |
| | Compound 34 |
| 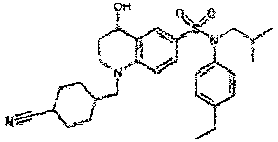 | 1-(4-Cyanocyclohexylmethyl)-4-hydroxy-1,2,3,4-tetrahydroquinoline-6-sulfonic acid (4-ethylphenyl)isobutylamide |
| | Compound 35 |
| 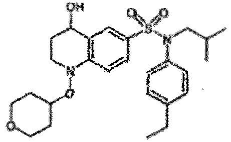 | 4-Hydroxy-1-(tetrahydropyran-4-yloxy)-1,2,3,4-tetrahydroquinoline-6-sulfonic acid (4-ethylphenyl)isobutylamide |
| | Compound 36 |
| 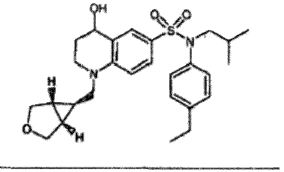 | 4-Hydroxy-1-[(1S,5R,6S)-1-(3-oxabicyclo-[3.1.0]hex-6-yl)methyl]-1,2,3,4-tetrahydroquinoline-6-sulfonic acid (4-ethylphenyl)isobutylamide |
| | Compound 37 |
| 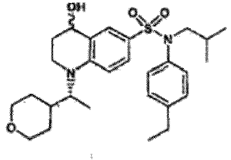 | 4-Hydroxy-1-[(R)-1-(tetrahydropyran-4-yl)ethyl]-1,2,3,4-tetrahydroquinoline-6-sulfonic acid (4-ethylphenyl)isobutylamide |
| | Compound 38 |
| 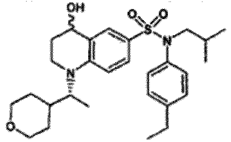 | 4-Hydroxy-1-[(R)-1-(tetrahydropyran-4-yl)ethyl]-1,2,3,4-tetrahydroquinoline-6-sulfonic acid (4-ethylphenyl)isobutylamide |
| | Compound 39 |
| 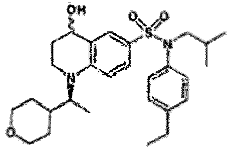 | 4-Hydroxy-1-[(S)-1-(tetrahydropyran-4-yl)ethyl]-1,2,3,4-tetrahydroquinoline-6-sulfonic acid (4-ethylphenyl)isobutylamide |
| | Compound 40 |
| 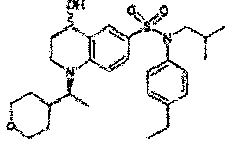 | 4-Hydroxy-1-[(S)-1-(tetrahydropyran-4-yl)ethyl]-1,2,3,4-tetrahydroquinoline-6-sulfonic acid (4-ethylphenyl)isobutylamide |
| | Compound 41 |
| 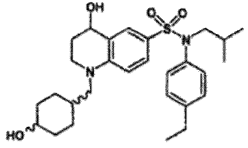 | 4-Hydroxy-1-(4-hydroxycyclohexylmethyl)-1,2,3,4-tetrahydroquinoline-6-sulfonic acid (4-ethylphenyl)isobutylamide |
| | Compound 42 |
| 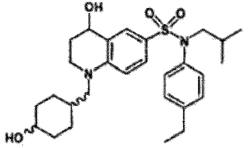 | 4-Hydroxy-1-(4-hydroxycyclohexylmethyl)-1,2,3,4-tetrahydroquinoline-6-sulfonic acid (4-ethylphenyl)isobutylamide |
| | Compound 43 |
| 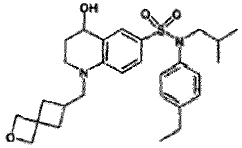 | 4-Hydroxy-1-(2-oxaspiro[3.3]hept-6-ylmethyl)-1,2,3,4-tetrahydroquinoline-6-sulfonic acid (4-ethylphenyl)isobutylamide |
| | Compound 44 |
| 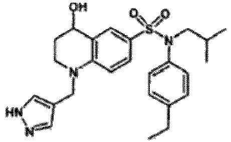 | 4-Hydroxy-1-(1H-pyrazol-4-ylmethyl)-1,2,3,4-tetrahydroquinoline-6-sulfonic acid (4-ethylphenyl)isobutylamide |
| | Compound 45 |
| 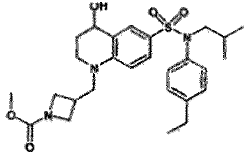 | 3-{6-[(4-Ethylphenyl)isobutylsulfamoyl]-4-hydroxy-3,4-dihydro-2H-quinolin-1-ylmethyl}azetidine-1-carboxylic acid methyl ester |
| | Compound 46 |
| 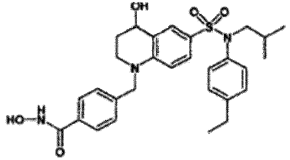 | 4-{6-[(4-Ethylphenyl)isobutylsulfamoyl]-4-hydroxy-3,4-dihydro-2H-quinolin-1-ylmethyl}-N-hydroxybenzamide |
| | Compound 47 |
| 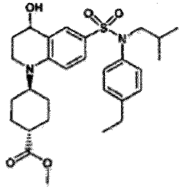 | Methyl (1r,4r)-4-(6-(N-(4-ethylphenyl)-N-isobutylsulfamoyl)-4-hydroxy-3,4-dihydroquinolin-1(2H)-yl)cyclohexane-1-carboxylate |
| | Compound 48 |
| 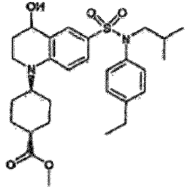 | Methyl (1s,4s)-4-(6-(N-(4-ethylphenyl)-N-isobutylsulfamoyl)-4-hydroxy-3,4-dihydroquinolin-1(2H)-yl)cyclohexane-1-carboxylate |
| | Compound 49 |
| 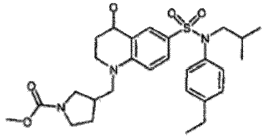 | 3-{6-[(4-Ethylphenyl)isobutylsulfamoyl]-4-hydroxy-3,4-dihydro-2H-quinolin-1-ylmethyl}pyrrolidine-1-carboxylic acid methyl ester |
| | Compound 50 |
| 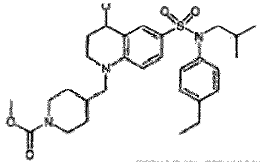 | 4-{6-[(4-Ethylphenyl)isobutylsulfamoyl]-4-hydroxy-3,4-dihydro-2H-quinolin-1-ylmethyl}piperidine-1-carboxylic acid methyl ester |
| | Compound 51 (Compound 255) |
| 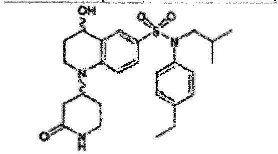 | **Diastereoisomer 1** |
| | 4-Hydroxy-1-(2-oxopiperidin-4-yl)-1,2,3,4-tetrahydroquinoline-6-sulfonic acid (4-ethylphenyl)isobutylamide |
| | Compound 52 |
| 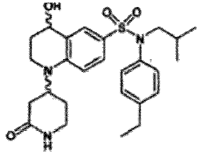 | **Diastereoisomer 2** |
| | 4-Hydroxy-1-(2-oxopiperidin-4-yl)-1,2,3,4-tetrahydroquinoline-6-sulfonic acid (4-ethylphenyl)isobutylamide |
| | Compound 53 |
| 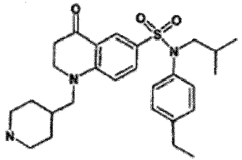 | 4-Oxo-1-piperidin-4-ylmethyl-1,2,3,4-tetrahydroquinoline-6-sulfonic acid (4-ethylphenyl)isobutylamide |
| | Compound 54 |
| 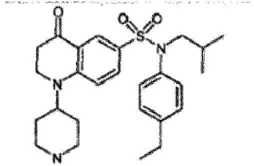 | 4-Oxo-1-piperidin-4-yl-1,2,3,4-tetrahydroquinoline-6-sulfonic acid (4-ethylphenyl)isobutylamide |
| | Compound 55 |
| 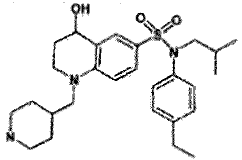 | 4-Hydroxy-1-piperidin-4-ylmethyl-1,2,3,4-tetrahydroquinoline-6-sulfonic acid (4-ethylphenyl)isobutylamide |
| | Compound 56 |
| 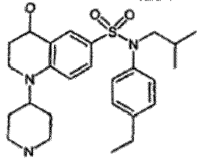 | 4-Hydroxy-1-piperidin-4-yl-1,2,3,4-tetrahydroquinoline-6-sulfonic acid (4-ethylphenyl)isobutylamide |
| | Compound 57 |
| 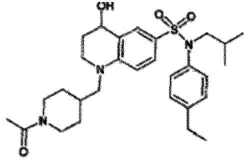 | 4-Hydroxy-1-piperidin-4-ylmethyl-1,2,3,4-tetrahydroquinoline-6-sulfonic acid (4-ethylphenyl)isobutylamide |
| | Compound 58 |
| 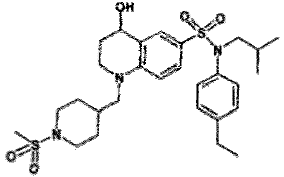 | 4-Hydroxy-1-piperidin-4-ylmethyl-1,2,3,4-tetrahydroquinoline-6-sulfonic acid (4-ethylphenyl)isobutylamide |
| | Compound 59 |
| 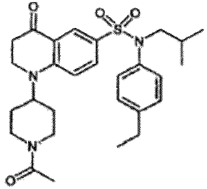 | 1-(1-Acetylpiperidin-4-yl)-4-oxo-1,2,3,4-tetrahydroquinoline-6-sulfonic acid (4-ethylphenyl)isobutylamide |
| | Compound 60 |
| 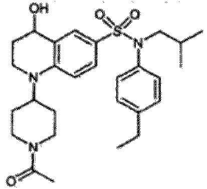 | 1-(1-Acetylpiperidin-4-yl)-4-hydroxy-1,2,3,4-tetrahydroquinoline-6-sulfonic acid (4-ethylphenyl)isobutylamide |
| | Compound 61 |
| 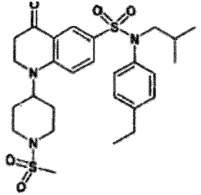 | 1-(1-Acetylpiperidin-4-yl)-4-oxo-1,2,3,4-tetrahydroquinoline-6-sulfonic acid (4-ethylphenyl)isobutylamide |
| | Compound 62 |
| 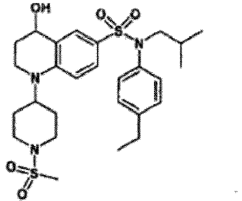 | 4-Hydroxy-1-(1-methanesulfonylpiperidin-4-yl)-1,2,3,4-tetrahydroquinoline-6-sulfonic acid (4-ethylphenyl)isobutylamide |
| | Compound 64 |
| 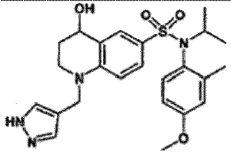 | 4-Hydroxy-1-(1H-pyrazol-4-ylmethyl)-1,2,3,4-tetrahydroquinoline-6-sulfonic acid isopropyl-(4-methoxy-2-methylphenyl) amide |
| | Compound 65 |
| 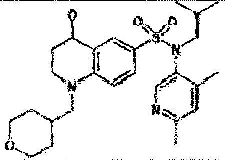 | 4-Hydroxy-1-(tetrahydropyran-4-ylmethyl)-1,2,3,4-tetrahydroquinoline-6-sulfonic acid (4,6-dimethylpyridin-3-yl)isobutylamide |
| | Compound 66 |

11. Compound according to any one of the preceding claims, for use thereof as a medicament.

12. Compound according to any one of Claims 1 to 10, for use thereof in the treatment of acne.

13. Compound (s) according to any one of Claims 1 to 10, for use thereof in the treatment of psoriasis.

14. Pharmaceutical composition comprising one or more compounds as defined according to any one of Claims 1 to 10.

15. Pharmaceutical composition as defined according to Claim 14, for use thereof in the treatment of acne, atopic dermatitis and/or psoriasis.
